# EUROPEAN PATENT SPECIFICATION

(11) **EP 4 284 387 B1**
(45) Date of publication and mention of the grant of the patent: **31.07.2024**
(21) Application number: 22740800.2
(22) Date of filing: 24.06.2022
(51) Int. Cl.: A61K 31/706, A61K 9/00, A61K 31/137, A61K 31/198, A61K 45/06, A61P 25/16

(54) **NAD-AUGMENTATION THERAPY FOR PARKINSON'S DISEASE**
NAD-VERSTÄRKUNGSTHERAPIE FÜR MORBUS PARKINSON
TRAITEMENT D'AUGMENTATION DE NAD CONTRE LA MALADIE DE PARKINSON

(30) Priority: 24.06.2021 GB 202109138; 24.01.2022 GB 202200883; 29.03.2022 GR 20220100277
(43) Date of publication of application: 06.12.2023
(73) Proprietor: VESTLANDETS INNOVASJONSSELSKAP AS (VIS), 5006 Bergen (NO)
(72) Inventor: TZOULIS, Charalampos, 5021 Bergen (NO); DÖLLE, Christian, 5021 Bergen (NO)
(74) Representative: Hoffmann Eitle
(86) International application number: PCT/EP2022/067412
(87) International publication number: WO 2022/269066

(56) References cited:
- WO-A1-2018/039207
- WO-A1-2020/257283
- WO-A1-2022/028678
- BRAKEDAL BRAGE ET AL: "The NADPARK study: A randomized phase I trial of nicotinamide riboside supplementation in Parkinson's disease", CELL METABOLISM, CELL PRESS, UNITED STATES, vol. 34, no. 3, 1 March 2022 (2022-03-01), pages 396, XP086977213, ISSN: 1550-4131, [retrieved on 20220301], DOI: 10.1016/J.CMET.2022.02.001
- SIMON: "Don't get mad! Get NAD! - The Science of Parkinson's", 11 June 2018 (2018-06-11), XP055963843, Retrieved from the Internet <URL:https://scienceofparkinsons.com/2018/06/11/nad/#more-50899> [retrieved on 20220922]

## Description

### FIELD OF THE INVENTION

The present invention generally relates to the treatment of Parkinson's disease in humans. More particularly, it relates to compounds for use in a method for treatment of Parkinson's disease in a human subject; pharmaceutical compositions, pharmaceutical combinations, and dosage forms for use in or as treatment of Parkinson's disease.

### BACKGROUND ART

Parkinson's disease (PD) is a major cause of death and disability and has a worldwide socioeconomic impact. The etiology and molecular pathogenesis underlying PD remain unknown. Due to the lack of understanding of the processes underlying disease initiation and progression, it has not been possible to develop effective therapies in the past. Patients therefore confront a future of progressive disability and premature death with treatments that are at best symptomatic.

While the etiology of PD remains unknown, increasing evidence suggests that mitochondrial dysfunction plays an important role. It has been shown that severe degeneration of the *substantia nigra pars compacta*, similar to that seen in PD, is caused by substances inhibiting mitochondrial complex I and by genetic mutations disrupting mitochondrial maintenance, dynamics, and/or quality control.

Somatic mitochondrial DNA (mtDNA) damage and mitochondrial respiratory chain (MRC) dysfunction have been found in sporadic PD. Abnormal mitochondrial quality control has been linked to familial PD including those caused by mutations in the genes encoding α-synuclein, LRRK2, PINK1, Parkin and DJ-1. Impaired regulation of neuronal mtDNA copy number, partly mediated by polygenic inherited risks, can provide a potential explanation for the loss of dopaminergic neurons in PD. Moreover, MRC deficiency preferentially affecting the mitochondrial respiratory complex I (CI) affects neurons throughout the brain of patients with PD, and is predicted to have profound effects on neuronal metabolism and survival, including ATP deficiency, and decreased rate of mitochondrial NADH oxidation, one of the most essential molecules for bioenergetic conversion and signalling in human cells.

Although methods for treating PD symptoms have been established, the existing methods have the drawback that they only target the symptoms, and not the underlying causes of PD.

Additionally, there are currently no animal models which recapitulate the pathogenesis and/or molecular pathophysiology of PD. To the best of our knowledge, PD does not occur naturally in other animals than humans, and it is not possible to make accurate animal models. That is, even if the results on an animal phenotype may be similar to symptoms of PD, the disease state is generally not comparable with PD. In this regard, PD is very different from other diseases where animal models are much more useful and accurate. Animal models are thus not predictive of human states and/or reactions in PD.

WO 2018/039207 relates to nicotinamide riboside and pterostilbene compositions and methods for treatment of neurodegenerative disorders.

There have been multiple attempts to transfer promising treatments from PD animal models to a human treatment, but these attempts have consistently failed. There is currently no neuroprotective therapy for PD capable making any significant impact on neuronal loss and disease progression. All compounds showing promising results in preclinical models have failed in human trials (Athauda D, Foltynie T. The ongoing pursuit of neuroprotective therapies in Parkinson disease. Nat Rev Neurol 2015;11(1):25-40. Some concrete examples include the STEADY-PD Clinical trial testing Isradipine, a Ca2+ channel blocker that showed promising results in animal models; or the α-synudein antibody drug cinpanemab, which did not achieve proof-of-concept and missed its primary and secondary endpoints.

There is thus a need for an efficient and safe method for treating the physiological causes of PD in humans, and not just for alleviating the disease symptoms. More specifically, there is a need for compounds capable of effectively increasing the NAD levels in the human brain, and/or which can be used as neuroprotective treatment for inhibiting PD progression in human patients. Specifically, there is a need to identify orally administered compounds capable of crossing the blood-brain barrier in humans, succeeding in augmenting cerebral NAD levels in humans, and affecting cerebral metabolism in humans.

It is therefore an object of the present invention to alleviate the drawbacks described above with respect to known methods, and/or provide improvements to the known treatment of Parkinson's disease.

### SUMMARY OF THE INVENTION

As a solution, the present invention provides (1) nicotinamide riboside for use in a method for treatment of Parkinson's disease (PD) in a human subject; (2) a pharmaceutical composition for use in such method, the composition comprising nicotinamide riboside; (3) a dosage form for use in such method, wherein the dosage form comprises nicotinamide riboside or a pharmaceutical composition comprising nicotinamide riboside; and (4) a pharmaceutical combination comprising nicotinamide riboside, a dopaminergic agent and a MAO-B inhibitor.

Currently, three major processes are believed to be associated with the pathogenesis of PD: (i) mitochondrial dysfunction; (ii) proteostasis failure (lysosome & proteasome); and (iii) inflammation. In addition, there is some evidence for (iv) increased DNA-damage, and epigenomic dysregulation, specifically histone hyperacetylation, in PD patients. A concept underlying the present invention is that increase of neuronal NAD levels via suitable drugs may (i) improve mitochondrial function, restore sirtuin activity and histone acetylation status and rescue neuronal dysfunction and death in PD; and that administration of the NAD precursor nicotinamide riboside, in particular oral administration, can improve neuronal NAD deficiency and mitochondrial dysfunction in PD. Correcting these molecular defects may rectify neuronal metabolism and inhibit neurodegeneration. These effects can result in amelioration of clinical symptoms and delayed PD progression. Further concepts underlying the present invention are that (ii) improvement of proteostasis; and/or (iii) reduction in inflammation can beneficially influence clinical symptoms and/or delay PD progression. In addition, (iv) NAD replenishment may ameliorate both DNA damage (by boosting repair) and histone hyperacetylation (by boosting the deacetylases, sirtuins).

The present inventors have now surprisingly identified nicotinamide riboside as a drug suitable for achieving effects underlying the above concepts.

They have surprisingly found that when administered to a human PD subject, nicotinamide riboside effectively crosses the blood-brain barrier and significantly increases the cerebral NAD levels. Therefore, nicotinamide riboside is believed to be excellently suited for use in the treatment of PD in humans, in particular, by correcting NAD deficiency, rectifying the metabolic impairment in PD, and providing a neuroprotective effect, and/or inhibiting PD progression.

NAD is the central redox coenzyme in cellular metabolism and critical supplier of energy equivalents to the respiratory chain. By regulating the activity of the deacetylase enzymes known as sirtuins, NAD regulates several fundamental cellular events including histone acetylation and, by extension, gene expression. The signalling turnover of NAD in human cells is strikingly high with the entire cellular NAD pool being renewed at least once a day. This rapid turnover suggests that decreased NAD synthesis occurs in the PD brain, which can have a profound and immediate impact on neuronal metabolism, including ATP deficiency, altered gene expression and compromised neuronal function and survival. In humans, NAD is either produced *de novo* from tryptophan or via salvage pathways from NAD precursor compounds.

Nicotinamide riboside (NR) is a precursor that effectively elevates NAD synthesis, increases sirtuin activity and is non-toxic to animals and humans. Nicotinamide riboside has good oral bioavailability in humans (Trammell SA, et al. Nat Commun 2016; 7: 12948). It is "Generally Recognized as Safe" (GRAS) for use in food products. Moreover, nicotinamide riboside extends lifespan in yeast (Belenky P, et al. Cell 2007; 129(3): 473-84), has strong neuroprotective effects in animals (Trammell SA, et al. Sci Rep 2016; 6: 26933; Brown KD, et al. Cell Metab 2014; 20(6): 1059-68; Vaur P, et al. FASEB J. 2017 Dec; 31(12):5440-5452; Gong Bet al. Neurobiol Aging 2013; 34(6): 1581-8), and has been shown to achieve substantial clinical improvement in patients with ALS (de la Rubia JE, et al. Amyotroph Lateral Scler Frontotemporal Degener 2019: 1-8). Model evidence supports that NR has neuroprotective effects in mice (Xie, X. et al. Metab Brain Dis 34, 353-366 (2019), and rescues mitochondrial defects and age-related dopaminergic neuronal loss in drosophila fly models (Schöndorf, D. C. et al. Cell Rep 23, 2976-2988 (2018).).

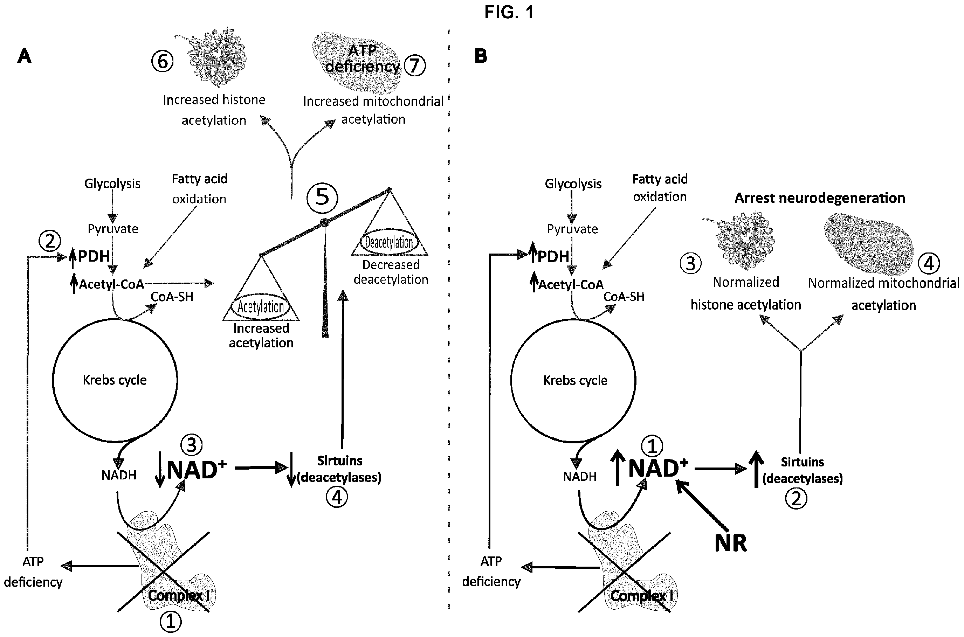

Without wishing to be bound by theory, it is believed that one aspect of NR supplementation is that it leads to NAD replenishment which can reverse mitochondrial dysfunction in PD in human patients. As shown in **FIG. 1 (A)****,** complex I deficiency (1) is a global neuronal phenomenon in PD (Flones IH et al. Acta Neuropathol. 2018 Mar;135(3):409-425). This can lead to decreased ATP synthesis, which in turn stimulates pyruvate dehydrogenase (PDH), causing the accumulation of acetyl-CoA, the main donor of acetyl groups (2). This can increase the acetylation potential in the cell. In addition, complex I deficiency can lead to decreased mitochondrial NADH oxidation and depletion of NAD (3), an essential co-factor for the deacetylases known as sirtuins (4). This combination of increased basal acetylation (2) and decreased deacetylation (4) can lead to a state of hyperacetylation with profound biological effects for neurons and potentially other cell populations in the brain. As shown in **FIG. 1 (B)****,** NAD replenishment via NR supplementation (1) will boost sirtuin activity (2) leading to normalization of acetylation in the nucleus (3) and mitochondria (4). It is therefore believed that NR has the potential to improve neuronal metabolism and arrest or delay neurodegeneration in PD.

Replenishing nicotinamide adenine dinucleotide (NAD) via supplementation of nicotinamide riboside (NR) has been shown to confer neuroprotective effects in healthy aging and models of neurodegenerative diseases, including Parkinson's disease (PD).

Nevertheless, to the best of our knowledge, the prior art provides no enabling disclosure of the suitability of NR to be used for treating PD in human patients. And, as mentioned above, there are currently no animal models which recapitulate the pathogenesis and/or molecular pathophysiology of PD. It is thus not possible to infer or predict the efficacy of NR for treating PD in human patients based on any model evidence.

Additionally, concerns have been repeatedly raised by experts in the research community about the consequences of raising levels of NAD in the body, particularly from the stand point of cancer, suggesting that raising NAD levels may increase the risk of certain types of brain cancer (particularly: glioma), e.g.: Lucena-Cacace, Antonio et al. Oncotarget vol. 8,59 99514-99530. 28 Aug. 2017, doi:10.18632/oncotarget.20577, PMID: 29245920; Gujar, Amit D et al. Proceedings of the National Academy of Sciences of the United States of America vol. 113,51 (2016): E8247-E8256. doi:10.1073/pnas.1610921114, PMID: 27930300.

Overall, the prior art provides no sufficient evidence showing the safety, tolerability and efficacy of using NR for the treatment of PD in human patients.

To assess safety, tolerability, and cerebral penetration of NR therapy in PD in human patients, we conducted a double-blinded, randomized, placebo-controlled phase I trial. 30 newly diagnosed, dopaminergic therapy-naive PD patients were randomized to 1000 mg of orally administered NR or placebo and assessed at baseline and after 30 days of exposure using a combination of clinical, neuroimaging, and molecular measures.

NR treatment led to a significant increase in cerebral NAD-levels, measured by ³¹Phosphorous magnetic resonance spectroscopy, as well as related metabolites in the cerebrospinal fluid. NR-recipients showing increased brain NAD levels exhibited altered cerebral metabolism, measured by ¹⁸F-fluoro-deoxyglucose positron emission tomography, inducing a specific treatment-related metabolic network, that overlapped with key elements of the PD-related spatial covariance pattern (PDRP), and was associated with mild clinical improvement, measured by MDS-UPDRS. In addition, NR substantially augmented the NAD metabolome and induced transcriptional upregulation of processes related to mitochondrial respiration, protection from oxidative stress, lysosomal and proteasomal function in blood cells and skeletal muscle. Furthermore, NR decreased the levels of multiple inflammatory cytokines in serum and cerebrospinal fluid. NR was well tolerated with no related adverse events.

We show that oral NR therapy increases brain NAD levels and impacts cerebral metabolism in patients with PD. Moreover, our findings suggest that NR may have neuroprotective potential by targeting multiple processes implicated in the pathophysiology of the disease. Taken together, the results support the use of NR as a potential neuroprotective therapy for PD. Nicotinamide riboside for use according to the present invention provides surprising tolerability and cerebral bioavailability. NAD supplementation treatment with nicotinamide riboside according to the present invention was studied in clinical trials, showing that the treatment is well tolerated with no adverse effects, and increases NAD levels in the brain as well as related metabolites in the CSF and peripheral tissues. Surprisingly, a highly significant increase in NAD levels in the NR group compared to the placebo group. The NR-recipients showing increased brain NAD levels exhibited altered cerebral metabolism, inducing a specific treatment-related metabolic network that overlapped with key elements of the PD-related spatial covariance pattern (or ameliorating PD-associated neurometabolic patterns), and was associated with a clinical improvement.

To further test NR as a neuroprotective therapy for PD, and in particular to assess NR in delaying nigrostriatal degeneration or denervation, and clinical disease progression in patients with early PD, a multi-centre phase II randomized double-blinded clinical trial is performed, comparing NR to placebo in individuals with early stage PD. In the patients who completed the study and performed brain MRI scans at baseline and week 52, there have been no cases of glioma or any other brain neoplasms among these individuals. The therapy can therefore be considered not to be associated with an increase in glioma prevalence over the duration of treatment.

These results suggest that NR is a safe and well-tolerated treatment in PD, and leads to substantial NAD supplementation in the brain, the target organ of the disease.

### BRIEF DESCRIPTION OF THE DRAWINGS

**FIG. 1** shows NAD replenishment via nicotinamide riboside supplementation could reverse mitochondrial dysfunction in PD.
**FIG. 2** shows 31P-MRS results from a clinical trial of tolerability and cerebral bioavailability of NAD supplementation therapy with nicotinamide riboside in PD.
**FIG. 3** shows 31P-MRS analysis reveals NR mediated increase in cerebral NAD.
**FIG. 4** shows metabolic brain network topography associated with NR treatment.
**FIG. 5** shows NR induces NAD metabolome augmentation in CSF and peripheral tissues of PD patients.
**FIG. 6** shows serological markers affected by NR treatment.
**FIG. 7** shows a CONSORT flow diagram for the study shown in Example 2.
**FIG. 8** shows changes in NRRP expression in the MRS responders treated with NR.
**FIG. 9** shows whole-brain displays of NRRP and PDRP brain network topographies in FDG PET.
**FIG. 10** shows metabolomic analysis in muscle (related to FIG. 5).
**FIG. 11** shows metabolomic analysis in PBMCs (related to FIG. 5).
**FIG. 12** shows cell composition of the PBMC fraction is unaffected by NR treatment.
**FIG. 13** shows cytokine levels in NR and placebo treated participants (related to FIG. 6)
**FIG. 14** shows a flowchart for dopaminergic therapy during screening and treatment period.
**FIG. 15** represents a box plots showing the change (delta) of the DAT-scan tracer uptake in the posterior putamen between the second and first examination (delta = scan2 - scan1), as described in Example 4.
**FIG. 16**: NR treatment increases total NAD levels in PBMCs.
**FIG. 17**: Exemplary pathogenic (i.e., definitely, probably or possibly pathogenic) mutations in SNCA, LRRK2, VPS35, PRKN, and PINK1.

### DETAILED DESCRIPTION OF THE INVENTION

The present invention is defined by the claims. Any subject-matter falling outside the scope of the claims is provided for information only.

Parkinson's disease (PD) affects 1-2% of the population above the age of 65, and is a major cause of death and disability, with a rapidly growing global socioeconomic impact. Current treatments for PD can provide partial symptomatic relief, mainly for motor symptoms, but make no substantial impact on disease progression. Despite several candidate neuroprotective therapies showing encouraging preclinical results, these have failed to show disease-modifying effects in clinical trials.

A growing body of evidence supports that boosting cellular levels of nicotinamide adenine dinucleotide (NAD) may confer neuroprotective effects in both healthy aging and neurodegeneration. NAD can shuffle between an oxidized (NAD⁺) and reduced (NADH) state, and constitutes an essential cofactor for metabolic redox reactions, including mitochondrial respiration. Furthermore, NAD⁺ is substrate to vital signaling reactions involved in DNA repair, histone and other protein deacetylation, and second messenger generation. These reactions consume NAD⁺ at high rates, requiring constant replenishment via NAD biosynthesis. NAD levels have been shown to decline with age and this is believed to contribute to age-related diseases. Conversely, increasing the NAD replenishment rate, via supplementation of precursors, and/or enhancing the NAD⁺/NADH ratio (e.g., via caloric restriction) have shown beneficial effects on life- and healthspan in multiple model systems, and evidence of neuroprotection in animal models of neurodegeneration and other age-related diseases.

Replenishing NAD could potentially help ameliorate several major processes implicated in the pathogenesis of PD, including mitochondrial respiratory dysfunction, neuroinflammation, epigenomic dysregulation and increased neuronal DNA damage.

NAD can be replenished via supplementation of nicotinamide riboside (NR), a vitamin B3 molecule and biosynthetic precursor of NAD. NR has undergone extensive preclinical testing and is well tolerated by adult humans, showing no evidence of toxicity with doses up to at least 2000 mg daily. Trials in healthy individuals have shown that oral intake of 1000 mg NR daily substantially elevates total levels of NAD and related metabolites in blood and muscle, boosts mitochondrial bioenergetics and decreases circulating inflammatory cytokines (Trammell SA, et al. Nat Commun 2016; 7: 12948). Moreover, evidence from cell and animal studies suggests that NR supplementation promotes healthspan and has neuroprotective effects in models of Cockayne syndrome, noise-induced injury, amyotrophic lateral sclerosis, Alzheimer's disease and PD (e.g., Brown KD, Maqsood S, Huang JY, Pan Y, Harkcom W, Li W et al. Cell Metab 2014; 20: 1059-68; Xie X, et al. Metab Brain Dis 2019; 34: 353-366; Schondorf DC, et al. Cell Rep 2018; 23: 2976-2988). Taken together, this evidence suggests that NR may hold promise as a potential neuroprotective agent for PD.

However, several critical knowledge gaps had not yet been addressed in the prior art. Specifically, it needs to be established whether NR is well-tolerated, augments cerebral NAD levels, and affects cerebral metabolism in human patients with PD. To address these questions, we conducted a double-blinded, randomized, placebo-controlled phase I study in newly diagnosed PD patients, naive to dopaminergic therapy to assess the tolerability, cerebral bioavailability and molecular effects of NR therapy in PD. A total of 30 individuals with newly diagnosed, drug-naive PD were randomized to NR 500 mg x2/day or placebo for 30 days, as described in Examples 1, 2 and 4. We found that NR supplementation was well tolerated with no adverse effects. Treatment increased brain NAD levels, as well as related metabolites in the CSF and peripheral tissues. At the individual level, the increase in cerebral NAD was particularly evident in 10/13 NR-recipients from whom data was available. The NR-recipients who showed increased brain NAD levels exhibited altered cerebral metabolism, inducing a specific treatment-related metabolic network that overlapped with key elements of the PD-related spatial covariance pattern (PDRP). NR was associated with clinical improvement. In summary, the use of NR can show multiple promising advantages: 1) NR is well-tolerated; 2) NR achieves brain penetration; 3) NR is associated with clinical improvement of PD; 4) NR has a major impact on cerebral metabolism; and/or 5) NR has widespread metabolic and regulatory effects. Our findings suggest that NR can be useful as a neuroprotective therapy in PD in human patients.

Additionally, we also observed that NR's effects were heterogeneous across the study population, raising the question of individualized dose-dependent responses. Doses over 1000 mg, although safe, have not been previously tested in disease. And, while NR doses of up to 2000 mg per day have been tested in humans with no signs of toxicity, doses over 2000 mg have not been previously tested in humans. Thus, several additional knowledge gaps have not been addressed in the prior art in order to further develop NR towards a PD-drug, so that its full therapeutic potential is harnessed while maximizing clinical benefit and impact: (1) What is the Optimal Biological Dose (OBD) of NR in PD; and (2) are higher NR doses safe in PD and healthy individuals.

We define the OBD of NR as the dose required to achieve an optimal neurometabolic response in PD, i.e., maximal cerebral NAD increase and optimal change in cerebral metabolic pattern, in the absence of toxicity. This dose may be uniform or individualized for specific patients. The phase I study mentioned above showed that the NR-mediated increase in cerebral NAD-levels, and accompanying metabolic and clinical response, can vary across individuals. The fact that all NR-recipients showed a robust metabolic response in blood, muscle and CSF, suggests that the variable cerebral NAD response may reflect interindividual variability in cerebral NAD metabolism (i.e., variation in the rate of NAD-synthesis or consumption). It is likely that such differences can be modulated by varying the substrate concentration (i.e., the intake dose of NR). This question is important, so that NR-therapy can be correctly dosed and tailored to individual patients to achieve an optimal neurometabolic response. To be able to optimize the dose for NR in PD, we aimed to establish the range of safe NR dosage by conducting a single center randomized double blinded safety study to assess the safety and tolerability of an oral dose of up to 3000 mg of NR in individuals with PD, as described in Example 5.

The present invention provides the following aspects and embodiments.

### Nicotinamide riboside for use in the treatment of PD

The present invention relates, in a first aspect, to nicotinamide riboside for use in a method for treatment of Parkinson's disease (PD) in a human subject. In the context of the present invention, the term "treatment" is intended to encompass any kind of therapeutic treatment of the human body. The terms "therapy" and "therapeutic treatment" cover prophylactic methods of treating a disease, curative methods of treating a disease, and/or methods for alleviation of symptoms of a disease. The terms "prophylactic treatment", "preventive treatment" and "preventing" have the same meaning and denote a treatment aiming at maintaining health by preventing ill effects that would otherwise arise. All salt forms of nicotinamide riboside are intended to be embraced by the scope of the present invention.

According to an embodiment, the treatment is for preventing, decreasing and/or delaying the progression of PD.

According to an embodiment, the treatment is a neuroprotective therapy. In the context of the present invention, the term "neuroprotective therapy" refers to a treatment which protect neurons from degeneration (i.e., dysfunction and/or death), and enables their recovery and restoring of their functions; more specifically, any intervention which delays or prevents the death of dopaminergic neurons and other neuronal populations and/or cell types of the central, peripheral and/or autonomic nervous systems, which are affected in PD, and, therefore, slows or halts disease progression and/or prevents disease initiation. While dopaminergic neurons are a cell type affected in PD, PD affects also many other different neuronal populations across the central and autonomic/peripheral nervous systems. Thus, neuroprotection as used herein generally relates to preventing the death of dopaminergic neurons and/or any other neuronal population affected by PD across the central and autonomic/peripheral nervous systems.

According to an embodiment, the PD is early PD.

In the context of the present invention, PD can be during its pre-motor or motor phase. PD during its pre-motor phase can refer to the time before the classic motor features of tremor, rigidity and bradykinesia become apparent.

Pre-motor phase PD can be divided into stages leading to manifest PD based on the presence of clinical, physiological or risk-markers of disease. Working backward from recognizable PD that could be diagnosed based on accepted criteria such as those by the UK Brain Bank, these stages include: 1) the pre-diagnostic phase, 2) the pre-motor phase, the 3) pre-clinical phase and 4) the pre-physiological phase. Diagnostic criteria and definitions for these phases are described in Siderowf, A. Mov Disord. 2012 April 15; 27(5): 608-616. According to a preferred embodiment, the PD is in the pre-diagnostic phase, pre-motor phase, pre-clinical phase or pre-physiological phase. Without wishing to be bound by theory, the patient to be treated is preferably in the early pre-motor or more preferably presymptomatic (pre-diagnostic) phases, from the viewpoint of achieving greater therapeutic success.

Alternatively, PD in the context of the present invention can be in Phase I (risk stage), Phase II (premotor stage), Phase III (early motor stage) and Phase IV (advanced PD), as defined in Bargiotas P, et al. Current Opinion in Neurology. 2016 Dec; 29(6):763-772. According to a preferred embodiment, the PD is in the Phase I (risk stage), Phase II (premotor stage) or Phase III (early motor stage).

PD affects ~2% of everyone above 65 years and -4% of everyone above 85. Since NR is non-toxic, it can be beneficial to use NR as a prophylactic agent, e.g., in the general elderly population, preferably for subjects over age of 60 to prevent PD (i.e., even before PD is diagnosed or symptoms occur).

In the context of the present invention, PD can be, inter alia: idiopathic (IDP), juvenile (parkinsonism beginning during childhood or adolescence; early-onset parkinsonism (with onset between ages 21 and 40 years is sometimes called young or early-onset Parkinson disease); secondary parkinsonism (brain dysfunction that is characterized by basal ganglia dopaminergic blockade and that is similar to Parkinson disease, but it is caused by something other than Parkinson disease (e.g., drugs, cerebrovascular disease, trauma, postencephalitic changes)); atypical parkinsonism (a group of neurodegenerative disorders that have some features similar to those of Parkinson disease but have some different clinical features, a worse prognosis, a modest or no response to levodopa, and a different pathology (e.g., neurodegenerative disorders such as multiple system atrophy, progressive supranuclear palsy, dementia with Lewy bodies, and corticobasal ganglionic degeneration)); vascular parkinsonism (also known as arteriosclerotic parkinsonism, affecting people with restricted blood supply to the brain); drug-induced parkinsonism (parkinsonism caused by drugs, e.g., neuroleptic drugs (used to treat schizophrenia and other psychotic disorders), which block the action of the dopamine in the brain); multiple system atrophy (MSA); progressive supranuclear palsy (PSP, including Steele-Richardson-Olszewski syndrome); and/or monogenic PD/parkinsonism, for example caused by mutations in the genes: *LRRK2, SNCA, PRKN, PINK1, DJ-1, VPS35.* That is, the PD can be monogenic PD wherein the subject has one or more mutation(s) in genes linked to monogenic PD in the genes *SNCA, LRRK2, VPS35, PRKN,* and/or *PINK1* as described further below; preferably, definitely or probably pathogenic mutation(s); more preferably, definitely pathogenic mutation(s).

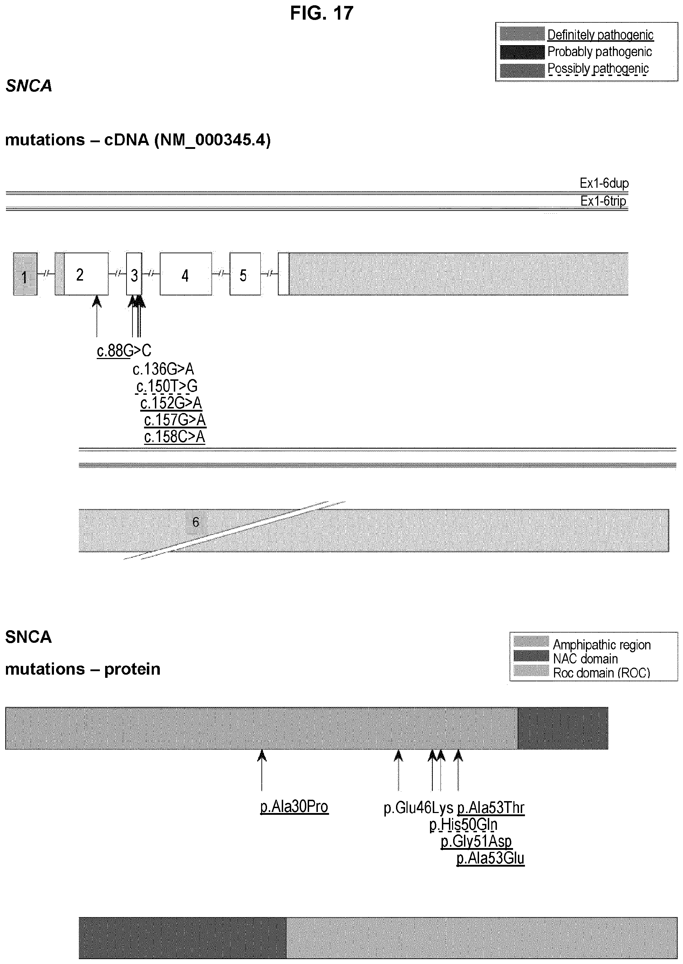

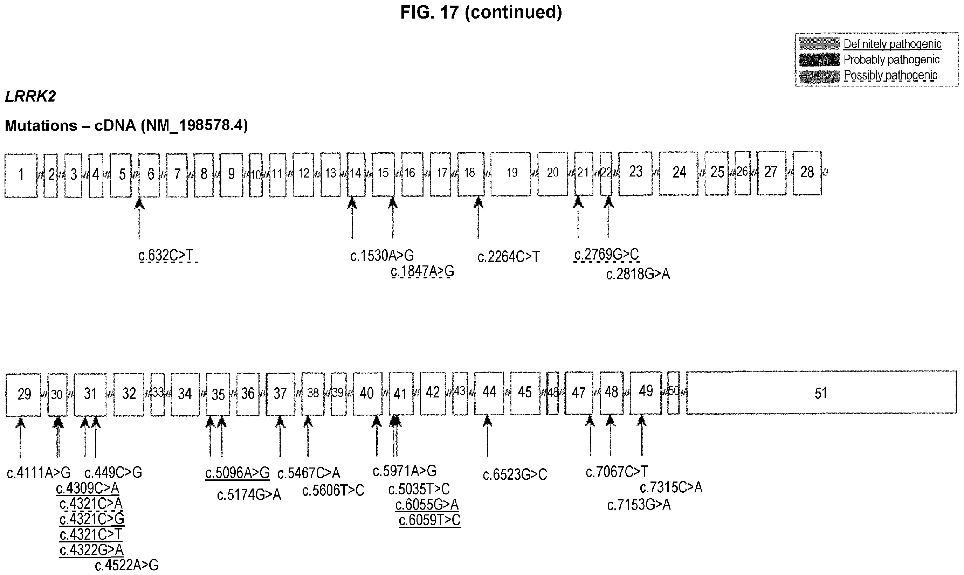

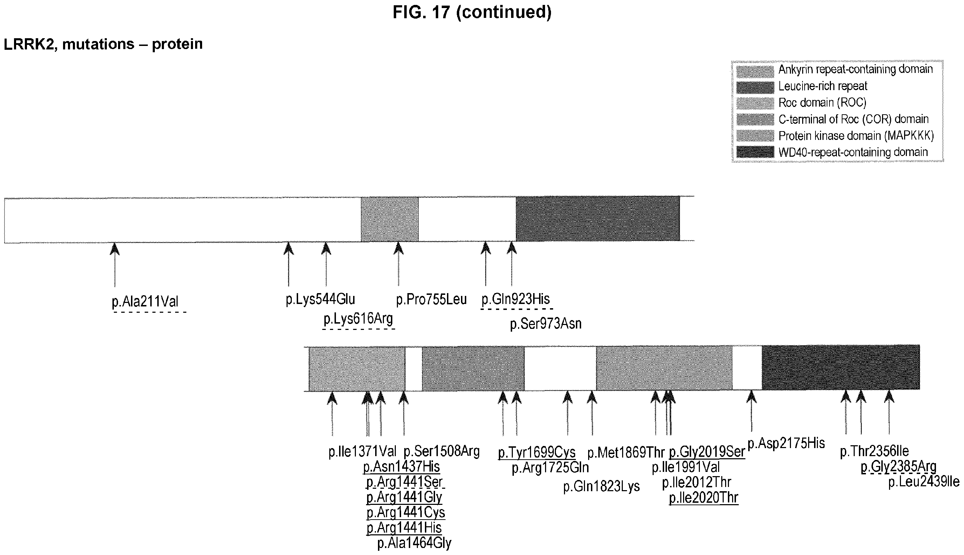

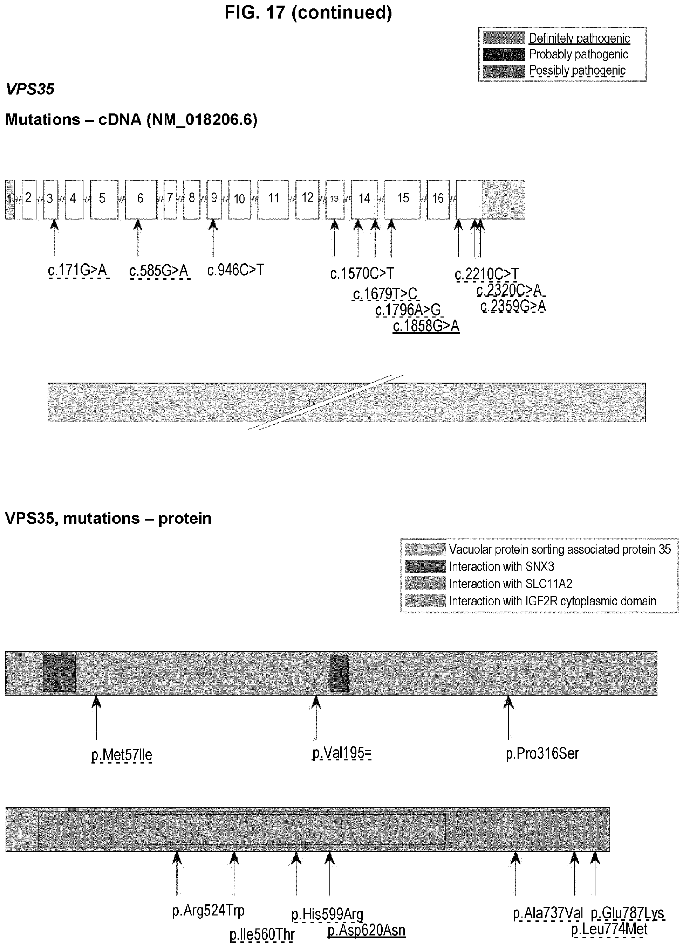

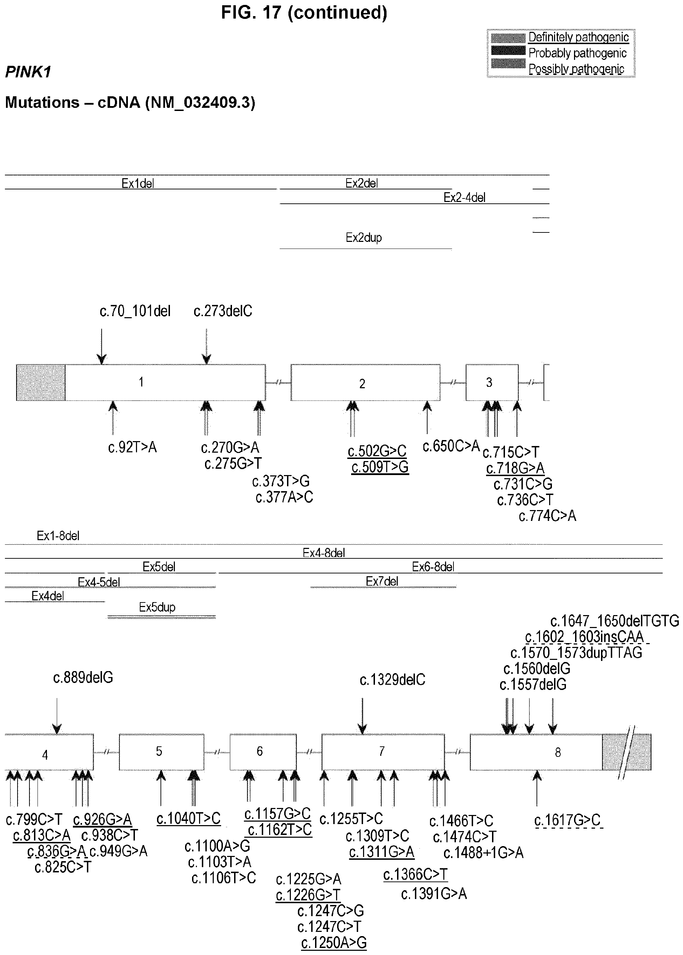

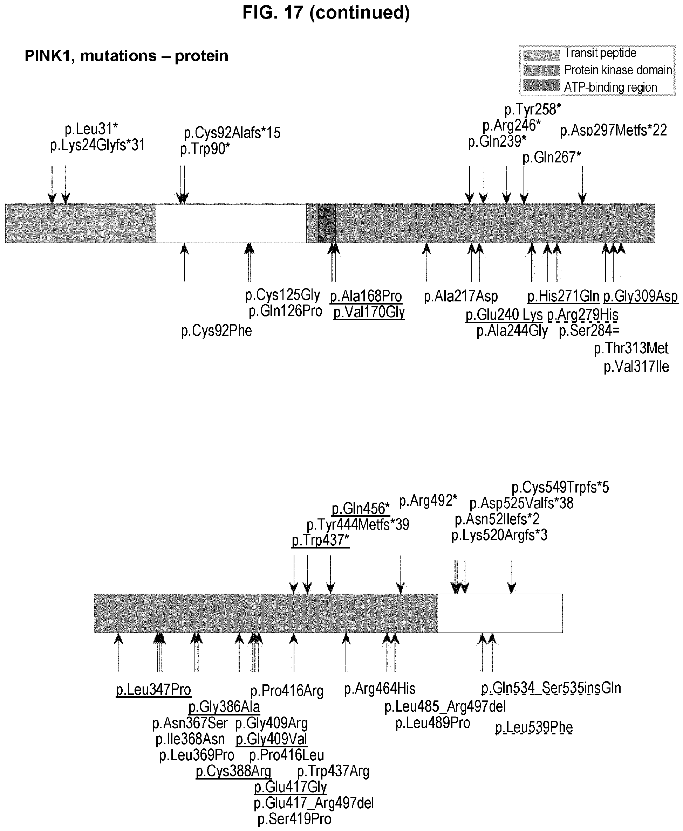

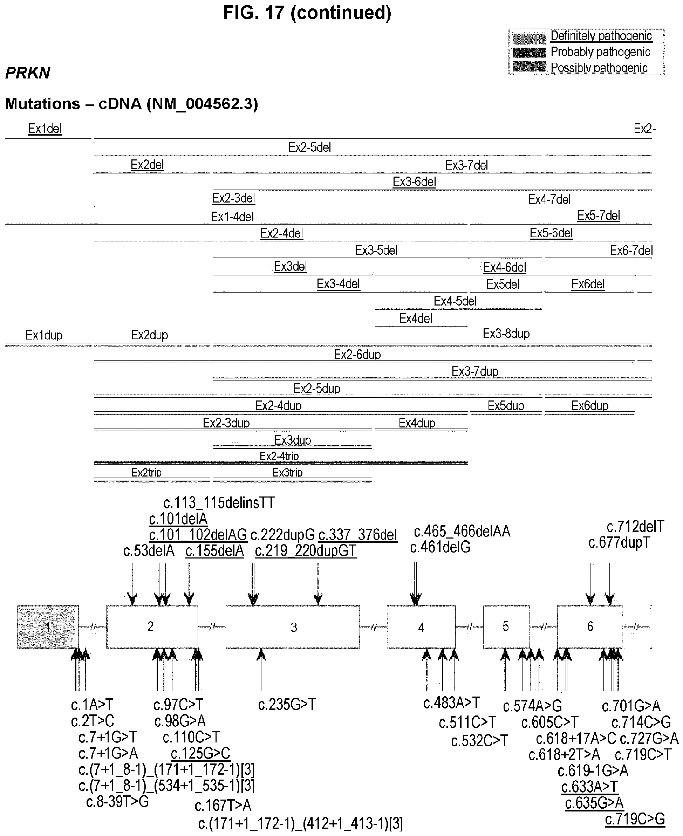

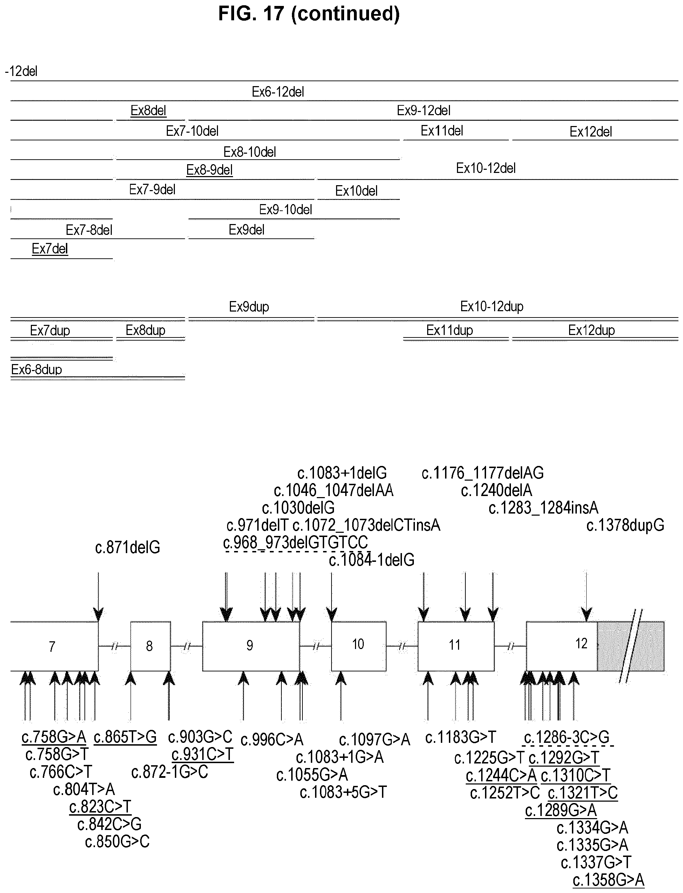

Preferably, the subject does not have mutations in genes linked to monogenic PD; more preferably, the subject has no pathogenic mutations, even more preferably no definitely and probably pathogenic mutations, in the genes *SNCA, LRRK2, VPS35, PRKN,* and/or *PINK1.* For instance, the subjects intended to be treated can be genetically characterized using RNA-sequencing data from blood cells (PBMCs) and/or muscle. Using this data, the patients can be assessed for known or novel established or potentially pathogenic mutations in all genes linked to monogenic PD, e.g.: SNCA (SEQ ID NOs: 1, 6), LRRK2 (SEQ ID NOs: 2, 7), VPS35 (SEQ ID NOs: 3, 8), PRKN (SEQ ID NOs: 4, 9), PINK1 (SEQ ID NOs: 5, 10). Canonical sequences of these proteins are shown in SEQ ID NO: 1 to 5, and the corresponding cDNA sequences are shown in SEQ ID NO: 6 to 10, respectively; exemplary pathogenic mutations are shown in **FIG. 17**. The presence of such mutations can be detected, in a DNA sample, e.g., from whole blood, muscle biopsy tissue and/or PBMC homogenate, by Sanger sequencing, SNP-Chip, rtPCR, or by other methods known in the art, e.g., as described in the Examples.For example, the subject having early PD may be diagnosed with PD within 1, 2, 3, 4 or 5, preferably 2 years or less before start of treatment. Diagnosis can be performed, for instance, according to the MDS clinical diagnostic criteria for Parkinson's disease, as explained above. Preferably, the subject's first PD symptom has been observed 50 months or less, preferably 45 months or less, more preferably 40 months or less, more preferably 30 months or less, more preferably 35 months or less, more preferably 25 months or less, more preferably 20 months or less, more preferably 15 months or less, more preferably 10 months or less, most preferably 5 months or less before begin of treatment.

According to an embodiment, the subject is newly diagnosed with PD, 1, 2, 3, 4 or 5, preferably 2 years before beginning of therapy. Preferably, the subject's first PD symptom has been observed 50 months or less, preferably 45 months or less, more preferably 40 months or less, more preferably 30 months or less, more preferably 35 months or less, more preferably 25 months or less, more preferably 20 months or less, more preferably 15 months or less, more preferably 10 months or less, most preferably 5 months or less since first PD symptom). Preferably, the subject is drug naive with respect to dopaminergic treatment; and/or has a clinical diagnosis of idiopathic PD according to the Movement Disorder Society Clinical Diagnostic Criteria for PD. Preferably, [¹²³I]FP-CIT single photon emission CT (DaTscan) of the subject confirms nigrostriatal denervation; and/or magnetic resonance imaging (MRI) is not suggestive of atypical parkinsonism; and/or the subject does not have dementia or other neurological disorder at baseline visit; and/or does not have a metabolic, neoplastic, or other physically or mentally debilitating disorder at baseline visit.

Preferably, the subject is not diagnosed with or does not suffer from one or more of the following: dementia or other neurodegenerative disorder at start of treatment; atypical parkinsonism, in particular PSP, MSA, corticobasal degeneration CBD); or vascular parkinsonism; a psychiatric disorder that would interfere with compliance; a severe somatic illness that would make the individual unable to comply; a metabolic, neoplastic, or other physically or mentally debilitating disorder at start of treatment; and/or a genetically confirmed mitochondrial disease. Optionally, the subject has not used vitamin B3 supplementation, e.g., high dose supplementation, within 30 days before start of treatment. According to one preferred embodiment, the PD is idiopathic (IPD). In the context of the present invention, a subject with idiopathic PD may have a clinical diagnosis according to the MDS clinical diagnostic criteria for Parkinson's disease, as detailed in Postuma RB, Berg D, Stern M, et al. MDS clinical diagnostic criteria for Parkinson's disease. Mov Disord 2015; 30(12): 1591-601 (see in particular Table 1 therein; a clinical diagnosis requires: 1. absence of absolute exclusion criteria; 2. at least two supportive criteria, and 3. no red flags).According to another preferred embodiment, the PD is secondary parkinsonism or drug-induced parkinsonism.

According to an embodiment, the subject has nigrostriatal degeneration or denervation. This can be confirmed by a positive [¹²³I]FP-CIT single photon emission CT (DaTscan).

According to an embodiment, the subject has a Hoehn and Yahr score < 3 at start of treatment. The Hoehn & Yahr score is assessed by the Hoehn & Yahr stage in MDS-UPDRS (The MDS-sponsored Revision of the Unified Parkinson's Disease Rating Scale. International Parkinson and Movement Disorder Society (2008), last updated August 13, 2019; https://www.movementdisorders.org/MDS-Files1/PDFs/Rating-Scales/MDS-UPDRS_English_FINAL_Updated_August2019.pdf). Accordingly, the Hoehn & Yahr stages are 0: asymptomatic, 1: unilateral involvement only, 2: bilateral involvement without impairment of balance, 3: mild to moderate involvement; some postural instability but physically independent; needs assistance to recover from pull test, 4: severe disability; still able to walk or stand unassisted, 5: wheelchair bound or bedridden unless aided. Preferably, the subject has had optimal symptomatic therapy, not requiring adjustments, for at least 1 month before start of treatment.

According to an embodiment, the treatment involves an increase in the subject's cerebral NAD levels. NAD is the central redox coenzyme in cellular metabolism and critical supplier of energy equivalents to the respiratory chain. By regulating the activity of the deacetylase enzymes known as sirtuins, NAD regulates several fundamental cellular events including histone acetylation and, by extension, gene expression. The signalling turnover of NAD in human cells is strikingly high with the entire cellular NAD pool being renewed at least once a day. This rapid turnover means that decreased NAD synthesis, as our findings suggest occurs in the PD brain, can have a profound and immediate impact on neuronal metabolism, including ATP deficiency, altered gene expression and compromised neuronal function and survival. The present inventors believe that increase of cerebral/neuronal NAD can improve mitochondrial function, restore sirtuin activity and histone acetylation status and rescue neuronal dysfunction and death in PD. Surprisingly, nicotinamide riboside for use according to the present invention is capable of passing the blood-brain barrier, and effectively increase the cerebral NAD levels.

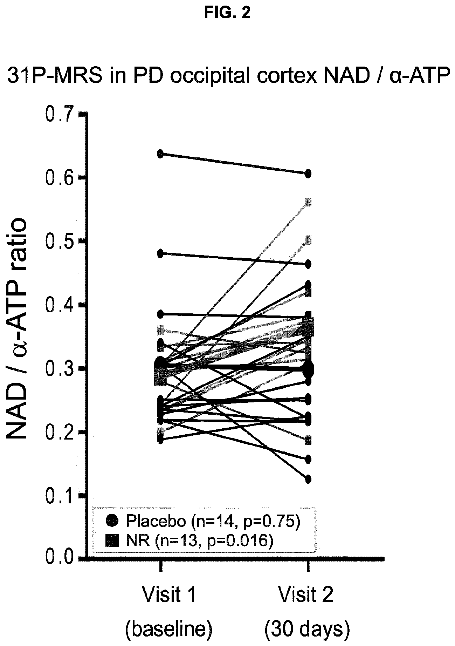

NAD supplementation therapy with nicotinamide riboside according was studied in a clinical trial. In vivo levels of cerebral NAD can be measured using phosphorus magnetic resonance spectroscopy 31P-MRS acquisition (31P-MRS). The study showed excellent compliance, tolerability and no signs of toxicity or adverse side effects. Surprisingly, 31P-MRS of the brain showed a highly significant increase in NAD levels in the NR group compared to the placebo group **(****FIG. 2****)**. These results suggest that NR is a safe and well-tolerated therapy in PD and leads to substantial NAD supplementation in the brain, the target organ of the disease. The increase in the subject's cerebral NAD levels can be defined as the difference between the mean NAD/α-ATP molar ratio, e.g., as measured by ³¹P-MRS between treatment group and placebo group; in the occipital cortex; and/or over at least 30 days. Preferably, an increase of 0.01 to 0.03, more preferably 0.01, 0.02 or 0.03 or more occurs. Yet further preferably, an increase of 0.03 or more, more preferably 0.07 or more, occurs in the NAD/α-ATP molar ratio as measured by ³¹P-MRS. Even more preferably, the increase occurs in the subject's occipital cortex over 30 days. Alternatively, when the increase in NAD/α-ATP molar ratio relative to baseline level is considered, this relative change preferably exceeds 10% of the baseline level.

According to an embodiment, subjects to be treated can be classified in subgroups based on their cerebral NAD-response, measured by 31P-MRS, upon nicotinamide riboside administration. Subjects showing increased cerebral NAD levels (e.g., after 4 weeks of nicotinamide riboside administration) are termed "MRS-responders," whereas subjects showing no increase in cerebral NAD levels are termed "MRS non-responders". Subjects belonging to MRS-responders subgroup may show not only increased NAD levels upon administration of nicotinamide riboside, but also particularly significant clinical improvement of PD. The clinical improvement may be further enhanced when the increase in the subject's cerebral NAD levels is >10% from baseline to 4 weeks of nicotinamide riboside administration.

By measuring the change in cerebral NAD levels, e.g., over 4 weeks, it is possible to identify a preferred patient subgroup. This subgroup may profit particularly well from the treatment, for instance with regard to symptomatic effects, such as change in MDS-UPDRS

According to an embodiment, the treatment impacts the neurometabolic profile of patients with PD, based on neuroimaging measures (FDG-PET, 31P-MRS), as described in Example 2.

According to an embodiment, the treatment increases the average total NAD levels in PBMCs relative to baseline before the treatment. Preferably, the average total NAD levels in PBMCs within 27-33, preferably 30 days are increased relative to baseline before the treatment. The average total NAD levels, as used herein, preferably refer to the group average within the group of patients/subjects, and can be measured, e.g., by metabolomic approaches, such as HPLC/MS, and/or by spectroscopic approaches, such as 31P-MRS. According to an embodiment, the treatment improves clinical symptoms measured using MDS-UPDRS, and/or augments the NAD metabolome in peripheral tissue of PD patients, as described in Example 2. These effects are preferably observed in subjects showing increased cerebral NAD levels after 4 weeks of nicotinamide riboside administration.

According to an embodiment, the treatment involves bilateral metabolic reductions in the caudate and putamen, extending into the adjacent globus pallidus, and in the thalamus, as determined by FDG-PET. Preferably, these changes are also associated with localized cortical reductions along the medial wall of the hemisphere involving the precuneus (BA 7), medial frontal cortex (BA 9, 10), anterior cingulate area (BA 24, 32), and in the posterior cingulate gyrus (BA 31).

According to an embodiment, the treatment involves an increase in the NRRP subject score determined as described in Example 2. This can have the effect of improving UPDRS motor ratings.

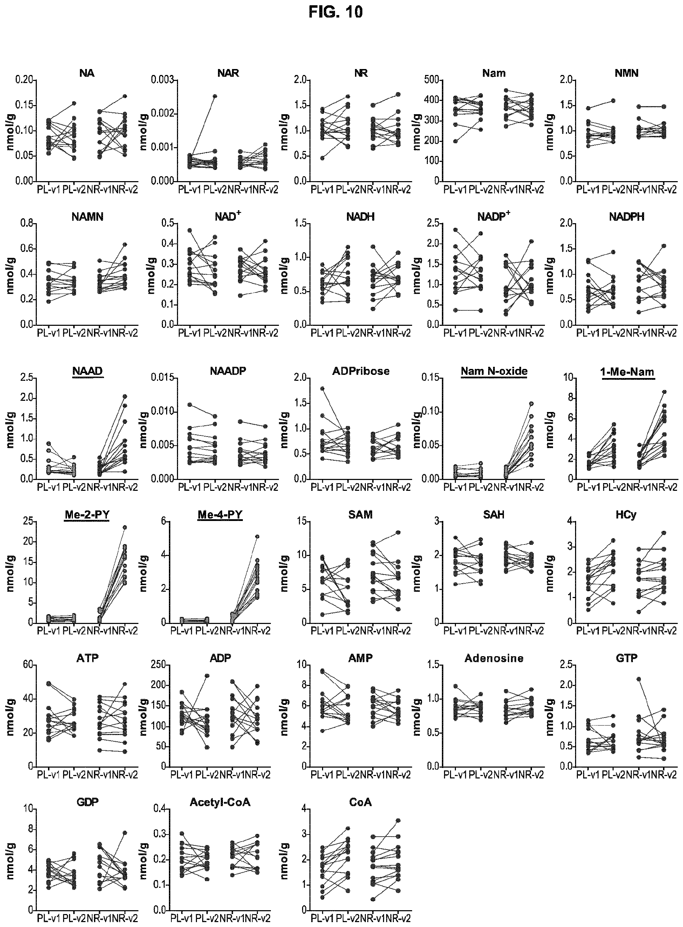

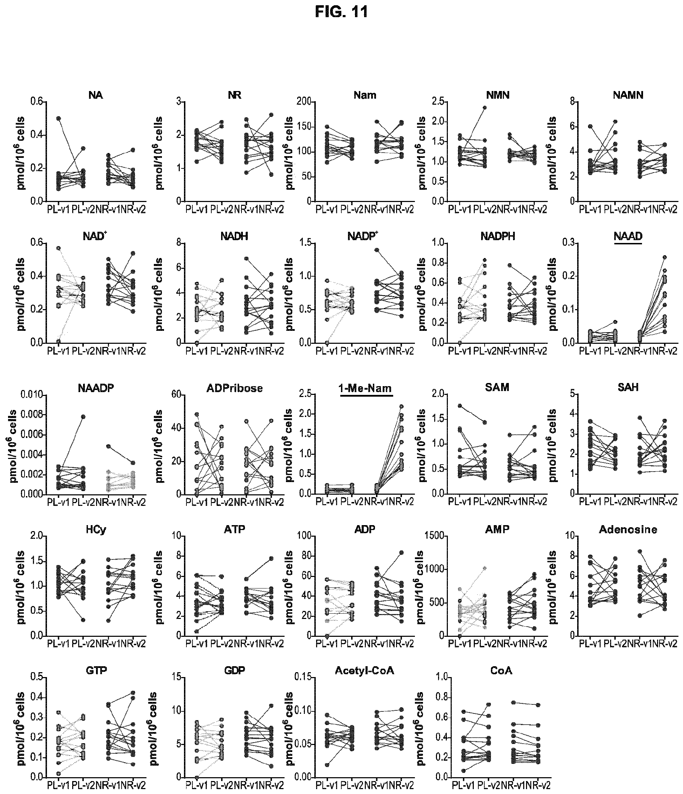

According to an embodiment, the treatment involves augmenting the NAD metabolome in peripheral tissues of PD patients, preferably by metabolomic analyses in skeletal muscle and PBMCs, e.g., as described in Example 2 and illustrated in **FIGS. 10** and **11****.**

According to an embodiment, the treatment induces the expression of mitochondrial, antioxidant, and proteostatic processes, e.g., as measured by out RNA-seq analysis in PBMCs and muscle tissue and described in Example 2. This can have the effect of upregulation of multiple biological processes, including ribosomal, proteasomal, lysosomal and mitochondrial function (oxidative phosphorylation).

While it has been hypothesised, based on animal models, that for long term NR treatment in patients or any individual with genetic or environmental reduction in 26S/20S function, may have adverse effects, this does not apply to the treatment of the present invention. Currently, no established single gene mutations or environmental factors directly disrupting the proteasome are known that cause PD in humans. Current evidence, e.g., from the studies of Examples 2 and 4, does not support the hypothesis that NR may adversely influence DA metabolism in patients with PD. And, in an optional embodiment, the subject to be treated has no genetic conditions or environmental risks known to influence the proteasome 26S/20S function.

According to an embodiment, the treatment involves reducing the levels of several inflammatory cytokines in serum and CSF.

According to an embodiment, the total score parts I, II, III and IV, or the total score of parts I, II and III according to the Movement Disorder Society Revision of the Unified Parkinson's Disease Rating Scale (MDS-UPDRS; see above) is improved. The MDS-UPDRS has four parts: Part I (non-motor experiences of daily living), Part II (motor experiences of daily living), Part III (motor examination) and Part IV (motor complications). Preferably, the improvement is an improvement in the mean difference in the total score after the treatment as compared to baseline before the treatment. Preferably, the improvement in the total MDS-UPDRS score according to parts I,-IV; or the total MDS-UPDRS score according to parts I-III; or any of the individual parts, is at least 1 point, more preferably 2 points.

According to an embodiment, a difference in the default restating state network in fMRI measurement is observed as compared to baseline before the treatment.

According to an embodiment, nicotinamide riboside for use in the methods any of the preceding embodiments or combinations thereof is provided, the method being for: a) improving motor, non-motor and/or cognitive symptoms; b) preventing motor, non-motor and/or cognitive symptoms; c) improving a score as measured by Part I, II, III or IV of MDS-UPDRS; d) improving the total score as measured by part I, II, III and IV of MDS-UPDRS; e) improving score measured by the Non-Motor Symptoms Questionnaire (NMSQ), by the Non-Motor Symptoms Scale for Parkinson's Disease (NMSS), on the Hoehn & Yahr scale, by the MoCA and/or EQ-5L; f) delaying nigrostriatal degeneration or denervation, and/or improving dopamine transporter density as measured by DaTscan; g) delaying brain atrophy (global or focal) as measured by MRI volumetry; h) lowering cortical atrophy as measured by cortical thickness with MRI; i) rectifying NAD metabolism and/or mitochondrial function as measured by multi-omics in patient biosamples and brain (31)P-MRS; j) correcting aberrant histone acetylation and gene expression profile as measured by multi-omics in patient biosamples; k) improving brain spatiotemporal functional and/or structural connectivity as measured by fMRI; I) delaying progression of neuronal loss, as measured by neurofilament light-chain in patient serum; and/or m) improving the score according to the MDS Non-Motor Rating Scale (MDS-NMS).

Preferably, the improved or prevented motor, non-motor and/or cognitive symptoms are one or more of the symptoms/criteria according to the MDS-UPDRS:
Part I - cognitive impairment; hallucinations and psychosis; depressed mood; anxious mood; apathy; features of dopamine dysregulation syndrome (DDS); sleep problems; daytime sleepiness; pain and other sensations; urinary problems; constipation problems; light headedness on standing; fatigue;
Part II - speech; saliva and drooling; chewing and swallowing; eating tasks; dressing; hygiene; handwriting; doing hobbies and other activities; turning in bed; tremor; getting out of bed; walking and balance; freezing; is the patient on medication?; patient's clinical state (OFF: typical functional state when patients have a poor response in spite of taking mediation or the typical functional response when patients are on no treatment for parkinsonism; ON: typical functional state when patients are receiving medication and have a good response); is the patient on levodopa?; if yes, minutes since last dose;
Part III - speech; facial expression; rigidity- neck; rigidity- RUE; rigidity- LUE; rigidity- RLE; rigidity-LLE; finger tapping- right hand; finger tapping- left hand; hand movements- right hand; hand movements- left hand; pronation- supination movements- right hand; pronation- supination movements-left hand; toe tapping- right foot; toe tapping- left foot; leg agility- right leg; leg agility- left leg; arising from chair; gait; freezing of gait; postural stability; posture; global spontaneity of movement; postural tremor- right hand; postural tremor- left hand; kinetic tremor- right hand; kinetic tremor- left hand; Rest tremor amplitude- RUE; Rest tremor amplitude- LUE; Rest tremor amplitude- RLE; Rest tremor amplitude- LLE; rest tremor amplitude- lip/jaw; constancy of rest tremor; were dyskinesias present?; did these movements interfere with ratings?; Hoehn and Yahr Stage;
Part IV - time spent with dyskinesias; functional impact of dyskinesias; time spent in the OFF state; functional impact of fluctuations; complexity of motor fluctuations; painful OFF-state dystonia.

Preferably, the improved or prevented motor symptoms are one or more of the symptoms/criteria according to more of the symptoms/criteria according to Parts II, III and IV of MDS-UPDRS. Preferably, the non-motor and/or cognitive symptoms are one or more of the symptoms/criteria according to Part I MDS-UPDRS.

Preferably, the improved or prevented non-motor symptoms are one or more of the symptoms/criteria according to the Non-Motor Symptoms Questionnaire (NMSQ: PD NMS QUESTIONNAIRE developed and validated by the International PD Non Motor Group (2006), International Parkinson and Movement Disorder Society, https://www.movementdisorders.org/MDS-Files1/Education/Rating-Scales/NMSQ.pdf):
Dribbling of saliva during the daytime; Loss or change in ability to taste or smell; Difficulty swallowing food or drink or problems with choking; Vomiting or feelings of sickness (nausea); Constipation (less than 3 bowel movements a week) or having to strain to pass a stool (faeces); Bowel (fecal) incontinence; Feeling that bowel emptying is incomplete after having been to the toilet; A sense of urgency to pass urine makes the subject rush to the toilet; getting up regularly at night to pass urine; Unexplained pains (not due to known conditions such as arthritis); Unexplained change in weight (not due to change in diet); Problems remembering things that have happened recently or forgetting to do things; Loss of interest in what is happening around the subject or doing things; Seeing or hearing things that the subject knows or is told are not there; Difficulty concentrating or staying focussed; Feeling sad, 'low' or 'blue'; Feeling anxious, frightened or panicky; Feeling less interested in sex or more interested in sex; Finding it difficult to have sex when you try; Feeling light headed, dizzy or weak standing; from sitting or lying; Falling; Finding it difficult to stay awake during activities, such as working, driving or eating; Difficulty getting to sleep at night or staying asleep at night; Intense, vivid dreams or frightening dreams; Talking or moving about in your sleep as if the subject is 'acting' out a dream; Unpleasant sensations in your legs at night or; while resting, and a feeling that you need to move; Swelling of the subject's legs;. Excessive sweating; Double vision; believing things are happening to the subject that other people say are not true).

Preferably, the improved or prevented non-motor symptoms are one or more of the symptoms/criteria according to the Non-Motor Symptoms Scale for Parkinson's Disease (NMSS: Non-Motor Symptom assessment scale for Parkinson's Disease developed by the International Parkinson's Disease Non-Motor Group (2007). International Parkinson and Movement Disorder Society, (https://www.movementdisorders.org/MDS-Files1/PDFs/Rating-Scales/NMSS.pdf):
*Domain 1: Cardiovascular including falls* - light-headedness, dizziness, weakness on standing from sitting or lying position; falling because of fainting or blacking out;
*Domain 2: Sleep*/*fatigue* - dozing off or fall asleep unintentionally during daytime activities; fatigue (tiredness) or lack of energy (not slowness) limit the patient's daytime activities; having difficulties falling or staying asleep; an urge to move the legs or restlessness in legs that improves with movement when he/she is sitting or lying down inactive; *Domain 3: Mood* /*Cognition* - loss of interest in his/her surroundings; loss of interest in doing things or lack motivation to start new activities; feeling nervous, worried or frightened for no apparent reason; seeming sad or depressed or has he/she reported such feelings; having flat moods without the normal "highs" and " lows"; having difficulty in experiencing pleasure from their usual activities or report that they lack pleasure;
*Domain 4: Perceptual problems*/*hallucinations* - the subject indicates that he/she sees things that are not there; the subject having beliefs that he/she know are not true; double vision (2 separate real objects and not blurred vision);
*Domain 5: Attention*/*Memory* - having problems sustaining concentration during activities; forgetting things that he/she has been told a short time ago or events that happened in the last few days; forgetting to do things;
*Domain 6: Gastrointestinal tract* - dribbling saliva during the day; having difficulty swallowing; suffering from constipation (Bowel action less than three times weekly);
*Domain* 7: *Urinary* - having difficulty holding urine (Urgency); having to void within 2 hours of last voiding (Frequency); having to get up regularly at night to pass urine (Nocturia);
*Domain 8: Sexual function* - having altered interest in sex; (Very much increased or decreased, please underline); having problems having sex;
*Domain 9: Miscellaneous* - suffering from pain not explained by other known conditions; reporting a change in ability to taste or smell; reporting a recent change in weight (not related to dieting); excessive sweating (not related to hot weather).

Preferably, the score improvement on the Hoehn & Yahr scale is assessed by the by the Hoehn & Yahr stage as given in MDS-UPDRS, i.e.: 0: asymptomatic, 1: unilateral involvement only, 2: bilateral involvement without impairment of balance, 3: mild to moderate involvement; some postural instability but physically independent; needs assistance to recover from pull test, 4: severe disability; still able to walk or stand unassisted, 5: wheelchair bound or bedridden unless aided.

More preferably, the improved or prevented non-motor symptoms are one or more of the symptoms/criteria according to the MDS Non-Motor Rating Scale (MDS-NMS, . Ray Chaudhuri, Anette Schrag, Daniel Weintraub, Alexandra Rizos, Carmen Rodriguez-Blazquez, Eugenia Mamikonyan and Pablo Martinez-Martin (2019): The International Parkinson and Movement Disorder Society - Non-Motor Rating Scale, https://www.movementdisorders.org/MDS-Files1/PDFs/Rating-Scales/MDS-NMS_FINAL.pdf accessed on June 22, 2021).

Yet more preferably, the improvement or prevention of non-motor symptoms refers to improving or preventing deterioration in the MDS-NMS non-motor fluctuations total score.

Preferably, the cognitive symptoms are one or more of the symptoms/criteria according to the Montreal Cognitive Assessment (MoCA; Z. Nasreddine MD Version November 7, 2004, https://www.parkinsons.va.gov/resources/MOCA-Test-English.pdf accessed on June 22, 2021).

Preferably, the subject's quality of life assessed according to the EQ-5D-5L questionnaire is improved. EQ-5D-5L is the five-level version of EQ-5D, as described by Herdman M, Gudex C, Lloyd A, et al. Qual Life Res. 2011;20(10):1727-1736. Preferably, the Sample UK English EQ-5D-5L is used (https://euroqol.org/eq-5d-instruments/sample-demo/, accessed on June 22, 2021: https://euroqol.org/wp-content/uploads/2020/09/Sample_UK-English-EQ-5D-5L-Paper-Self-Complete-v1.2-ID-24700.pdf).

Genome-wide aberrant histone hyperacetylation and altered transcriptional regulation occur in the brain of individuals with PD. Preferably, the subject's aberrant histone acetylation is characterised by increased acetylation of multiple sites on histones H2B, H3 and H4, more preferably, H3K27 hyperacetylation (H3K27ac) as compared to a control subject not suffering from PD. Preferably, the aberrant gene expression profile is characterised by increased levels of SIRT1 and SIRT3 proteins in the brain as compared to a control subject not suffering from PD. Without wishing to be bound by theory, it is possible that NR may mitigate epigenomic dysregulation in PD, by regulating histone acetylation. Increasing neuronal NAD levels may boost the activity of the NAD-dependent histone deacetylases of the sirtuin family, potentially ameliorating histone hyperacetylation in PD.

According to an embodiment, the treatment acts as a neuroprotective, disease modifying therapy for PD dementia (PDD) and/or dementia with Lewy bodies (DLB). PDD and DLB are characterized by widespread neuronal death in both cortical and subcortical areas. The neuronal loss occurs and progresses over several years after the patient is diagnosed. There is, therefore, a substantial neuronal pool that could be rescued if the NR intervention starts as early as possible. Based on the present findings, there is a good technical rationale that NR is suitable as neuroprotective, disease modifying therapy for PDD and DLB. Based on the present study results, as well as a body of preclinical evidence for NR-mediated neuroprotection, and without wishing to be bound by theory, it is considered that NR may increase neuronal resilience in the face of cellular stress, including but not limited to: mitochondrial respiratory dysfunction, free radical damage, aberrant lysosomal and/or proteasomal function, neuroinflammation, pathological protein aggregation such as α-synuclein, tau, TDP-43 and beta-amyloid. By increasing neuronal resilience and, therefore, survival, NR exerts a neuroprotective action, delaying and/or preventing the death of neurons. According to an embodiment, the subject is of age equal to or greater than 35 years at begin of treatment; preferably equal to or greater than 65 years, more preferably 35 to 85, more preferably 40 to 80, more preferably 55 to 75, more preferably 60 to 75, most preferably 65 to 75 years.

According to an embodiment, the treatment is not associated with an increase in the prevalence of glioma and/or any other brain neoplasms. Preferably, the patient over the duration of the treatment does not develop glioma and/or any other brain neoplasms.

According to an embodiment, the preferred route of NR administration is oral. Alternatively or additionally, nicotinamide riboside may be administered by other suitable route(s).

According to an embodiment, nicotinamide riboside is administered to the subject at a total dosage of 200 to 2000 or 500 to 2000, preferably 800 to 1200 mg per day, more preferably 1000 mg per day. Such total dosage is within the approved range for use in the human population. NR is well tolerated with no evidence of toxicity in adult humans with doses up to at least 2000 mg daily.

According to an embodiment, nicotinamide riboside is administered to the subject at a dosage of more than 2000 mg and up to 3000 mg per day, preferably 3000 mg per day. Without wishing to be bound by theory, we consider that oral administration of the NAD precursor NR in dosages of up to 3000 mg daily is unlikely to cause moderate or severe side effects as measured biochemically and physiologically, e.g., when administered short-term for 4 week; and that it is unlikely to have significant tolerability issues for treated individuals.

According to an embodiment, nicotinamide riboside is administered to the subject at a total dosage of 200 to 3000, preferably of 500 to 3000 mg, more preferably 800 to 3000 mg per day, even more preferably 1000 to 3000 mg per day, most preferably 1000 or 3000 per day.

According to an embodiment, nicotinamide riboside is administered to the subject at a total dosage of 200 to 3000 mg per day, preferably 500 to 3000 mg per day or 1200 to 3000 mg per day, more preferably 1000 to 3000 mg per day, most preferably 1000 or 3000 mg per day.

Preferably, nicotinamide riboside is administered to the subject at a dosage of 400 to 800 mg twice per day, preferably 500 mg twice per day, more preferably 2 × 250 mg twice per day. More preferably, nicotinamide riboside can be administered twice per day, e.g., 500 mg twice per day, or 2 × 250 mg twice per day, to ensure a stable bioavailable dosage throughout the day. Alternatively, nicotinamide riboside is administered to the subject at a dosage of more than 1000 mg and up to 1500 mg twice per day, preferably 1500 mg twice per day, more preferably 6 × 250 mg twice per day.

In the context of the present invention, "nicotinamide riboside" or "nicotinamide riboside active ingredient" generally refers to its cationic form, i.e. C₁₁H₁₅N₂O₅⁺, present in salt form with any suitable counterion. Preferably, or unless specified otherwise, weights are expressed as nicotinamide riboside chloride, i.e. C₁₁H₁₅N₂O₅Cl (M = 290.7 g/mol). If a counterion X⁻ other than chloride is present, the respective NR weight is to be recalculated based on the ratio of molar weights of C₁₁H₁₅N₂O₅X and C₁₁H₁₅N₂O₅Cl.

According to an embodiment, nicotinamide riboside is administered in combination with a dopaminergic agent and/or MAO-B inhibitor. Preferably, the MAO-B inhibitor comprises or is selegiline. Preferably, the dopaminergic agent comprises or is levodopa in combination with a decarboxylase inhibitor such as carbidopa or benserazide, with or without the addition of a COMT-inhibitor such as entacapone or tolcapone. Alternatively or additionally, the dopaminergic agent may be a dopamine-agonist such as pramipexole, ropinirole, rotigotine, bromocriptine or pergolide. In one preferred aspect of this embodiment, 8-12 mg, preferably 10 mg selegiline is administered to the subject per day; and 150-800-mg, preferably 300-450 mg levodopa, and 37.5-200 mg, preferably 75-112.5 mg carbidopa are administered to the subject per day; more preferably, 10 mg selegiline is administered to the subject once per day; and 100 mg levodopa and 25 mg carbidopa are each administered to the subject three times per day. In another preferred aspect of this embodiment, 8-12 mg, preferably 10 mg selegiline is administered to the subject per day; and 150-800- mg, preferably 300-450 mg levodopa, and 37.5-200 mg, preferably 75-112.5 mg benserazide are administered to the subject per day; more preferably, 10 mg selegiline is administered to the subject once per day; and 100 mg levodopa and 25 mg benserazide are each administered to the subject three times per day.

Therapeutic effects associated with the dopaminergic agent include motor symptom improvement (symptomatic).

Therapeutic effects associated with the MAO-B inhibitor include mild motor symptomatic effect + a very mild neuroprotective effect; MAO-B inhibitors have been shown of being are able to minimally delay disease progression.

Therapeutic effects associated with the combination of the dopaminergic agent with the MAO-B inhibitor include motor symptom control + very mild effect on disease progression.

Without wishing to be bound by theory, it is believed that NR exerts a therapeutic effect in humans both alone as well as in combination with a dopaminergic agent and/or a MAO-B inhibitor.

NR alone is believed to achieve neuroprotection and delay disease progression. However, it is unethical to do trials of novel agents in PD without also giving dopaminergic agent + MAO-B to the patients, so these agents have to be additionally given in clinical studies.

NR may be combined with dopaminergic therapy to also provide adequate motor symptom control to patients, i.e.: NR delays/ameliorates the disease; dopaminergic therapy controls existing symptoms. That is, while NR is believed delay/arrest disease progression and even improve symptoms, dopaminergic agents would still add benefit by providing motor symptom control. On the other hand, if patients start in the presymptomatic or premotor phase, and NR arrests further progression, then dopaminergic treatment may not be necessary.

NR may be combined with a MAO-B inhibitor to offer the patient the combined benefits, i.e.: potentially even greater delay of disease progression.

Again, without wishing to be bound by theory, it is believed that the combination of NR + dopaminergic agent + MAO-B is particularly advantageous, from the viewpoints of: (a) providing neuroprotection and substantial effect on disease progression by NR, (b) augmented this effect by the mild but certain effect of the MAO-B, and (c) controlling the motor symptoms by the dopaminergic agent.

According to an embodiment, the duration of the treatment is at least 1, 2, 6 or 12 months, preferably 52 weeks. The maximum duration of the treatment is not particularly limited, and can be for the entire remaining life of the patient.

According to an embodiment, the nicotinamide riboside is administered as a monotherapy, or as a monotherapy in combination with a dopaminergic agent plus MAO-B inhibitor. According to an embodiment, nicotinamide riboside is administered as a pharmaceutically acceptable salt, solvate and/or hydrate thereof.

In the context of the present invention, the term "pharmaceutically acceptable" refers to a compound, ingredient or ion acceptable for use in medicine and health care. Salts, hydrates and solvates which are suitable for use in medicine are those wherein the counter-ion or associated solvent is pharmaceutically acceptable. However, salts, hydrates and solvates having non-pharmaceutically acceptable counter-ions or associated solvents are within the scope of the present invention, for example, for use as intermediates in the preparation of other compounds and their pharmaceutically acceptable salts, hydrates and solvates. Suitable salts according to the invention include those formed with either organic and inorganic acids or bases.

Preferably, the salt is selected from fluoride, chloride, bromide, iodide, formate, acetate, ascorbate, aspartate, benzoate, butyrate, carbonate, citrate, carbamate, formate, gluconate, glutamate, lactate, malate, methyl bromide, methyl sulfate, nitrate, phosphate, propionate, diphosphate, succinate, sulfate, sulfonate, hydrogen tartrate, hydrogen malate, trifluoroacetate, tribromomethanesulfonate, trichloromethanesulfonate, and trifluoromethanesulfonate. Preferably, the salt is a halogenide. More preferably, the salt is a chloride. Alternatively, the salt can be an acidic NR+ salt of tartaric or malic acid, such as NR _{D}-hydrogen tartrate, NR _{L}-hydrogen tartrate, NR _{L}-hydrogen tartrate, or NR o-hydrogen tartrate.

Most preferably, the nicotinamide riboside is nicotinamide riboside chloride.

### Pharmaceutical composition

The present invention relates, in a second aspect, to a pharmaceutical composition, the composition comprising nicotinamide riboside as defined in any one of the above embodiments. The composition can be for use in a method for treatment according to any of the preceding embodiments or combinations thereof.

In the context of the present invention, the term "pharmaceutical composition" is intended to encompass a product comprising the claimed compound in therapeutically effective amounts, as well as any product that results, directly or indirectly, from combinations of the claimed compounds. Nicotinamide riboside may be incorporated with or without an excipient and used in the form of capsule, tablet, powder, granule, sachet, troche, pill, wafer, gelcap, elixir, suspension, syrup, drops, spray, inhaler, suppository, solution, injection solution, cream, ointment, lotion, gel, patch, depot, or the like.

Preferably, the pharmaceutical composition further comprises a pharmaceutically acceptable excipient. In the context of the present invention, the term "excipient" refers to a carrier, a binder, a disintegrator and/or a further suitable additive for galenic formulations, for instance, for liquid oral preparations, such as suspensions, elixirs and solutions; and/or for solid oral preparations, such as, for example, powders, capsules, gelcaps and tablets. Carriers, which can be added to the mixture, include necessary and inert pharmaceutical excipients, including, but not limited to, suitable suspending agents, lubricants, flavourings, sweeteners, preservatives, coatings, granulating agents, dyes, and colouring agents.

Preferably, the excipient(s) include one or more, more preferably each of microcrystalline cellulose, hydroxypropyl methylcellulose and magnesium stearate.

Preferably, the pharmaceutical composition comprises, more preferably consists of, nicotinamide riboside chloride, microcrystalline cellulose, hydroxypropyl methylcellulose and magnesium stearate.

Preferably, the pharmaceutical composition comprises nicotinamide riboside in an amount of about 0.001% to 100% by weight, more preferably about 0.01% to about 50% by weight, more preferably 0.1% to about 10% by weight.

Preferably, the nicotinamide riboside is not administered together with, and the pharmaceutical composition and the dosage form do not comprise one or more of, more preferably any of: epigallocatechin gallate (EGCG); ginsenoside Rg3 or a pharmaceutically acceptable salt thereof; Acetyl L-Carnitine HCL; R-Alpha Lipoic Acid; Rhodiola rosea; ancient peat and apple extract; denosinetriphosphate disodium; pterostilbene; a urolithin; pterostilbene; N-acetylcysteine, L-carnitine tartrate, and serine; a PARP inhibitor; and a mitochondrial uncoupler.

According to an embodiment, the composition comprises nicotinamide riboside as the sole active ingredient, or as the sole active ingredient in combination with one or more of a dopaminergic agent and MAO-B inhibitor.

### Dosage form

The present invention relates, in a third aspect, to a dosage form comprising nicotinamide riboside or a pharmaceutical composition as defined in any one of the embodiments above or combinations thereof. and can be for use in any in any one of the methods for treatment as defined above or combinations thereof.

The dosage form is preferably an oral dosage form.

The dosage form is preferably a capsule. Alternatively, further suitable dosage can be used, such as tablet, buccal tablet, pill, powder, granule, sachet, troche, lozenge, wafer, gelcap, suspension, syrup, elixir, drops, spray, gel, patch and depot.

Preferably, the dosage form comprises 50 to 2000 mg; more preferably 100 to 1000 mg; more preferably 100, 125, 250, 300, 500 or 1000 mg; most preferably 250 mg of nicotinamide riboside active ingredient per unit, and/or comprises nicotinamide riboside in an amount of about 0.001% to 100% by weight, more preferably about 0.01% to about 50% by weight, more preferably about 0.1% to about 10% by weight.

According to an embodiment, the dosage form is an oral capsule comprising, preferably consisting of, nicotinamide riboside chloride, microcrystalline cellulose, hydroxypropyl methylcellulose and magnesium stearate.

### Pharmaceutical combination

The present invention relates, in a third aspect, to pharmaceutical combination comprising nicotinamide riboside, a dopaminergic agent and a MAO-B inhibitor.

The MAO-B inhibitor preferably comprises or is selegiline. The dopaminergic agent preferably comprises or is levodopa in combination with a decarboxylase inhibitor such as carbidopa, or benserazide with or without the addition of a COMT-inhibitor such as entacapone or tolcapone. Alternatively or additionally, the dopaminergic agent may be a dopamine-agonist such as pramipexole, ropinirole, rotigotine, bromocriptine or pergolide.

As described in detail further above, the function or effect of the dopaminergic agent includes achieving motor symptom control.

As described in detail further above, the function or effect of the MAO-B inhibitor includes providing a mild neuroprotective effect, thereby delaying disease progression. Without wishing to be bound by theory, further improvements are expected by combining NR with a) agents further increasing NAD availability; b) agents activating the sirtuins (in particular SIRT1 and SIRT3); c) agents capable of replenishment of methylation equivalents, e.g., by combining NR with folate; and/or d) anti-oxidant treatments

According to an embodiment, the pharmaceutical combination comprises nicotinamide riboside, levodopa and carbidopa; or nicotinamide riboside, levodopa and benserazide.

According to one embodiment, nicotinamide riboside, a dopaminergic agent and a MAO-B inhibitor are simultaneously present within the same dosage form ("fixed-dose combination"). Alternatively, each active ingredient is provided in a separate dosage form, but they are nevertheless provided in combination (a "set" or "kit of parts"), e.g., within a common container, enclosure, or box, and/or are intended for administration in such way that a preferred dosage of each individual active ingredient, as explained above, is provided to the to the subject. Alternatively, at least one of the active ingredients is provided in a separate dosage form, whereas two or more of the remaining active ingredients are provided within the same dosage form; preferably, nicotinamide riboside and the MAO-B inhibitor can be provided as separate dosage forms, whereas the dopaminergic agent in the combination can be provided as a fixed dose combination, e.g., levodopa 100 mg + carbidopa 25 mg per unit; or levodopa 100 mg + benserazide 25 mg per unit, but the dosage forms are nevertheless provided in combination (a "set" or "kit of parts"), e.g., within a common container, enclosure, or box, and/or are intended for administration in such way that a preferred dosage of each individual active ingredient, as explained above, is provided to the to the subject.

According to one embodiment, the pharmaceutical combination may comprise separate unit dosage forms for each active ingredient, and instructions for administering the MAO-B inhibitor, preferably selegiline, more preferably in 10 mg unit dosage form(s), once per day; and the dopaminergic agent, preferably levodopa, carbidopa and/or benserazide, more preferably levodopa in 100 mg unit dosage form(s), carbidopa in 25 mg unit dosage form(s) and/or benserazide in 25 mg unit dosage form(s).

Preferably, the combination comprises:
(a) nicotinamide riboside, which preferably comprises or is nicotinamide riboside chloride, in dosage form(s) comprising 50 to 1200; more preferably 100 to 1000; more preferably 100, 125, 250, 300, 500 or 1000 mg; most preferably 250 mg of active ingredient per unit;
(b) MAO-B inhibitor, in dosage form(s) comprising 5 to 20; more preferably 8 to 12; most preferably 10 mg of selegiline per unit; and
(c) dopaminergic agent in dosage form(s) comprising:
   (i) 80-500 mg, preferably 100-300 mg, more preferably 100 mg levodopa per unit; and 20-130 mg, preferably 20-75 mg, more preferably 25 mg carbidopa per unit; or
   (ii) 80-500 mg, preferably 100-300 mg, more preferably 100 mg levodopa per unit; and 20-130 mg, preferably 20-75 mg, more preferably 25 mg benserazide per unit.

### Abbreviations and Definitions of Terms

| **Abbreviation or special term** | **Explanation** |
|---|---|
| 31P-MRS | Phospohorus-31 Magnetic Resonance Spectroscopy |
| AE | Adverse event |
| CBD | Corticobasal degeneration |
| CRF | Case report form (electronic/paper) |
| CSA | Clinical study agreement |
| CSI | Chemical shift imaging |
| CTC | Common toxicity criteria |
| CTCAE | Common terminology criteria for adverse event |
| DAE | Discontinuation due to adverse event |
| DaTscan | Dopamine transporter scan |
| EC | Ethics Committee, synonymous to Institutional Review Board (IRB) and Independent Ethics Committee (IEC) |
| FDG | 18F Fluorodeoxyglucose |
| GCP | Good clinical practice |
| IB | Investigator's brochure |
| ICF | Informed consent form |
| ICH | International Conference on Harmonization |
| IND | Investigational new drug |
| IP | Investigational product (includes active comparator and placebo) |
| MDS-UPDRS | Movement Disorders Unified Parkinson's Disease Rating Scale |
| MoCA | Montreal Cognitive Assessment |
| MRAC | Magnetic resonance based attenuation correction |
| MRC | Mitochondrial respiratory chain |
| mtDNA | mitochondrial DNA |
| NAD | Nicotinamide adenine dinucleotide |
| NAD (NAD⁺, NADH) | Nicotinamide adenine dinucleotide (oxidized, reduced form) |
| NMSQ | Non-Motor Symptoms Questionnaire |
| NMSS | Non-Motor Symptom assessment scale for Parkinson's Disease |
| NOE | Nuclear Overhauser effect |
| NR | Nicotinamide riboside |
| NRRP | NR-related metabolic pattern |
| OBD | Optimal biological dose |
| PD | Parkinson's Disease |
| PDRP | PD-related spatial covariance pattern |
| PET | Positron emission tomography |
| SAE | Serious adverse event |
| SD | Stable disease |
| SIRT | Sirtuin |
| SNc | Substantia nigra compacta |
| SOP | Standard operating procedure |

### EXAMPLES

The tolerability and cerebral bioavailability, efficacy, safety and particular effects of nicotinamide riboside in the treatment of Parkinson's disease (PD) are shown in the following examples.

### EXAMPLE 1: Tolerability and cerebral bioavailability of NAD supplementation therapy with NR in PD

A trial of tolerability and cerebral bioavailability of NAD supplementation therapy with nicotinamide riboside ("NR"), provided in the form of nicotinamide riboside chloride (Niagen^{®} from Chromadex) in PD patients was completed. In the trial, 30 individuals with newly diagnosed, drug naive PD were randomized and given NR 500 mg × 2/day or placebo for a period of 30 days. 31P-MRS was performed at baseline (Visit 1) and at on the last day of treatment (Visit 2). The scans of 27 individuals passed quality control at both visits.

In **FIG. 2**, the Y-axis shows measured levels of NAD normalized for α-ATP. Datapoints from individual subjects are connected with lines showing the change between baseline and 30 days. The bold points and lines show the mean of each group. The NR group is shown in grey (squares) and the placebo in black (dots). The treatment group shows a highly significant increase in cerebral NAD levels (n = 13, paired t-test p = 0.016), whereas no difference is observed in the placebo group (n = 14, paired t-test p = 0.75).

The study showed excellent compliance, tolerability and no signs of toxicity or adverse side effects. Intriguingly, 31P-MRS of the brain showed a highly significant increase in NAD levels in the NR group compared to the placebo group **(****FIG. 2****)**. These results suggest that NR is a safe and well-tolerated therapy in PD and leads to substantial NAD supplementation in the brain, the target organ of the disease.

### EXAMPLE 2: Randomized phase I trial of nicotinamide riboside supplementation in Parkinson's disease

### 1. Methods

### 1.1. Participants and study design

A single-center, double-blinded, randomized, placebo-controlled phase I trial of oral NR supplementation in newly diagnosed patients with PD was conducted. Eligible patients had a new clinical diagnosis of PD according to the Movement Disorder Society Clinical Diagnostic Criteria (Postuma RB, Berg D, Stern M, Poewe W, Olanow CW, Oertel W et al. MDS clinical diagnostic criteria for Parkinson's disease. Movement disorders: official journal of the Movement Disorder Society 2015; 30: 1591-601), Hoehn and Yahr Scale ≤ 3, pathological 1231-loflupane dopamine transporter imaging scan (DaTscan) confirming nigrostriatal denervation, no prior use of antiparkinson drugs, no clinical and/or biochemical signs of dementia, metabolic-, neoplastic-, or other physical or neurological disorders upon enrolment, and no signs of atypical parkinsonism or other neurological disease on magnetic resonance imaging (MRI) examination at Only two patients in our cohort of newly-diagnosed patients where below the age of 50 years, and of these one participant had disease onset below the age of 40 years. Due to the early onset, this patient underwent genetic investigation with whole-exome sequencing and SNP-chip based genome-wide copy number variation analysis, which excluded pathogenic mutations in PD-related genes, including PINK1 and PRKN. screening. Patient recruitment, inclusion, and follow-up was carried out by a GCP certified investigator. A full list of the inclusion and exclusion criteria is provided in Table 3. Sample size of 30 participants was chosen based on previously reported brain metabolic network analyses.

RNAseq from the study subjects were used to assess sequence variation in the genes linked to monogenic, recessive or dominant, forms of PD (LRRK2, SNCA, VPS35, PRKN, PINK1). Specifically, we assessed the data for definitely and probably pathogenic mutations in these genes, as previously reported (Kasten, M. et al. Mov Disord 33, 730-741 (2018); Trinh, J. et al. Mov Disord 33, 1857-1870 (2018)) and compiled in the www.mdsgene.org database. These analyses revealed no pathogenic mutations.

### 1.2. Randomization and masking

Thirty PD patients were randomly assigned (Block size 6, allocation 1:1) to 500 mg NR, provided in the form of nicotinamide riboside chloride (Niagen^{®} from Chromadex), or placebo twice per day, 12 hours apart, using the electronic Case Report Form (Viedoc.com). The drug containers were sequentially marked by an independent third party. The NR (Niagen^{®}) and placebo were provided by ChromaDex, USA. Each NR capsule contained 250 mg of NR. Placebo capsules contained microcrystalline cellulose. The vials and capsules containing NR and placebo were identical in shape, colour, and smell. Study drug compliance was calculated from counting returned number of study capsules and self-reporting. Participants, examining physicians, medical- and research personnel were blinded for the duration of the trial. Sample size was determined by comparing previous literature with respect to the primary objective.

### 1.3. Procedures

At screening for trial entry, candidates underwent physical and neurological examination, brain MRI, and DaTscan. Eligible patients who entered the trial were assessed at baseline (visit 1, V1) and after four weeks of exposure to either NR or placebo (visit 2, V2). At both visits, patients underwent physical and neurological examination, including assessment by the Movement Disorder Society Unified Parkinson's Disease Rating Scale section I-IV (MDS-UPDRS), and biological sampling. MR-PET was performed within one week prior to visit 1, and at visit 2. All participants remained naive for antiparkinson drugs for the duration of the trial. All clinical assessments were performed by a blinded movement disorders expert. Each patient was examined by the same neurologist at both visits. Methodological details on each procedure are provided below. Safety was monitored through routine clinical blood tests and registration of adverse events. Adverse events (AE) were recorded and scored by the investigative physician. Severity was scored according to severity grading defined by Common Terminology Criteria for Adverse Events v5.0 (CTCAE) as (1) mild, (2) moderate, (3) severe, (4) life-threatening or (5) death. The AE relation to the study drug was scored as (1) unrelated, (2) unlikely, (3) possible, (4) probable or (5) definitely.

### 1.3.1. Biological sampling

Biological material was collected at both visits, following a minimum 6-hour fasting and within 6 hours after study drug intake for visit 2. Sampling included whole blood, serum, peripheral blood mononuclear cells (PBMCs), cerebrospinal fluid (CSF) and muscle biopsy. Lumbar puncture was performed according to standard clinical procedures to collect 10 ml CSF. Needle biopsy of the vastus lateralis muscle was performed using a Bard Magnum biopsy gun (BD^{©}, United States) and 12Gx10cm biopsy needles. Muscle biopsies were immediately dissected to remove any non-muscle tissue (e.g., fat or fascia) and snap-frozen in liquid nitrogen.

### 1.3.2. Magnetic Resonance Imaging and Spectroscopy (MRI/S)

MRI/S was conducted on a 3T Biograph mMR MR-PET scanner (Siemens Healthcare, Germany). Phosphorous MRS (31P-MRS) was performed on a double-resonant transmit/receive 1H/31P volume head-coil (Rapid Biomedical, Germany).

31P spectroscopy data were acquired using a 3D chemical shift imaging (CSI) FID sequence with WALTZ4 1H decoupling and continuous wave nuclear Overhauser effect (NOE) enhancement (Peeters TH, et al. 3D 31P MR spectroscopic imaging of the human brain at 3 T with a 31P receive array: An assessment of 1H decoupling, T1 relaxation times, 1H-31P nuclear Overhauser effects and NAD+. NMR in Biomedicine. 2019;e4169). A CSI grid with a 8x8matrix and nominal voxel size of 30 × 30 × 80 mm³ 1024 samples, readout length = 512 ms, 1000 Hz bandwidth, field of view (FOV) = 240x240x80mm3, TE/TR = 2.3ms/3.0s, 10 averages, flip angle = 90 degrees and total acquisition duration of 14.5 minutes. Rectangular NOE pulses of 10 ms length, interpulse delay 1ms, train length 10 prior and WALTZ4 decoupling (2ms pulses, 180 deg. flip angle) were applied prior to 31P-excitation and during the first half of the acquisition window respectively. The FOV was centered on the brain midline and aligned parallel to the anterior and posterior commissure.

An anatomical T1-weighted image with the following sequence parameters was acquired to aid positioning of the 31P-imaging slab: MPRAGE 3D T1-weighted sagittal volume, TE/TR/TI = 1.96ms/1.5s/900ms, acquisition matrix = 128 × 128 × 176, FOV = 256 × 256 mm², 200 Hz/px readout bandwidth, flip angle = 8 degrees and total acquisition duration of 3.1 minutes; or MPRAGE 3D T1-weighted sagittal volume, TE/TR/TI = 2.26 ms /2.4 s /900 ms, acquisition matrix = 256 × 256 × 192, FOV = 256 × 256 × 192 mm3, 200 Hz/px readout bandwidth, flip angle = 8 degrees and total acquisition duration of 5.6 minutes.

Spectra from the occipital region were aligned using an adaption of the Spectral Registrion implementation from Gannet 3.0, subject to thresholding on SNR (>=3) to eliminate the majority of out-of-brain voxels. Voxels were averaged before being processed in Matlab 9.5 (the MathWorks, Natick, MA) using the OXSA toolbox utilising first order phase correction and fitting with AMARES. Custom prior information was created based on literature values for membrane phospholipids (MP), glycerophosphocholine (GPC), glycerophosphoethanolamine (GPE), inorganic phosphate (Pi), phosphocoline (PC), phosphoethanolamine (PE) as well as alpha-, beta- and gamma resonances of adenosine triphosphate (ATP-α, -β, and -γ, respectively) in reference to the phosphocreatine (PCr) peak. Additional information for the properties of nicotinamide adenine dinucleotide (NAD) was added based on the framework developed by Lu et al (Lu M, et al. Magn Reson Med 2014; 71: 1959-1972) by calculating field-strength dependent chemical shift differences, relative amplitudes and frequency separations for oxidised and reduced NAD (NAD⁺ and NADH, respectively). Linewidths were fixed to be equal for NAD⁺, NADH and ATP-α. At 3T and to comply with normal-mode specific absorption rate (SAR) restrictions, peak separation for NAD⁺ and NADH was limited and therefore only combined values of total NAD (NAD⁺ and NADH together) are reported.

### 1.3.3. Positron emission tomography (PET)

PET-MRI imaging was performed with a 3T Siemens Biograph mMR scanner, software version VE11P-SP03. Subjects were administered 200 MBq of 18F Fluorodeoxyglucose (FDG) before being positioned in the scanner. After 31P-MRS spectra had been acquired, an 8-channel Siemens mMR Head coil was used for further MR imaging, which was performed simultaneously with FDG PET. In all subjects, PET acquisition began 25 to 30 minutes after the injection of FDG and lasted 30 minutes. The resulting emission data were reconstructed using Siemens HD-PET algorithm with Siemens HiRes Brain MRAC, with voxel size of 2.3 × 2.3 × 5.0 mm³. PET images were anonymized and archived as DICOM files with a matrix size of 344 × 344 × 127 and an isotropic voxel size of 2.09 × 2.09 × 2.03 mm³.

FDG-PET scans were transferred electronically to the Center for Neurosciences at The Feinstein Institutes for Medical Research (Manhasset, NY, USA) and analyzed using automated computing pipelines implemented in MATLAB 7.5 (MathWorks, Natick, MA) using in-house Scan Analysis and Visualization (ScAnVP) software (available at http://feinsteinneuroscience.org). Images were first preprocessed using Statistical Parametric Mapping (SPM8) software (http://fil.ion.ucl.ac.uk/spm; Wellcome Centre for Human Neuroimaging, London, UK). Of the 30 participants, the baseline scan of one NR subject was excluded on technical grounds. In the remaining subjects, FDG-PET scans acquired at visit 1 and visit 2 were aligned to produce a mean image, which was spatially normalized in standard Montreal Neurological Institute (MNI) anatomic space along with the individual scans from each time point. The normalized images were then smoothed with a 10-mm Gaussian filter in three dimensions to enhance the signal to noise ratio.

For the PET-analyses, the subjects in the NR group were classified based on their cerebral NAD-response, measured by 31P-MRS. Subjects showing increased cerebral NAD levels at visit 2 were termed "MRS-responders," whereas subjects showing no increase in cerebral NAD were termed "MRS non-responders".

To identify an NR-related metabolic pattern (NRRP), we analyzed paired metabolic scan data from the 10 MRS responders in the NR group (ATP-beta; NAD-total; ATP-alpha; ATP-gamma; PCr (Phosphocreatine); MP (membrane phospholipids); GPC (glycerophosphocholine); GPE (glycerophosphoethanolamine); Pi (inorganic phosphate); Pc (phosphocholine); PE (phosphoethanolamine); Total NAD / PCr; Total NAD / ATP-alpha) using ordinal trends/canonical variates analysis (OrT/CVA), a supervised form of principal component analysis (PCA) (Habeck C, Krakauer JW, Ghez C, Sackeim HA, Eidelberg D, Stern Y et al. A new approach to spatial covariance modeling of functional brain imaging data: ordinal trend analysis. Neural computation 2005; 17: 1602-1645).

This multivariate approach is designed to detect and quantify regional covariance patterns (i.e., metabolic networks) for which expression values (i.e., subject scores) increase or decrease with treatment in all or most of the subjects (Niethammer M, Tang CC, Vo A, Nguyen N, Spetsieris P, Dhawan V et al. Gene therapy reduces Parkinson's disease symptoms by reorganizing functional brain connectivity. Science translational medicine 2018; 10; Ko JH, Feigin A, Mattis PJ, Tang CC, Ma Y, Dhawan V et al. Network modulation following sham surgery in Parkinson's disease. The Journal of clinical investigation 2014; 124: 3656-3666). The significance of the resulting OrT/CVA topographies was assessed using nonparametric tests, i.e., permutation testing of the subject scores to show that the observed ordinal trend did not occur by chance. Likewise, the reliability of the voxel loadings (i.e., region weights) on the resulting network topography was assessed using bootstrap resampling procedures (Mure H, Hirano S, Tang CC, Isaias IU, Antonini A, Ma Y et al. Parkinson's disease tremor-related metabolic network: characterization, progression, and treatment effects. Neuroimage 2011; 54: 1244-1253; Habeck C, Stern Y. Multivariate data analysis for neuroimaging data: overview and application to Alzheimer's disease. Cell biochemistry and biophysics 2010; 58: 53-67).

in the present study, we restricted the analysis to the top six PC patterns, which together accounted for greater than 75% of the subject × voxel variance in the longitudinal data. Expression values for these PCs were entered singly and in all possible linear combinations to identify significant monotonic trends in the individual subject data, i.e., consistent increases (or decreases) in pattern expression across the subjects with few if any violations (p < 0.05; permutation test, 1000 iterations). The resulting coefficients were applied to the corresponding PC patterns to construct the NR-related topography. For the NRRP to be significant, we required that voxel weights have low dispersion (inverse coefficient of variation (ICV) |*z*| >1.96, p<0.05; bootstrap resampling (1,000 iterations), indicating that regional loadings were not driven by outliers). The current analysis was performed within the population gray matter brain mask defined by the FDG PET scans. To standardize NRRP subject scores, we computed expression values for this pattern in an age-matched group of 22 healthy volunteers (11M/11F; age 62.9 ± 8.6 years) scanned at the Feinstein Institutes. Values in all trial participants (NR and placebo) were standardized (z-scored) with respect to these scans (Spetsieris P, Ma Y, Peng S, Ko JH, Dhawan V, Tang CC et al. Identification of disease-related spatial covariance patterns using neuroimaging data. Journal of visualized experiments: JoVE 2013; Peng S, Ma Y, Spetsieris PG, Mattis P, Feigin A, Dhawan V et al. Characterization of disease-related covariance topographies with SSMPCA toolbox: Effects of spatial normalization and PET scanners. Wiley Online Library, 2014).

To further examine the effect of NR therapy on metabolic network organization, we compared the NRRP to a specific PD-related metabolic pattern (PDRP) that has been extensively validated in prior studies (Schindlbeck KA, et al. Network imaging biomarkers: insights and clinical applications in Parkinson's disease. The Lancet Neurology 2018; 17: 629-640). PDRP expression levels (subject scores) were computed in all trial participants. These values were correlated with corresponding NRRP subject scores and UPDRS motor ratings in each condition. Correlations were likewise computed between the changes in these measures in the two groups.

### 1.4. RNA sequencing

For all subjects at both visits, total RNA was extracted from muscle biopsy tissue and PBMC homogenate using RNeasy plus mini kit (Qiagen) with on-column DNase treatment according to manufacturer's protocol. The final elution was made in 45 ul of dH2O. The concentration and integrity of the total RNA were estimated by Ribogreen assay (Thermo Fisher Scientific), and Fragment Analyzer (Advanced Analytical), respectively, and 10 ng of total RNA was used for downstream RNA-seq applications using the SMARTer Stranded Total RNA-Seq Kit v2 - Pico Input Mammalian Kit (IlluminaTakara Bio USA, Inc., Mountian View, CA) as per manufacturer's recommended protocol. Library quantity was assessed by Picogreen Assay (Thermo Fisher Scientific), and the library quality was estimated by utilizing a DNA High Sense chip on a Caliper Gx (Perkin Elmer). Accurate quantification of the final libraries for sequencing applications was determined using the qPCR-based KAPA Biosystems Library Quantification kit (Kapa Biosystems, Inc.). Each library pooled equimolar prior to clustering. One hundred bp paired-end sequencing was performed on an Illumina NovaSeq S4 sequencer (Illumina, Inc.). Two subjects were discarded from the PBMC samples and two from the muscle samples due to either insufficient tissue quantity, participant drop out/lack of sample, or being outliers in expression. RNA quality, as measured by the RNA integrity number (RIN), was satisfactory (mean = 7.6 ± 1.1, range = 3.1-9.1 for PBMC samples; mean = 9.1 ± 1.3, range = 5-10 for muscle), and was not significantly associated with visit or treatment (PBMC treatment-RIN, p = 0.47; PBMC visit∼RIN, p = 0.37; muscle treatment~RIN, p = 0.21; muscle visit~RIN, p = 0.99, F-test).

FASTQ files were assessed using fastQC version 0.11.9. Quantification at the transcript level was calculated using Salmon version 1.3.0 with fragment-level GC bias correction against the GENCODE version 35, with the whole genome as decoy sequence. Transcript-level quantification was collapsed onto gene-level using the tximport R package version 1.8.0. Genes in non-canonical chromosomes and scaffolds and transcripts encoded by the mitochondrial genome were filtered out, together with non-protein-coding genes. Low-expressed genes were likewise removed from downstream analyses (expression below 10 reads for 75% of the samples). PBMC and muscle datasets were analysed independently. For PBMC samples, cell type composition was assessed employing ABIS. Differential gene expression was performed using the DESeq2 R package version 1.22.2. The generalized linear model formula accounted for both interindividual variability between subjects and the normal course of the experiment (baseline for each subject and baseline for the time-course). Multiple hypothesis testing was performed with the default automatic filtering of DESeq2 followed by false discovery rate (FDR) calculation by the Benjamini-Hochberg procedure. Functional enrichment of both over- and under-expressed genes was carried out by ranking genes according to their p-value (accounting for the direction of change) and employing the gene score resampling method implemented in the ermineR package version 1.0.1, an R wrapper package for ermineJ with the complete Gene Ontology (GO) database annotation to thus obtain lists of up- and down-regulated pathways for each dataset.

### 1.5. Metabolomic analysis

### Muscle and PBMCs metabolomics

Standard substances of all the targeted compounds were used to prepare stock standard solutions and these solutions were mixed and diluted to make serially diluted standard solutions in an internal standard solution containing 11 isotope-labeled compounds (NAD-13C5, NADH-d5, nicotinamide riboside-d4, nicotinic-d4 acid, nicotinic acid-d4 riboside, AMP-15N5, ATP-13C10, nicotinamide-d4, 1-methylnicotinamide-d3, adenosine-13C5 and GTP-13C10) in a concentration range of 0.0002 to 20 nmol/mL. Muscle tissues were added with water at 2.5 µL/mg raw material and then homogenized on an MM 400 mixer mill for 1 min twice. Methanol at 7.5 µL/mg raw material was then added and the samples were homogenized for 1 min three times. The samples were then placed at -20 °C for 2 h before centrifugation. 20 µL of the supernatant was mixed with 180 µL of the internal standard solution. The samples were then placed at 5 °C for 1 h and then centrifuged for 5 min at 21,000 g. The clear supernatants were used for LC-MS analyses. For metabolomic analyses in PBMCs, several aliquots of one sample were thawed on ice, centrifuged (10 min, 350 xg, 4 °C), resuspended in ice-cold PBS and counted on a countess II cell counter. The equivalent volume of 10 Mio live cells was transferred into a new tube, centrifuged (10 min, 350 xg, 4 °C), the supernatant removed, and the pellet immediately frozen on dry ice. Each PBMC sample was made into 75% methanol at a concentration of 50 µL per 1 Mio cells. After cell lysis on a MM 400 mixer mill for 1 min twice, the sample was centrifuged. 20 µL of the clear supernatant was mixed with 180 µL of the internal standard solution and was then used for LC-MS analysis.

For UPLC-MRM/MS, 10 µL aliquots of each standard solution and each sample solutions were injected to run LCMRM/MS either on a Waters Acquity UPLC coupled to a Sciex QTRAP 6500 Plus mass spectrometer with (-) ion detection or ESI or on an Agilent 1290 UHPLC system coupled to an Agilent 6495B QQQ mass spectrometer with (+) ion detection. For UPLC-(-) MRM/MS, a reversed-phase C18 LC column (2.1*100 mm, 1.8 µm) was used for LC separation, with the use of a tributylamine-ammonium acetate buffer (solvent A) and acetonitrile (solvent B) as the mobile phase for gradient elution at 50 °C and 0.25 mL/min. For UPLC-(+) MRM/MS, a reversed-phase C18 column (2.1*150 mm, 1.8 µm) was used for LC separation, with the use of hepatofluorobutyrate buffer (solvent A) and methanol (solvent B) as the mobile phase for gradient elution at 50 °C and 0.3 mL/min. Concentrations of individual metabolites detected in each sample were calculated by constructing linear-regression curves of individual metabolites with the analyte-to-internal standard peak area ratios (As/Ai) of standard solutions in each set of LC-MS runs and then by interpolating the calibration curves with the as/Ai values measured from injections of sample solutions in an appropriate concentration range for each compound. Sample preparation and metabolomic analyses were conducted at Creative Proteomics, NY, USA.

### CSF metabolomics

The extraction of metabolites from CSF samples was carried out as follows: 400 µl of each sample were mixed with 1600 µl of ice-cold UHPLC grade methanol. The samples were vortexed for 10 sec before centrifugation at 16,000 xg for 20 min. 1800 µl of the clear supernatant were transferred to a fresh tube and freeze-dried at -105 °C in a SpeedVac (Thermo Scientific) coupled with a refrigerated vapor trap. The residue was reconstituted in 40 µl of acetonitrile spiked with sulfadimethoxine at a concentration of 50 pg/ml. The samples were then centrifuged at 16,000 xg for 10 min, and the clear supernatants were used for LC-MS analyses. Recovery rate was tested for several relevant nucleotides and determined to be 93-96%, depending on the nucleotide.

Metabolite analysis was conducted using a Dionex UltiMate 3000 instrument coupled with an electrospray ionization (ESI) QExactive mass spectrometer (Thermo Scientific). The separation of metabolites was achieved on a ZIC-cHILC column (50 × 2.1 mm, 3µm, Merck), which was kept at 40 °C during analysis. The injection volume was 5 µl and the flow rate was kept at 0.3 ml/min. The mobile phase consisted of 10 mM ammonium carbonate pH 8.0, 3 % acetonitrile (solvent A) and 10 mM ammonium carbonate pH 8.0, 90 % acetonitrile (solvent B). The applied gradient was: 85 % B for 0.5 min, decreased to 70 % B during 0.1 min, constant at 70 % B for 1 min, and decreased to 60 % B during 4.3 min. Then, B concentration was decreased to 40 % during 0.1 min, kept at 40 % B for 0.7 min as washout, returned to 85 % B during 0.1 min and was kept for 1.2 min at 85 % B for equilibration prior to the next run. Total run time was 8 min. Electron spray ionization was operated in positive ion polarity mode with a spray voltage of 3.5 kV. Sheath flow gas flow rate was 48 units with an auxiliary gas flow rate of 11 units and a sweep gas flow rate of 2 units. Mass spectra were recorded using targeted single ion monitoring with an isolation window of 10 m/z with automatic gain control set to a target of 5 × 105 and a maximum accumulation time of 300 ms. Data analysis was conducted in the Thermo Xcalibur software (Thermo Scientific) using an automated processing method. Absolute concentrations were calculated by external calibration curves using pure standards.

### 1.6. Nf-L, GDF15, FGF21 and Cytokine detection

Neurofilament light chain (Nf-L) measurement in CSF and serum samples was carried out in duplicates using the Simoa NF-light Advantage (SR-X) Kit (Quanterix) according to the manufacturer's recommendations and analyzed on an Simoa SR-X instrument (Quanterix). GDF15 and FGF21 detection was carried out using ELISA kits GDF-15/MIC-1 Human ELISA (RD191135200R) and Fibroblast Growth Factor 21 Human ELISA (RD191108200R) from BioVendor according to manufacturer's recommendations. For CSF analyses of GDF15, CSF was diluted 1:2 in dilution buffer. All samples were analyzed twice in duplicates. Inflammatory Cytokine screening was carried out using the Human Cytokine Magnetic 35-plex panel (Invitrogen), detecting FGF-Basic, IL-1 beta, G-CSF, IL-10, IL-13, IL-6, IL-12, RANTES, Eotaxin, IL-17A, MIP-1 alpha, GM-CSF, MIP-1 beta, MCP-1, IL-15, EGF, IL-5, HGF, VEGF, IL-1 alpha, IFN-gamma, IL-17F, IFN-alpha, IL-9, IL-1RA, TNF-alpha, IL-3, IL-2, IL-7, IP-10, IL-2R, IL-22, MIG, IL-4, IL-8. CSF and serum samples were analyzed according to the manufacturer's recommendations and measured on a BioPlex 200 instrument (BioRad).

### 1.7. Outcomes

The primary objective of the study was to assess target engagement, by determining whether oral NR treatment increases cerebral NAD and impacts the neurometabolic profile of patients with PD, based on neuroimaging measures (FDG-PET, 31P-MRS). Secondary objectives were to determine if NR improves clinical symptoms measured using MDS-UPDRS and whether it augments the NAD metabolome in peripheral tissue of PD patients. Additional secondary measures included frequency of adverse events, changes in vital signs, and clinical laboratory values. Exploratory outcome measures assessed the effect of NR on gene-expression in PBMC and muscle (RNA-seq), blood and CSF biomarkers of neuronal damage, mitochondrial function, and inflammation.

### 1.8. Statistical Analyses

The between visit change ([visit 2] - [visit 1]) in MDS-UPDRS (total of subsections I-III; and individual subsections I, II and III) was compared between the NR and placebo groups using independent Student's *t*-test. In addition, the between visit change in MDS-UPDRS (total and subsections) within the NR, MRS-responder, and placebo groups was assessed by paired Student's t-test. For the 31P-MRS analyses, the change in measured metabolites between visit 1 and visit 2 was assessed in the placebo- and NR group using paired Student's *t*-test. In addition, the between-visit change in NAD/ATP-α was compared between the NR and placebo group by independent Student's t-test. For PET analyses, changes in network scores with treatment were evaluated for each group separately using paired Student's t-tests or permutation tests. Relationships between network values, NAD levels and MDS-UPDRS motor ratings or between treatment-related changes in these variables were evaluated using Pearson's product-moment correlations; Spearman rank-order correlation coefficients were computed for non-normal distributions of the variables. These statistical tests were performed using SPSS (SPSS Inc., Chicago, IL); results were considered significant for p ≤ 0.05 (two-tailed). Metabolite quantification data, Nf-L; GDF15, FGF21 and cytokine data were analyzed by paired sample Wilcoxon test using GraphPad Prism v 6.07.

### 2. Results

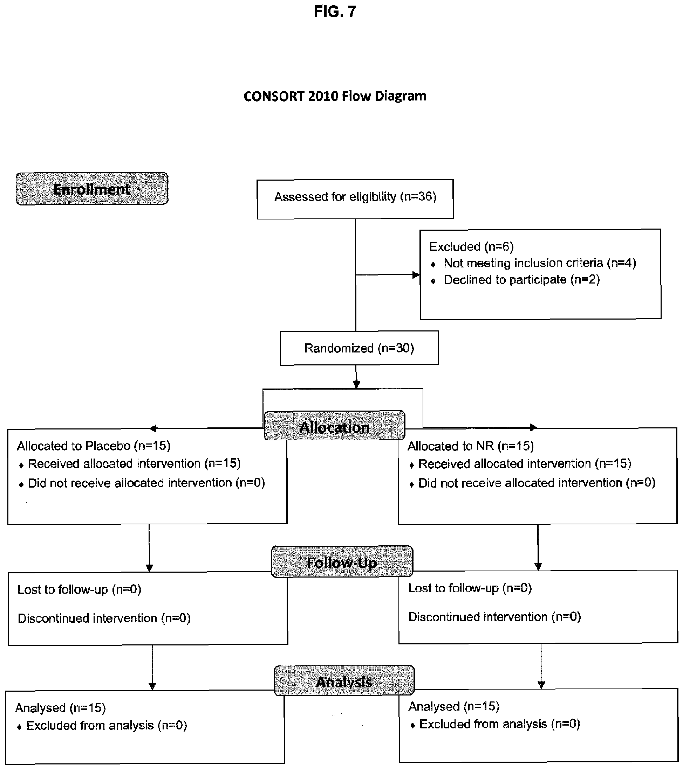

In total, 36 patients were screened, and 30 eligible patients were enrolled, all of whom completed the study **(****FIG. 7****)**. There were no significant demographic differences between the NR and placebo group (Table 1). Seven subjects in the NR group and three subjects in the placebo group reported adverse events, all of which were minor and considered to be unrelated to NR (Table 4). Based on the number of remaining capsules in returned vials, we estimated a mean study drug adherence in the NR and placebo group to 98%. The average time on study drug was 32.5 (±2.7) days for the NR group and 32.4 (±2.53) days for the placebo group. Four participants (two in the NR and two in the placebo group) did not return any capsules but reported they were drug compliant.

### 2.1. NR increases cerebral NAD levels.

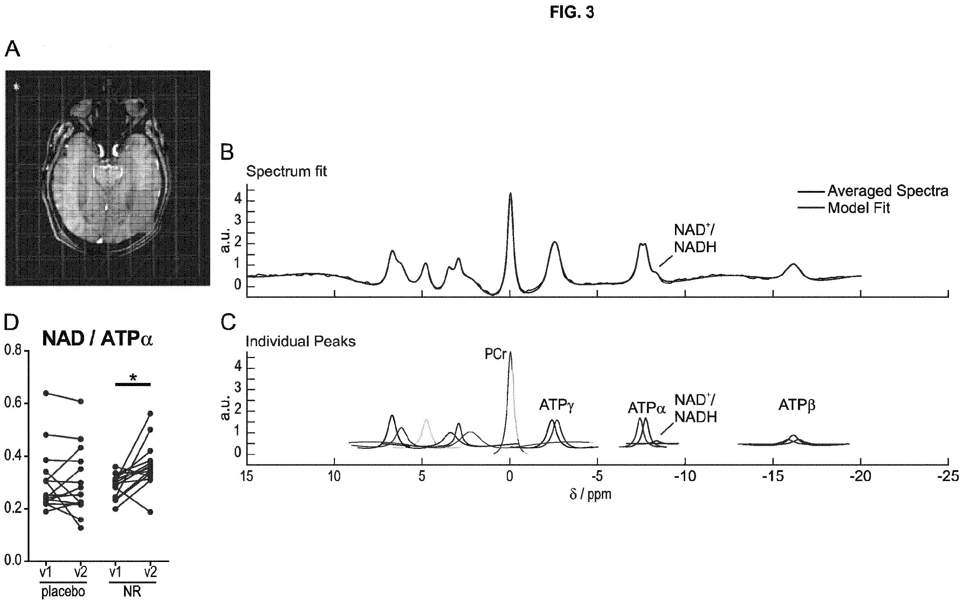

31P-MRS allowed for identification and quantitation of multiple phosphorylated compounds **(**FIG. 3A-C). NAD levels, normalized to ATP-α, showed a significant increase in the NR group (paired t-test: p = 0.016), but not in the placebo group **(****FIG. 3D****)**. Direct comparison between the groups showed that the between visit change (visit 2/visit 1) in the NAD/ATP-α ratio was significantly higher in the NR group compared to the placebo group (t-test: p =0.025). No other detected metabolites exhibited significant change in either the NR- or placebo group between visits. At the individual level, the cerebral NAD response was heterogeneous, with only 10/13 patients showing an increase, of whom 9 showed a change exceeding 10% of baseline levels. We will henceforth refer to the subgroup of 10 NR recipients who showed an increase in cerebral NAD levels as MRS-responders. Since this variability raised the possibility of heterogeneous treatment effects, we chose to stratify downstream neurometabolic and clinical analyses.

### 2.2. NR induces a novel treatment-related metabolic network.

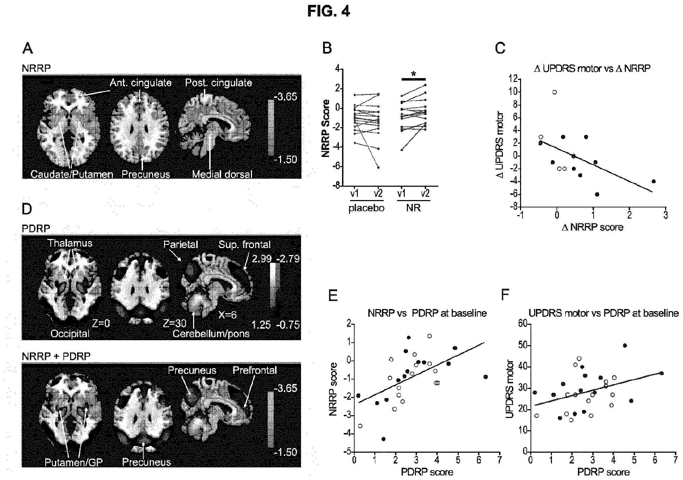

We next interrogated the FDG PET data of the NR recipients to determine whether treatment-related increases in cerebral NAD levels were associated with a significant metabolic brain network. To this end, we applied a supervised PCA algorithm (OrT/CVA) to paired scan data from the NR participants (n=10) for whom brain NAD levels were simultaneously increased with treatment. The analysis revealed a significant ordinal trend pattern, which was represented by the first principal component (PC1), accounting for 20.6% of the variance in the paired data. This NR-related metabolic pattern (NRRP, **FIG. 4A**) was characterized by bilateral metabolic reductions in the caudate and putamen, extending into the adjacent globus pallidus, and in the thalamus (Table 5). As part of the network, these changes were also associated with localized cortical reductions along the medial wall of the hemisphere involving the precuneus (BA 7), medial frontal cortex (BA 9, 10), anterior cingulate area (BA 24, 32), and in the posterior cingulate gyrus (BA 31). The reliability of these regions was demonstrated by bootstrap iteration [ICV z = -3.83, -2.58, p < 0.005; 1000 iterations]. Areas with relative metabolic increases did not contribute significantly to this topography.

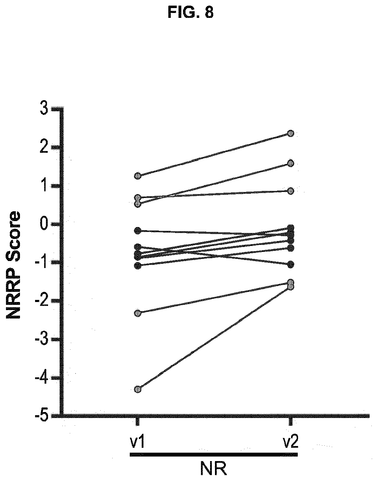

At the individual subject level **(****FIG. 8****)**, a significant ordinal trend in NRRP expression was observed (p<0.018; permutation test, 1000 iterations) in the 10 NR recipients in the pattern identification group (**FIG. 4B**). Black lines indicate individuals with a positive NAD response in the MRS analysis,. Grey lines indicate the remaining four individuals who either showed no NAD response in the MRS. (n=2), or for whom MRS data was not available (n=2). While analogous NR-mediated increases in pattern expression were not seen in the remaining four NR recipients who either did not exhibit NAD responses on MRS, or for whom MRS data was not available **(****FIG. 4B**, *gray lines*), the ordinal trend remained significant for the NR group as a whole (p=0.027). That said, consistent changes in NRRP expression were not significant in the placebo group (p = 0.497). Significant correlations were not observed between treatment-related changes in cerebral NAD levels and NRRP expression in either of the trial groups (p > 0.10).

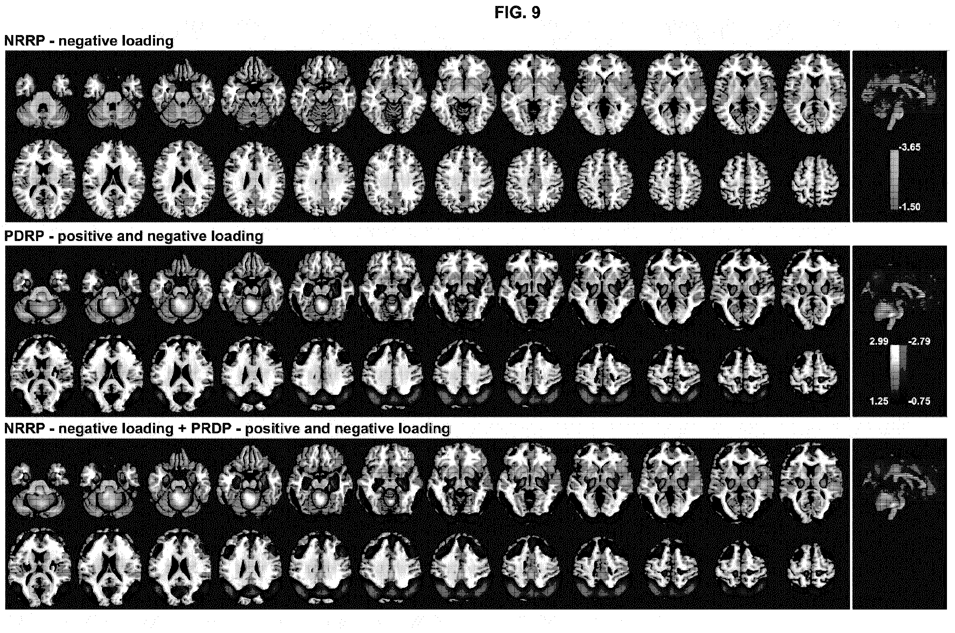

Changes in NRRP expression in the NR group correlated significantly (r = -0.59, p = 0.026) with the changes in UPDRS motor ratings recorded at the time of PET (**FIG. 4C****).** Thus, the largest NRRP increases were observed in the NR subjects with the greatest improvement in motor ratings. An analogous correlation was not seen in the placebo group (p = 0.79). We noted that certain NRRP regions were shared between the NRRP with the previously validated PDRP topography **(****FIG. 4D**, *top*)*.* In particular, areas of reduced activity in the caudate and putamen, extending into the adjacent globus pallidus, overlapped with corresponding areas of overactivity in the PDRP **(****FIG. 4D**, *bottom, hot* voxels, **FIG. 9**). Likewise, spatial overlap was observed between underactive regions in the frontal and parietal cortex for the two patterns **(****FIG. 4D**, *bottom, purple* voxels, **FIG. 9**). Indeed, a significant correlation was observed between baseline PDRP and NRRP expression values across the trial population (r = 0.56, p = 0.002; **FIG. 4E**). As typically seen in PD populations, PDRP expression values at baseline correlated with UPDRS motor ratings (r = 0.374, p = 0.046 for the two groups; **FIG. 4F**). In contrast to NRRP, in which the changes in pattern expression with NR correlated with clinical improvement **(****FIG. 4C****)**, no correlation with outcome was observed for the PDRP changes (p = 0.73).

### 2.3. NR-induced increase in cerebral NAD is associated with clinical improvement of PD.

A trend for decreased MDS-UPDRS was seen in the MRS-responder subgroup (mean decrease 1.9 ± 2.78, paired t-test: p = 0.071), and this reached statistical significance when only the nine individuals showing >10% increase in cerebral NAD levels were considered (mean decrease 2.33 ± 2.35; paired t-test: p = 0.017). This effect appeared to be mainly driven by subsections I and III of the MDS-UPDRS.

### 2.4. NR supplementation augments the NAD metabolome in peripheral tissues of PD patients.

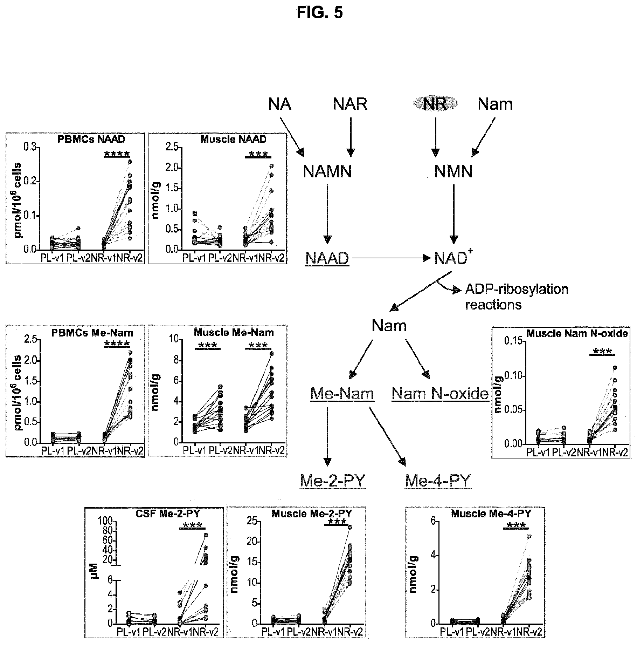

Metabolomic analyses were carried out in skeletal muscle and PBMCs, to confirm NR intake and investigate potential changes in related metabolic pathways. In muscle tissue, several NAD-related metabolites, including the nicotinamide (Nam) degradation products Nam N-oxide, methyl-Nam (Me-Nam), and the methyl pyridones Me-2-PY and Me-4-PY, as well as the acid form of NAD, NAAD, were strongly elevated after treatment with in all NR recipients NR (**FIG. 5**). Other related metabolites, including NAD+, NADH, NMN, NAAD and Me-Nam, were below the detection limit in the CSF. This finding further corroborated that oral NR therapy increases brain NAD levels across the blood brain barrier, as indicated by the 31P-MRS data The direct effect of NR treatment on the central nervous system is revealed by the robust detection of increased Me-2-PY in the CSF. All NR-recipients showed an increase in CSF Me-2-PY levels. This suggests that central nervous system penetration was universal.

In muscle tissue, several NAD-related metabolites, including Nam degradation products Nam N-oxide, methyl-Nam (Me-Nam), and the methyl pyridones Me-2-PY and Me-4-PY, as well as the acid form of NAD, NAAD, were strongly elevated after treatment with NR. The steady state levels of NAD itself (both oxidized NAD+ and reduced NADH), and NAD precursors and intermediates, were not significantly changed by NR treatment **(****FIG. 10****)**, similar to reports from previous trials in humans. PBMCs showed less extensive changes, recapitulating the increase in NAAD and Me-Nam **(****FIG. 5****,** **FIG. 11****).** Additional metabolites that could potentially be affected by changes in the NAD metabolome were investigated, including acetyl-CoA and CoA, the methyl donor S-adenosylmethionine (SAM) and its metabolites, and energy compounds such as ATP. These showed no significant change in PBMCs or muscle **(****FIGS. 10****,** 11). Of note, substantial metabolic changes were seen in the entire NR group, irrespective of the cerebral NAD-response in 31P-MRS.

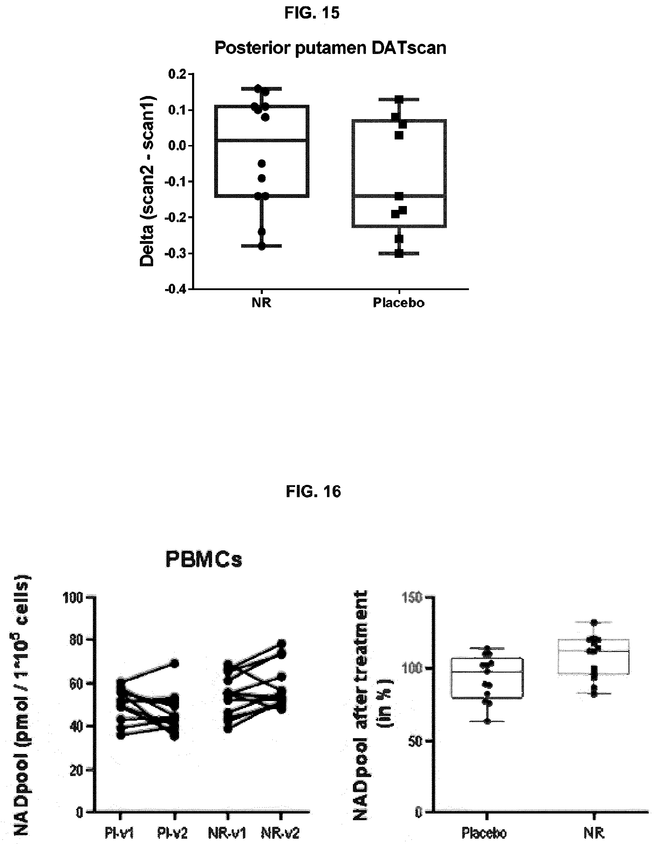

In addition to these analyses, we measured NAD levels in patient PBMC using a different approach (NADMed method). This data showed an increase of NAD in the NR but not in the placebo group. The increase did not reach statistical significance in a paired t-test, due to high variation in the data and the small sample size. However, the trend was clear and convincing. Moreover, a between group comparison of the between visit change in NAD levels showed a significantly higher increase in the NR group, compared to the placebo **(****FIG. 16****)**.

### 2.5. NR therapy induces the expression of mitochondrial, antioxidant, and proteostatic processes.

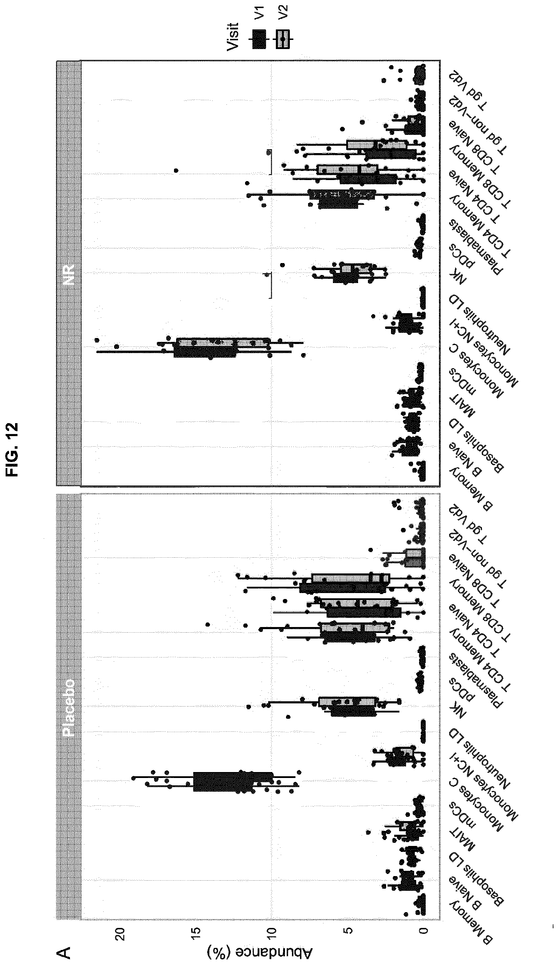

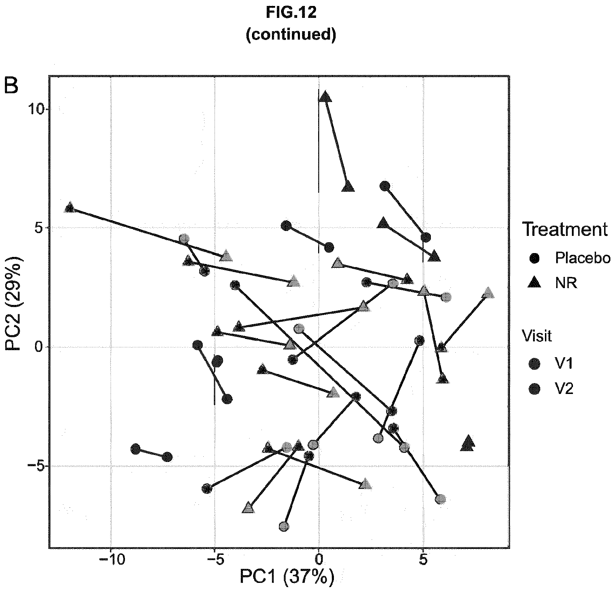

To investigate the effects of NR therapy on gene expression, we carried out RNA-seq analysis in PBMCs and muscle tissue from all study participants and assessed the between visit differences in the NR group compared to the placebo group. In muscle tissue, NR supplementation was significantly associated with differential expression of 58 genes (FDR < 0.05). These included substantial upregulation of *KLF*2, which is associated with decreased adipogenesis and induction of the Nuclear factor erythroid 2-related factor 2 (Nrf2), a transcription factor with a key role in protection against oxidative damage, which has been linked to PD. We also noted upregulation of genes linked to NAD-degradation, such as ADP-ribosylation (*PARP15*)*,* and NAD-dependent redox processes, such as the glycine cleavage system (*AMT*), as well as mitochondrial translation and respiratory complex assembly (*FARS2, TMEM242*)*.* Gene set enrichment analyses revealed NR-induced upregulation of biological processes, including proteasomal function, and RNA transport and stability. In PBMCs, a total of 13 genes were significantly associated with NR supplementation, including upregulation of *BLOC1S2,* a component of the BLOC-1 complex involved in lysosomal biogenesis and trafficking. Functional enrichment revealed highly significant upregulation of multiple biological processes, including ribosomal-, proteasomal-, lysosomal- and mitochondrial (oxidative phosphorylation) pathways. No significant changes were found in genes encoding known NAD biosynthetic including *NMRK1,* encoding nicotinamide riboside kinase 1 which converts NR to NMN. Finally, NR treatment did not show a significant association with estimated cell type proportions in the PBMC samples (p > 0.5, visit:treatment interaction term, linear mixed model, Welsh-Satterthwaite's t-test< 0.05; **FIG. 12**).

NR treatment caused gene expression changes affecting a range of mitochondrial pathways, mostly concerning oxphos, but also the mitochondrial ribosome, RNA metabolism, and ox stress defence, as shown in the below table, summarizing the pathway enrichment analyses against MitoCarta v3.0. of the differential gene expression results in PBMCs from the present study.

| | **Name** | **Direction** | **P value** | **Corrected P value** |
|---|---|---|---|---|
| **1** | OXPHOS subunits | UP | 1.082e-12 | 4.544e-11 |
| **2** | OXPHOS | UP | 0.00001102 | 0.0002315 |
| **3** | Complex V | UP | 0.000032 | 0.000448 |
| **4** | Cl subunits | UP | 0.0005231 | 0.005493 |
| **5** | Mitochondrial ribosome | UP | 0.001011 | 0.008493 |
| **11** | Complex IV | UP | 0.03004074 | 0.21028517 |
| **13** | ROS and glutathione metabolism | UP | 0.035772 | 0.214632 |
| **6** | mtRNA metabolism | DOWN | 0.003136 | 0.13170171 |
| **7** | Cl assembly factors | DOWN | 0.013122 | 0.183708 |
| **8** | Mitochondrial ribosome assembly | DOWN | 0.027256 | 0.190792 |
| **9** | mtRNA granules | DOWN | 0.018336 | 0.192528 |
| **10** | mt-tRNA modifications | DOWN | 0.009994 | 0.209874 |
| **12** | mt-tRNA synthetases | DOWN | 0.03548 | 0.21288 |
| **14** | Vitamin metabolism | DOWN | 0.02605831 | 0.21888977 |
| **15** | OXPHOS assembly factors | DOWN | 0.04239743 | 0.2225865 |

### 2.6. NR modulates biomarkers of inflammation and mitochondrial dysfunction.

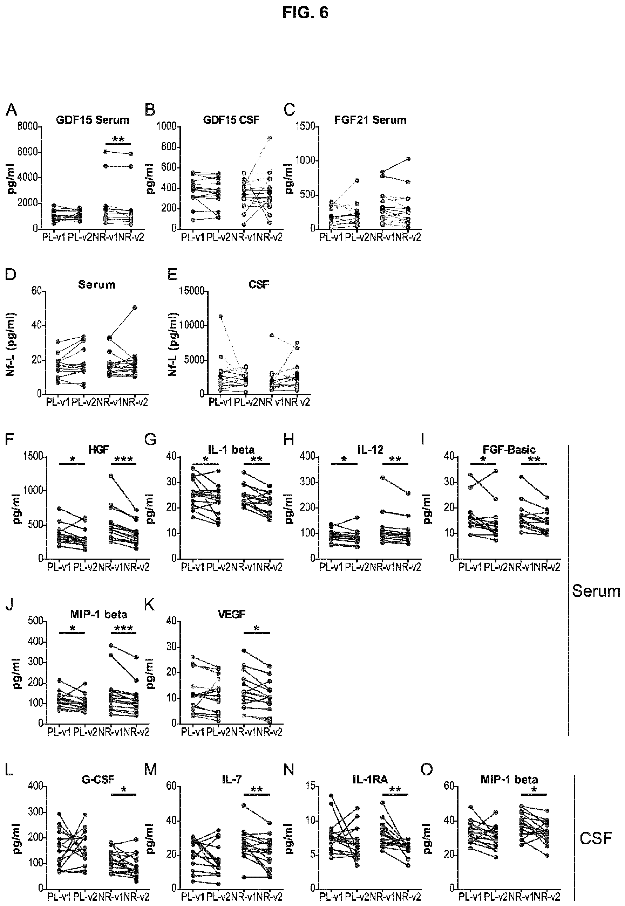

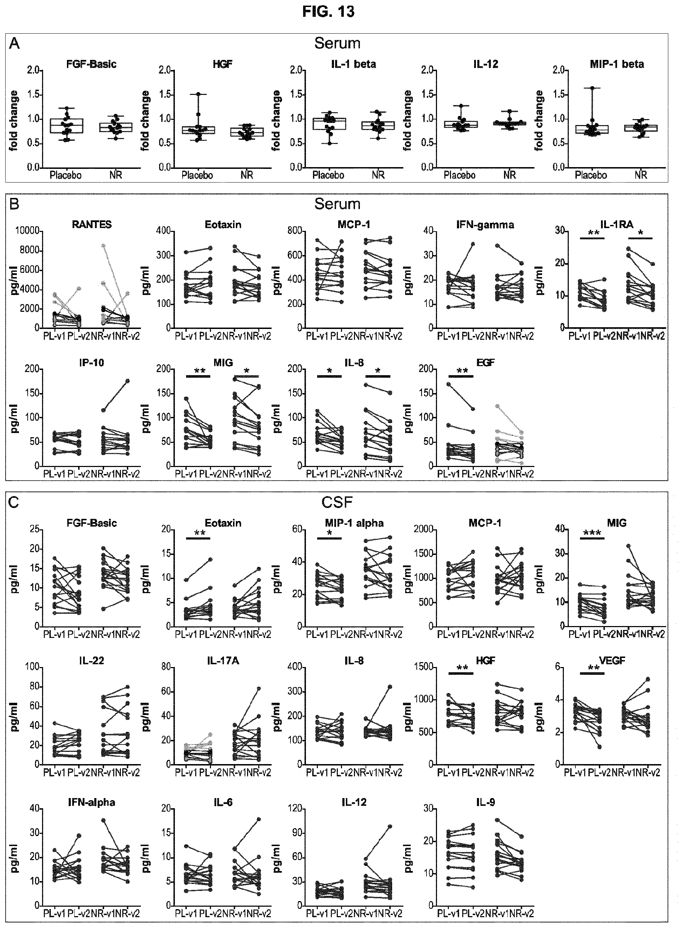

Since mitochondrial dysfunction and inflammation have been associated with the pathophysiology of PD, we sought to identify NR-induced changes in relevant biomarkers in the serum and CSF of our patients. We assessed the levels of growth factors FGF21 and GDF15, which have been associated with mitochondrial dysfunction, and a panel of 35 inflammatory cytokines. Growth factor analysis revealed a mild but significant decrease of GDF15 levels in serum, but not in CSF. FGF21 was unchanged in the serum and was below detection limit in CSF. NR treatment reduced the levels of several inflammatory cytokines in serum and CSF **(****FIG. 6****,** **FIG. 13****).** Several inflammatory cytokines decreased in the serum and/or CSF of the NR-treated group reduced the levels of several inflammatory cytokines in serum and CSF. Notably, several of the serum cytokines showed a significant decrease also in the placebo group. Comparing the fold change of those cytokines between the groups revealed no significant difference. However, the within group decrease (paired t-test) is still significant. In addition, we assessed the levels of neurofilament light chain (Nf-L), which are indicative of neuronal damage. These were unchanged by NR treatment in both serum and CSF. Cytokine decrease in CSF remains significant, and highly relevant, since it suggests effect against neuroinflammation.

### 2.7. Discussion

We present the results of the first NR trial in PD. Our study met its primary outcome, which was to assess target engagement, and established that orally administered NR leads to an increase in cerebral NAD and affects cerebral metabolism in patients with PD. A significant increase in cerebral NAD levels was detected by 31P-MRS in the NR group, even if this effect was not universal. Three patients showed no evidence of cerebral NAD increase, despite a clear peripheral metabolic response, confirming treatment compliance and an impact on the NAD metabolome in blood and muscle. The discordance between peripheral and central effects may be due to individual variation in cerebral penetrability and/or downstream metabolism of NR in the brain, or it may reflect limited sensitivity of the NAD measurement by 31P-MRS. Irrespective of the mechanisms underlying this variable response, our findings indicate that assessment of cerebral NAD-levels may be an important monitoring parameter in clinical trials evaluating NR supplementation for brain health and disease. Variability in the achieved NAD increase in the patient brain raises the possibility of a heterogeneous biological and, potentially, clinical response to NR-therapy. Increase in cerebral NAD levels was, indeed, associated with both neurometabolic and clinical response in our patients.

FDG-PET analyses identified the NRRP - a novel network topography that was induced in PD trial participants receiving NR but not placebo, under blinded conditions. Apart from its defining ordinal trend, changes in pattern expression with NR correlated with clinical improvement as measured by reductions in MDS-UPDRS ratings. The NRRP consisted mainly of decreased glucose uptake in the basal ganglia and neocortical areas. This may reflect a more efficient bioenergetic state, requiring less glucose consumption to maintain required ATP production. Increased NAD⁺ levels would promote fatty acid beta-oxidation, thereby providing an increased supply of reducing equivalents (i.e., NADH, FADH₂) to the respiratory chain, independent of glycolysis. While the organization of the NRRP remains unknown at the systems level, the network shares a number of topographic lectures with the PDRP, which support a potential role for NR in PD therapeutics. The overlap between metabolically active PDRP regions in the posterior putamen and globus pallidus and areas of treatment-related metabolic reduction in the NRRP is particularly relevant. In recent graph theoretic studies of PDRP organization, we found that these regions comprise a discrete core zone which drives overlap network activity. Metabolic reductions in core nodes have been observed in symptomatic PD treatments such as levodopa administration and subthalamic deep brain stimulation. The presence of similar changes with NR suggest that these regions may also be modulated by this treatment. In any event, clinical outcomes in the NR group relate more to induction of NRRP than modulation of the PDRP activity - a situation similar to that encountered with subthalamic gene therapy.

Metabolomic analyses in PBMCs and muscle confirmed NR intake in the treatment group and excluded this in the placebo group. Notably, a highly significant increase in established markers of NR-mediated NAD biosynthesis, such as NAAD and Me-Nam, was seen in all patients in the NR group An increased flux in the NAD metabolome was detected in both PBMCs and muscle of our patients, indicating higher NAD availability in the NR group.

In addition to the NAD-metabolome, we investigated other metabolites, which are functionally linked to NAD metabolism and signalling and may be affected by NR treatment. Synthesis of Me-Nam, which was greatly increased by NR supplementation, requires the methyl-donor SAM. This, in turn, could limit SAM availability for other essential methylation reactions, such as DNA and histone methylation, and neurotransmitter synthesis, including dopamine. We found no significant changes in SAM or its related metabolites SAH and homocysteine, indicating that NR supplementation does not limit SAM availability for other vital reactions.

Another metabolite of interest was acetyl-CoA, donor of acetyl-groups for acetylation reactions, including histone acetylation. Higher NAD availability could lead to increased acetyl-CoA synthesis by promoting glucose and fatty acid catabolism. This could, in turn, exacerbate the histone hyperacetylation state observed in the PD brain, further dysregulating gene expression. Our analyses detected no significant changes in acetyl-CoA or CoA levels upon NR supplementation. Finally, we monitored energy metabolites such as ATP, ADP, AMP as well as GTP and GDP. None of these showed a significant change upon NR supplementation.

The downstream metabolic impact of NR treatment was supported by the transcriptomic analyses in PBMC and muscle, which revealed effects in multiple disease-relevant pathways. Notably, NR was associated with upregulation of genes involved in mitochondrial respiration, antioxidant response, and protein degradation, including the proteasome and lysosome. Quantitative and functional respiratory deficiency (Flones IH, et al. Acta Neuropathol 2018; 135: 409-425), increased oxidative damage (Jenner P. Ann Neurol 2003; 53 Suppl 3: S26-36; discussion S36-38; Dias V, et al. J Parkinsons Dis 2013; 3: 461-491), and impaired proteasomal and lysosomal function (Lehtonen S, et al. Front Neurosci 2019; 13. doi:10.3389/fnins.2019.00457), have all been strongly implicated in the pathophysiology of PD. Therefore, these findings are encouraging and support a potential neuroprotective effect of NR in PD. Furthermore, our results indicate that NR may have anti-inflammatory action, not only peripherally but also in the central nervous system. NR treatment in our patients was associated with decreased levels of several inflammatory cytokines in serum and the CSF. Neuroinflammation has been implicated in the pathogenesis of PD and is considered a potential target for neuroprotection (Rocha NP, et al. BioMed Research International 2015; 2015: e628192).

Mitochondrial biomarker analyses revealed a mild, but significant decrease of GDF15 in the serum, but not CSF, of NR recipients. This is in line with the observed transcriptomic upregulation of mitochondrial genes, and indicative of improved mitochondrial function in our patients. Nf-L levels were not significantly affected by NR treatment in serum or CSF. However, Nf-L is only mildly elevated in PD (especially in early stages of the disease), compared to healthy controls, and is better suited as a biomarker for monitoring disease progression over time.

The present study had adequate power to robustly support the primary and most of the secondary and tertiary outcomes, in spite of sample size. The observed trend for clinical improvement among the NR-recipients with increased cerebral NAD levels is encouraging, even if it may be subject to careful interpretation due to the low number of subjects, short observation time and high interindividual variability in MDS-UPDRS scores. While we were able to confidently detect and measure total cerebral NAD-levels by 31P-MRS, the analyses were limited by the strength of the magnet used in this study as well as adhering to normal-mode SAR levels, which did not allow to confidently discriminate between the oxidized (NAD⁺) and reduced (NADH) forms. A higher field strength or more effective decoupling by increasing SAR deposition limit may make discrimination possible. More detailed analysis of the NAD⁺/NADH redox ratio in the brain upon NR treatment would improve our knowledge about the metabolic impacts of NR in the brain.

### 2.8. Conclusions

Our findings provide robust evidence of target engagement for NR treatment in PD, and suggest that it may have neuroprotective potential, by targeting multiple processes implicated in the pathophysiology of the disease. These include mitochondrial respiratory dysfunction, oxidative damage, lysosomal and proteasomal impairment, and neuroinflammation. In addition, it is possible that NR may mitigate epigenomic dysregulation in PD, by regulating histone acetylation. Genome-wide histone hyperacetylation and altered transcriptional regulation occur in the brain of individuals with PD. Increasing neuronal NAD levels would boost the activity of the NAD-dependent histone deacetylases of the sirtuin family, potentially, ameliorating histone hyperacetylation in PD. Taken together, our findings support the use of NR as a potential neuroprotective agent against PD.

**Table 1. Demographic & Anamnestic information.**

| | Placebo | NR | MRS Responders^{a} |
|---|---|---|---|
| | N = 15 | N= 15 | N = 10 |
| Sex (Male/Female): | 14/1 | 11/4 | 8/2 |
| Mean age | 63.7 ±11.0 | 63.9 ±8.0 | 63.2 ±9.8 |
| Height (cm) | 179.0 ±6.0 | 176.9 ±7.8 | 179.3 ±7.6 |
| Weight (kg) | 83.6 ±11.7 | 83.0 ±14.0 | 87.1 ±10.1 |
| Drug Compliance, % | 98.53 ±0.99 | 97.3 ±2.38 | 96.9 ±2.7 |
| Time on Study Drug (days) | 32.4 ±2.5 | 32.5 ±2.7 | 32.5 ±2.5 |
| MDS Diagnosis^{b} (Established/Probable) | 5/10 | 9/6 | 6/4 |
| Anamnestic Loss of smell (Yes/No) | 9/6 | 12/3 | 7/3 |
| Anamnestic RBD^{c} (Yes/No) | 4/11 | 3/12 | 3/7 |
| Anamnestic PD symptoms^{d} (months) | 20.3 ±13.2 | 26.6 ±11.5 | 27.8 ±12.3 |

| | | | |
|---|---|---|---|
| ^{a}Magnetic Resonance Spectroscopy Responders, ^{b}Movement Disorder Society Clinical Diagnostic Criteria, ^{c}Rapid Eye Movement Sleep Behaviour Disorder, ^{d}Time from first subjective symptom of parkinsonism to visit 1. | | | |

**Table 2. MDS-UPDRS mean clinical scores.**

| | | Placebo | NR | MRS Responders^{a} |
|---|---|---|---|---|
| | | N = 15 | N= 15 | N= 10 |
| **Mean score** ± SD | | | | |
| MDS-UPDRS^{b} I | | 6.3 | 6.3 | 6.2 |
| | Visit 1 | ±4.5 | ±4.0 | ±4.6 |
| | Visit 2 | 6.0 ±3.7 | 5.4 ±3.9 | 5.3 ±4.3 |
| MDS-UPDRS II | | 7.5 | 7.9 | 6.4 |
| | Visit 1 | ±4.4 | ±4.3 | ±4.3 |
| | Visit 2 | 7.6 ±4.6 | 8.3 ±4.3 | 6.4 ±3.8 |
| MDS-UPDRS III | | 27.0 | 31.1 | 29.6 |
| | Visit 1 | ±8.7 | ±10.0 | ±11.0 |
| | Visit 2 | 26.3 ±11.0 | 30.5 ±9.8 | 28.7 ±10.6 |
| Total score MDS-UPDRS (I-III) | | 40.7 | 45.2 | 42.2 |
| | Visit 1 | ±13.3 | ±13.2 | ±15.3 |
| | Visit 2 | 39.9 ±14.3 | 44.1 ±13.2 | 40.4 ±14.2 |
| Hoehn & Yahr^{c} Stage | | 1.9 | 1.8 | 1.7 |
| | Visit 1 | ±0.6 | ±0.4 | ±0.5 |
| Hoehn & Yahr Stage | | 1.8 | 1.9 | 1.8 |
| | Visit 2 | ±0.6 | ±0.5 | ±0.4 |

| | | | | |
|---|---|---|---|---|
| ^{a}Magnetic Resonance Spectroscopy Responders, ^{b} Movement Disorder Society Unified Parkinson's Disease Rating Scale, ^{c} Hoehn & Yahr Rating Scale | | | | |

**Table 3. Inclusion and exclusion criteria for the study of Example 2**

| Inclusion Criteria | |
|---|---|
| | - Newly diagnosed with PD |
| | - Drug naïve with respect to dopaminergic treatment |
| | - Clinical diagnosis of idiopathic PD according to the Movement Disorder Society Clinical Diagnostic Criteria for PD. |

| Exclusion Criteria | |
|---|---|
| | - [¹²³I]FP-CIT single photon emission CT (DaTscan) without evidence of nigrostriatal degeneration or denervation. |
| | - Magnetic resonance imaging (MRI) suggestive of atypical parkinsonism. |
| | - Dementia or other neurological disorder at baseline visit |
| | - Metabolic, neoplastic, or other physically or mentally debilitating disorder at baseline visit |

**Table 4. Adverse events**

| **Adverse Event** | **NR** | **Placebo** | **Causal Relationship to Study Drug (n)** | **CTCAE^{d} Grading Grade 1-5 (n)** |
|---|---|---|---|---|
| Hematoma (leg)^{c} | 2 | | Unrelated (2) | Grade 1 (1) |
| | | | | Grade 2 (1) |
| Diarrhea | | 1 | Unrelated (1) | Grade 1 (1) |
| Leg Pain^{c} | 1 | | Unrelated (1) | Grade 1 (1) |
| Headache^{a,b} | 3 | 1 | Unlikely (3) | Grade 1 (4) |
| | | | Unrelated (1) | |
| Nausea^{a} | 1 | | Unlikely (1) | Grade 1 (1) |
| Viral Gastroenteritis | | 1 | Unrelated (1) | Grade 1 (1) |
| Cholelithiasis^{b} | 1 | | Unrelated (1) | Grade 1 (1) |
| Fatique^{b} | 1 | | Unlikely (1) | Grade 1 (1) |
| Anorexia^{b} | 1 | | Unlikely (1) | Grade 1 (1) |
| Upper Respiratory infection | 1 | | Unlikely (1) | Grade 1 (1) |
| Inguinal hernia | 1 | | Unrelated (1) | Grade 1 (1) |

| | | | | |
|---|---|---|---|---|
| ^{a} Multiple adverse events registered by subject 01-020. ^{b} Multiple adverse events registered by subject 01-028. ^{c} Adverse events were secondary to muscle biopsy, ^{d} Common Terminology Criteria for Adverse Events v5.0. | | | | |

**Table 5: FDG-PET; Brain regions with reduced metabolic activity as part of the NRRP network¹**

| Brain region | Brodmann area | MNI coordinates* | | | Zmax |
|---|---|---|---|---|---|
| | | x | y | z | |
| Lentiform Nucleus (left) | Putamen | -20 | 10 | -8 | 3.5 |
| | | -28 | -2 | -2 | 3.3 |
| Lentiform Nucleus (right) | Putamen | 22 | 10 | -8 | 3.9 |
| Thalamus (left) | Anterior | -10 | -22 | 4 | 2.5 |
| Thalamus (right) | Medial Dorsal | 8 | -18 | 4 | 3.0 |
| Anterior Cingulate (left) | 32 | -4 | 44 | 18 | 2.2 |
| Anterior Cingulate (right) | 32 | 4 | 46 | -8 | 2.6 |
| | 24 | 4 | 8 | 48 | 2.3 |
| Posterior Cingulate (bilateral) | 31 | 0 | -30 | 36 | 3.5 |
| Precuneus (left) | 7 | -6 | -72 | 40 | 2.6 |
| Middle Frontal Gyrus (left) | 8 | -26 | 30 | 42 | 1.9 |
| Superior Frontal Gyrus (left) | 9 | -32 | 44 | 28 | 2.5 |
| | 10 | -28 | 56 | 6 | 2.5 |
| Prefrontal Cortex (right) | 9 | 44 | 16 | 30 | 3.0 |

| | | | | | |
|---|---|---|---|---|---|
| * Montreal Neurological Institute standard space. ¹ Voxel weights for these NRRP regions were reliable on bootstrap estimation (p<0.005; ICV range -3.95 to 4.09; 1000 iterations). [ICV = inverse coefficient of variation] | | | | | |

### Figure legends

**FIG. 3**. 31P-MRS analysis reveals NR mediated increase in cerebral NAD. (A-C) Exemplary data from one subject: **(A)** spectroscopy voxel position in the occipital cortex. Spectra were acquired for each grid position. **(B)** Averaged processed spectra from several occipital lobe voxels in black. The model fit of the data is shown in red. The model fit is composed of the convolution of all spectral contributions of a simulated dataset fitted to the experimental data shown in (C). The position of NAD+/NADH is indicated. **(C)** Phosphocreatine (PCr) was chosen as the chemical shift reference at 0 ppm. Other detected phosphometabolites are indicated. **(D)** Relative total NAD levels normalized to ATPα at visit 1 and 2. Datapoints show individual measurements (grey) and averages (black). *: p-value=0.0105 (Wilcoxon test).
**FIG. 4****. Metabolic brain network topography associated with NR treatment. (A)** NR-related metabolic pattern (NRRP) identified with FDG PET in MRS responders with negative loading (*blue*)*.* The values of region weight were threshold at a Z-value of -1.5. **(B)** Between visit changes showing increased NRRP expression in the NR versus the placebo group. The red line indicates mean values before and after the treatment. Black lines indicate individuals with a positive NAD response in the MRS analysis. Grey lines indicate the remaining individuals (i.e., the four individuals who either showed no NAD response in the MRS, (n=2), or for whom MRS data was not available (n=2)). Refer to **FIG. 8** for the expression of the ordinal trend associated with the identification of NRRP in the 10 NR subjects with increased cerebral NAD levels (i.e., MRS responders). *: p =0.027 (permutation test, 1000 iterations). **(C)** NR-related changes in UPDRS motor ratings and NRRP expression correlated in the NR group (r=-0.590, p=0.026) including the MRS responders (*filled circles*) and the others (*open circles*)*.* The correlation was still significant (r =-0.556, p=0.048) after excluding data point with the greatest increase in UPDRS motor score at the top left corner of the graph. **(D)** Top: PD-related metabolic pattern (PDRP) with positive (*hot*) and negative (*purple*) loadings previously identified with FDG PET in the American patient population^{32,49}. Bottom: composite imaging display showing overlap in putamen and pallidum or prefrontal cortex and precuneus between NRRP (*blue*) and PDRP (*hot or purple*)*.* **(E-F)** Correlations of NRRP and PDRP expression scores at baseline (r=0.546, p=0.002) and corresponding UPDRS motor ratings and PDRP expression at baseline (r=0.374, p=0.046) in the combined NR (*filled circles*) and PL (*open circles*) group.
**FIG. 5****. NR augments the NAD metabolome in CSF and peripheral tissues of PD patients.** The graph shows NAD⁺ biosynthesis and degradation routes including precursors, intermediates, and catabolic end products. Boxed plots show concentrations in placebo and NR treated individuals at visit 1 and visit 2 for those detectable metabolites that changed significantly with treatment in muscle, PBMCs and CSF. Metabolites that changed significantly with NR treatment are highlighted in green and underlined. Datapoints show individual measurements (grey) and averages (black). NA: nicotinic acid, NAR: nicotinic acid riboside, Nam: nicotinamide, NR: nicotinamide riboside, NAMN: nicotinic acid mononucleotide, NMN: nicotinamide mononucleotide, NAAD: nicotinic acid adenine dinucleotide, NAD: nicotinamide adenine dinucleotide, 1-Me-Nam: methyl nicotinamide, Nam-N-oxide: nicotinamide N-oxide, Me-2-PY: N-methyl-2-pyridone-5-carboxamide, Me-2-PY: N-methyl-4-pyridone-5-carboxamide. *: p<0.05, **: p<0.01, ***: p<0.001 (Wilcoxon test).
**FIG. 6****: Serological markers affected by NR treatment. (A-C)** Serological marker analysis in serum and CSF of PD patients in the placebo (PL) and NR-treated (NR) group before (V1) and after (V2) treatment. Datapoints show individual measurements (grey) and averages (black). Levels of GDF15 in Serum (A) and CSF (B), and serum levels of FGF21 (C) are shown. **(D-E)** Neurofilament light (Nf-L) chain levels in serum and CSF. **(F-O)** Cytokine analysis in serum (F-K) and CSF (L-O). The plots show the cytokines that changed only, or with stronger significance in the NR group. *: p<0.05, **: p<0.01, ***: p<0.001 (Wilcoxon test).
**FIG. 7****: CONSORT flow diagram.** 36 Patients with PD were screened for inclusion. Four subjects did not meet the inclusion criteria for the study. Two subjects declined to participate in the study because they wished to commence dopaminergic treatment. None of the included randomized participants withdrew from the study.
**FIG. 8****: Changes in NRRP expression in the MRS responders treated with NR.** NRRP expression increased in 8 of 10 subjects (p =0.018, permutation test, 1000 iterations) showing elevated occipital NAD levels. Individual (gray lines) and average values (black line) are shown.
**FIG. 9****: Whole-brain displays of NRRP and PDRP brain network topographies in FDG PET.** Top: NR-related metabolic pattern (NRRP) identified in the MRS responders with negative loading (*blue*)*.* Middle: PD-related metabolic pattern (PDRP) with positive (*hot*) and negative (*purple*) loadings previously identified and validated in the American patient population. Bottom: composite overlay showing the overlap in putamen and pallidum or prefrontal cortex and precuneus between NRRP (*blue*) and PDRP (*hot or purple*)*.* All patterns were displayed on the standardized MRI brain template and thresholded at the Z-values that were highly reliable based on the bootstrap analysis (p < 0.005, 1000 iterations).
**FIG. 10****: Metabolomic analysis in muscle. Related to** **FIG. 5****.** Boxed plots show concentrations for all examined and detectable metabolites in muscle tissue from placebo (PL) and NR treated (NR) individuals at visit 1(v1) and visit 2 (v2). Plots of metabolites that changed significantly with NR treatment are highlighted in green and underlined, and are also presented in **FIG. 5**. NA: nicotinic acid, NAR: nicotinic acid riboside, Nam: nicotinamide, NR: nicotinamide riboside, NAMN: nicotinic acid mononucleotide, NMN: nicotinamide mononucleotide, NAAD: nicotinic acid adenine dinucleotide, NAADP: nicotinic acid adenine dinucleotide phosphate, NAD(P)⁺: oxidized nicotinamide adenine dinucleotide (phosphate), NAD(P)H: reduced nicotinamide adenine dinucleotide (phosphate), 1-Me-Nam: methyl-nicotinamide, Nam-N-oxide: nicotinamide N-oxide, Me-2-PY: N-methyl-2-pyridone-5-carboxamide, Me-2-PY: N-methyl-4-pyridone-5-carboxamide. SAM: S-adenosyl methionine, SAH: S-adenosyl homocysteine, HCy: homocysteine. AMP: adenosine monophosphate, ADP: adenosine diphosphate, ATP: adenosine triphosphate, GTP; Guanosine triphosphate, GDP: guanosine diphosphate, CoA: Coenzyme A, Acetyl-CoA: acetyl-Coenzyme A.
**FIG. 11****. Metabolomic analysis in PBMCs. Related to** **FIG. 5****.** Boxed plots show concentrations for all examined and detectable metabolites in PBMCs from placebo (PL) and NR treated (NR) individuals at visit 1(v1) and visit 2 (v2). Plots of metabolites that changed significantly with NR treatment are highlighted in green and underlined, and are also presented in **FIG. 5**. Similar to muscle, levels of NAD were lower than previously reported concentrations (Elhassan et al., 2019; Martens et al., 2018; Trammell et al., 2016). NA: nicotinic acid, Nam: nicotinamide, NR: nicotinamide riboside, NAMN: nicotinic acid mononucleotide, NMN: nicotinamide mononucleotide, NAAD: nicotinic acid adenine dinucleotide, NAADP: nicotinic acid adenine dinucleotide phosphate, NAD(P)⁺: oxidized nicotinamide adenine dinucleotide (phosphate), NAD(P)H: reduced nicotinamide adenine dinucleotide (phosphate), 1-Me-Nam or Me-Nam: methyl-nicotinamide, SAM: S-adenosyl methionine, SAH: S-adenosyl homocysteine, HCy: homocysteine. AMP: adenosine monophosphate, ADP: adenosine diphosphate, ATP: adenosine triphosphate, GTP; Guanosine triphosphate, GDP: guanosine diphosphate, CoA: Coenzyme A, Acetyl-CoA: acetyl-Coenzyme A.
**FIG. 12****: Cell composition of the PBMC fraction is unaffected by NR treatment. (A)** Deconvolution of immune cell types using ABIS for all RNA-seq PBMC samples. Samples are stratified by treatment (Placebo: left panel, NR: right panel) and visit (V1: red, V2: blue (*)). **(B)** Summarized per-sample cellularity represented in the two first principal components of the cell type proportions. Paired samples belonging to the same subject (V1 and V2) are joined with a segment. Percentage of variance explained by each component is indicated.
**FIG. 13****: Cytokine levels in NR and placebo treated participants. Related to** **FIG. 6**. (A) Fold change comparison of cytokines that showed significant changes in serum in both the placebo and NR group. **(B-C)**. Concentrations in serum **(B)** and CSF **(C)** from placebo (PL) and NR treated (NR) individuals at visit 1 (v1) and visit 2 (v2) are shown for detectable cytokines not presented in **FIG. 6**. Datapoints show individual measurements (grey) and averages (black). *: p<0.05, **: p<0.01, **: p<0.001.
**FIG. 16****:** NAD measurement in PBMCs from the study cohort, using the NADMed method. NR treatment increases total NAD levels in PBMCs, but increase does not reach statistical significance. On the group level, increase in the NR group is significantly different from placebo group (p= 0.0256).

### EXAMPLE 3: Trial of NR as a neuroprotective therapy for PD

To further test NR as a neuroprotective therapy for PD, and in particular to assess NR in delaying nigrostriatal degeneration or denervation, and clinical disease progression in patients with early PD, a multi-centre phase II randomized double-blinded clinical trial is performed, comparing NR to placebo in individuals with early stage PD.

### 1. Study objectives and related endpoints

Primary objective is to determine whether NR delays disease progression in PD, as measured by the difference between the NR and placebo groups in total MDS-UPDRS (part I - IV) score change after 52 weeks of follow-up. Secondary objective is to determine whether NR:
- Improves and/or prevents specific motor, non-motor and cognitive symptoms and/or improves patient quality of life in PD as measured by individual subsections of the MDS-UPDRS (e.g. MDS-UPDRS part I, II, III and IV), as well score difference measured by NMSQ, NMSS, Hoehn&Yahr, MoCA and EQ-5D.
- Delays nigrostriatal degeneration or denervation, as measured by DaTscan
- Delays brain atrophy (global or focal) as measured by MRI volumetry
- Rectifies NAD metabolism and mitochondrial function as measured by multi-omics in patient biosamples and brain (31)P-MRS
- Corrects aberrant histone acetylation and gene expression profile as measured by multi-omics in patient biosamples
- Improves brain spatiotemporal functional and structural connectivity measured by fMRI.
- Delays the progression of neuronal loss, measured by neurofilament light-chain in patient serum.

The primary endpoint is the between-group difference of change in total MDS-UPDRS (part I - IV) score after 52 weeks of treatment, comparing the active NR arm versus placebo arm. Secondary endpoints are differences between the NR and placebo arm in:
- Individual subsections of the MDS-UPDRS (e.g., MDS-UPDRS part I, II, III and IV), as well score difference measured by NMSQ, NMSS, Hoehn&Yahr, MoCA and EQ-5D
- Dopamine transporter density as measured by DaTscan.
- Brain volume (total and area specific) measured by MRI
- Brain spatiotemporal functional and structural connectivity measures using MRI and fMRI
- Default resting state measured using fMRI
- NAD metabolites measured by mass spectrometry (LC-MS/MS Q-Exactive HF)
- Epigenomic profile: histone acetylome (ChIP-Seq) for selected markers
- Transcriptome (RNA-seq)
- Proteome (LC-MS) or
- Neurofilament light-chain difference, measured in plasma.

### 2. Study population

400 patients are included in this trial.

### 2.1. Inclusion Criteria

All of the following conditions must apply to the prospective patient at screening prior to receiving study agent:
- Have a clinical diagnosis of idiopathic PD according to the MDS criteria
- Positive [¹²³I]FP-CIT single photon emission CT (DAT-scan), confirming nigrostriatal degeneration or denervation
- Diagnosed with PD within two years from enrolment
- Hoehn and Yahr score < 3 at enrolment
- Optimal symptomatic therapy, not requiring adjustments, for at least 1 month
- Age equal to or greater than 35 years at time of enrolment.
- The study includes only idiopathic PD. No individuals with genetic conditions or environmental risks known to influence the proteasome 26S/20S function are included.

### 2.2. Exclusion Criteria

Patients are excluded from the study if they meet any of the following criteria:
- Dementia or other neurodegenerative disorder at baseline visit
- Diagnosed with atypical parkinsonism (PSP, MSA, CBD) or vascular parkinsonism
- Any psychiatric disorder that would interfere with compliance in the study.
- Any severe somatic illness that would interfere with compliance and participation in the study.
- Use of high dose vitamin B3 supplementation within 30 days of enrolment.
- Metabolic, neoplastic, or other physically or mentally debilitating disorder at baseline visit.
- Genetically confirmed mitochondrial disease.

### 3. Treatment

NR (provided as Nicotinamide riboside chloride, Niagen^{®} from Chromadex) is defined as Investigational Product(s) (IP). IMP includes also active comparator and placebo. NR (Niagen^{®} from Chromadex) and placebo are manufactured and provided from Chromadex. Both NR and Placebo are prepared as identical capsules. Both the NR and placebo are to be stored in room temperature with temperature < 25 °C.

### 3.1. Dosage and Drug Administration

Each capsule of NR contains 250 mg of NR. Patients are administered orally 2 capsules (each 250 mg) two times a day (1000 mg daily total) for the duration of the study (52 weeks). Placebo capsules are administered similarly (2 capsules two times a day). The study medication is to be taken every day during the treatment period, including prior to study visits and imaging.

### 3.2. Duration of Therapy

Therapy duration for the study is up to 52 weeks.

### 3.3. Dopaminergic Therapy During Screening and IP treatment period

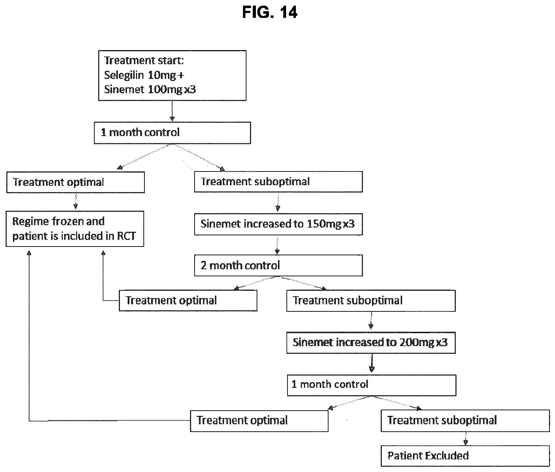

Eligible and consenting men and women with PD are given dopaminergic therapy plus MAO-B inhibitor titrated to optimal clinical effect. The treatment regime is then frozen and will remain unchanged for the study period (52 weeks). Newly diagnosed and/or treatment naive patients are given Selegiline 10 mg/day PO and Sinemet^{®} (levodopa 100mg + carbidopa 25mg) or Madopar^{®} (levodopa 100mg + benserazide 25mg) 100/25mg × 3 a day at the first screening visit. Treatment efficacy is assessed upon re-examination every 1 month. If adequate symptomatic relief is not achieved, the dopaminergic therapy may be increased to 150 mg x3 levodopa until optimal effect or a maximum dose of Sinemet^{®}/Madopar^{®} 200mg × 3. if adequate symptomatic relief is not achieved on this dose, the patient is excluded and further followed-up at the regular outpatient service. Once optimal effect is reached (i.e. stable treatment for at least 1 months, the regime is frozen for the duration of the study period (52 weeks), see **FIG. 14**. If adverse effects occur due to the dopaminergic therapy after enrolment, the treatment is adjusted according to good clinical practice. At the end of study visit (week 52), the physician determines (yes/no) whether the patient is still adequately treated for his/her parkinsonism with their current dopaminergic treatment.

### 3.4. Concomitant Medication

There are no restrictions on any other use of medications. All patients should use medications prescribed prior to enrolment in the study. There are no restrictions with respect to start new medications that are necessary for the patient. The Patient should not take any Vitamin B3 supplements for the duration of the study. All concomitant medication (incl. vitamins (exception Vitamin B3, herbal preparation and other "over-the-counter" drugs)) used by the patient are recorded in the patient's file and CRF.

### 4. Study procedures

### 4.1. Flow Chart

**Table 6. Trial flow chart**

| | Screening Period | | Treatment Period Study Visit: | | | | | End of study visit |
|---|---|---|---|---|---|---|---|---|
| Time | First Screening | Last screening¹ | Week 0/ baseline | Week 4 | Week 13 | Week 26 | Week 39 | Week 52 |
| Informed consent | X | | X | | | | | |
| Inclusion/exclusion Evaluation | X | X | X | | | | | |
| Informed consent biobank, optional | | | X | | | | | |
| Anamnestic information | X | | X | | | | | |
| Physical Examination³ | X | X | X⁷ | | | | | |
| Record of concomitant medication | X | X | X | X | X | X | X | X |
| Medical history | X | X | X | | | | | |
| DatScan/MRI² | | | X | | | | | X |
| Vital signs⁵ | | | X | X | X | X | X | X |
| MDS-UPDRS | | | X | X | X | X | X | X |
| NMSS & NMSQ | | | X | | | X | | X |
| MOCA | | | X | | | | | X |
| EQ-5L | | | X | | | | | X |
| Blood samples⁶ | | | X | X | X | X | X | X |
| Treatment (IP) administration/dispensation | | | X | | | | | |
| Dopaminergic treatment stable⁴ | X | | | | | | | X |
| Adverse event | | | | X | X | X | X | X |

| | | | | | | | | |
|---|---|---|---|---|---|---|---|---|
| ¹ The patient has to be on a stable dopaminergic treatment. When the patient is on a stable dopaminergic treatment then screening is over and patient can be included to the study. The Dopaminergic treatments and its flow chart is listed in section: 3.3. ² DatScan and MRI should be performed within 2 weeks prior to Study Visit. ³ Generall Neurological examination. ⁴ See section 3.3. ⁵ Anamnestic information includes: Family history of Neurological illness, smoking history, anamnestic months since first clinical PD symptoms, Occurrence and duration of REM sleep disorder symptoms, occurrence and duration of loss of smell. ⁶ Blood Pressure, Pulse, Body Weight. ⁷ CRP, ALAT, ASAT, GT, Bilirubin, ALP, Creatinin, Urea, RBC, Hb, WBC with differential, Platelets, CK, FT4, TSH, B12, Folic acid, homocysteine, Methylmalonic acid, Sodium, Potassium, Calcium (ionized). Biobanking: see lab manual for details. ⁸ Height (measured at Baseline visit) | | | | | | | | |

### 4.2. By Visit

### 4.2.1. Screening Visits/ Before start of Investigational Product (IP)

**The first screening visit** aims to determine if patient is eligible to be included for the study. A full physical examination and anamnestic medical history is performed. If the patient fulfils the inclusion/exclusion criteria and gives informed consent, the patient is put on one of the three dopaminergic treatments. See Dopaminergic treatment flowchart in section 3.3.

**Last screening:** Patient is then called back for a new screening visit 1 month later. If the PD patient is optimally treated, this dopaminergic treatment is then frozen for the remainder of the study. The patient is then referred to DatScan and MRI examination. The patient is then called in for the baseline study visit (week 0 study visit). The Baseline study visit should be within 2 weeks since the DatScan was performed. The patient should at screening be advised to stop using any Vit B3 supplement to fulfil inclusion criteria.

**Baseline/Week 0:** At the first study visit the investigator needs to verify the informed consent for study and offer the subject to sign the informed consent for storage and analysis of biological material. Anamnestic information is gathered at screening, current use of medication and medical history are verified. Fulfilment of inclusion and exclusion criteria are verified. If the subject is enrolled (fulfils the inclusion/exclusion criteria) then the subject is randomized to study medication in the CRF. The patient is instructed to take the study medication every day for the remainder of the study. The study medication is to be taken 2 capsules two times a day (2 capsules × 2), morning and evening. There is no specified time of day the dosages should be taken, only that they should be taken with about 12 hours apart if possible. Should a dosage be forgotten then the patient can take the forgotten dosage if it shorter time to the forgotten dosage than to the next scheduled dosage. There are no restriction with respect to other medication or to take it together with food.

### 4.2.2. During treatment

See flowchart (section 4.1) for which clinical examinations are performed at each study visit.

### 4.3. MRI protocol

Equipment: Scanner: Siemens Biograph mMR (PET/MR); Software: E11P; Coils: Siemens mMR Head/Neck coil and multi-core coil 31P 1H head coil (Rapid).

Protocol: Positioning: Localizer; Autoalign if possible. MRI recording: 3D T1 (sagital, 1x1x1 mm); 3D T2 FLAIR (sagital, 1x1x1 mm); 2D T2 axial (4 mm slice thickness, vinkle i forhold til CC /ACPC); 2D DTI (axial, 2x2x2 mm, vinkle i forhold til CC /ACPC); (DWI med 3 retninger dersom ikke DTI mulig).

Extended protocol Bergen: 2D fMRI resting state (2.4 × 2.4x 3 mm, axial slices, angle relative to CC /ACPC); MRS recording: CSI (15 min), multi-core coil. Total recording time: 60 min including CSI.

Comments: Eyes closed for fMRI-recording.

### 5. Safety and tolerability assessments

Safety requirements to be fulfilled preferably comprise those based on assessment of biochemistry (by routine blood analysis); vital signs (pulse, blood-pressure); and/or registration of adverse events. At different time points during the clinical trial, the following biological samples are collected, processed and stored based on standard operating procedures: whole blood, serum, plasma, PBMCs, blood cells.

### 6. Statistical methods and data analysis

### 6.1. Determination of Sample Size; Randomization; Population for Analysis

Power analyses were based on ParkWest, a prospective longitudinal cohort study of patients with Parkinson disease from western Norway and the DATATOP study testing the neurorprotective effect of selegiline in PD. We selected 150 PD patients from the ParkWest cohort who had been drug naive for dopaminergic treatment at the time of inclusion and calculated the mean UPDRS change in a 52 week period, after dopaminergic treatment was commenced. Since this reflects the natural evolution of PD under optimal dopaminergic therapy, we considered this progression rate to be representative of the placebo group in the present study. The mean increase of UPDRS in this group after 52 weeks was 7.6 ± 6.3 units. The DATATOP study showed a 50% decrease of motor decline in PD patients using L-Deprenyl (selegiline) compared to tocopherol. Therefore, a conservative estimate for our study is a decrease of 2 MDS-UPDRS points among NR users compared to placebo, during 52 weeks.

### 6.2. Analyses

### 6.2.1. Primary analysis

Under the intention-to-treat principle, all randomized patients are included in the primary analyses. As the MDS-UPDRS is repeated measures dependent variable, a linear mixed model with intraindividual random effect added to the model is performed. The mean change in MDS-UPDRS during the repeated study visits is compared. The primary analysis is unadjusted for covariates. The missing data is assumed to be Missing at Random (MAR) or Missing Completely at Random (MCAR).

### 6.2.2. Sensitivity analysis

To assess the robustness of the primary analysis, several sensitivity analyses are performed. Analysis is performed using linear mixed models unless otherwise specified. Unless specified, the NR arm and the placebo arm are compared.
- Analyses with and without outliers are performed. Outliers are defined by boxplot assessment.
- The analysis with Covariates is performed: Age, Hohn & Yahr stage, Gender as covariates (fixed effects).
- Analysis with covariates with and without adjusting for center together with analysis
- Analysis with and without adjustment for baseline characteristics such as initial MDS-UPDRS score, initial Hoehn & Yahr stage and levodopa dosage is performed during the study.
- As-treated analysis is performed. Only subjects who completed the study (completed study visit week 52) as well as having drug compliance >90%. This is done unadjusted and as well as with covariates and site covariate.
- Only complete cases with drug compliance >90% are analyzed. This is done unadjusted and as well as with covariates and site covariate.
- The missing data is imputed using multiple imputation methods and redo the analysis stated above. The missing data is assumed to be MAR. Model based multiple imputation are used where the number of imputations used is the largest value between 10 and the proportion of missing values *100.
- If there is non-normality, log and logit transformations are applied to abovementioned analysis.
- Analysis is also performed using repeating measurement ANOVA as well as linear regression and ANCOVA comparing mean difference between Baseline and Week 52 MDS-UPDRS scores.

### 6.2.3. Secondary analyses

- Mean difference for each MDS-UPDRS Parts I-III using linear mixed model. Intraindividual random effect are added to the model. Unadjusted and adjusted analysis is performed using covariates as stated in primary sensitivity analysis.
- Mean difference between the NMSQ total score using linear mixed model with added intraindividual random effect to the model. Unadjusted and adjusted analysis are performed using covariates as stated in primary sensitivity analysis.
- Mean difference between the NMSS total score using linear mixed model with added intraindividual random effect to the model. Unadjusted and adjusted analysis are performed using covariates as stated in primary sensitivity analysis.
- Paired t-test for mean difference in total MOCA score between the NR and Placebo arm.
- Paired t-test for 5Q-5L questionnaire score between NR and placebo arm.
- Generalized estimating equation with Hohn & Yahr stage as categorical dependent variable. This is performed unadjusted and adjusted.
- Kaplan Meyer and Cox regression model where the event is defined as an increase of Hohn & Yahr stage by 1 point. The Cox regression model includes covariates as stated in sensitivity analysis for the primary analysis.
- Compare baseline and 52 Week DatScan with respect to mean difference in nigrostriatal metabolism between the NR and Placebo arm.
- Perform volumetric analysis between the Baseline and 52 week MRI imaging with respect to volumetric changes in cortex as well as basal ganglia in brain regions of interest comparing the NR and placebo arm.
- Comparison of adverse events reported between the NR and placebo arm using chi-squared test.
- Single- and multi-omics analyses are performed.

### 6.2.4. Subgroup analysis

- To stratify the Primary analysis of mean MDS-UPDRS change between the NR and placebo arm, a stratified analysis comparing the tremor dominant and PIGD dominant (defined by MDS-UPDRS) subgroups in NR and placebo arm is performed. Linear mixed model is used with intraindividual random effect added to the model.
- If applicable: stratification of the primary analysis with a subpopulation of NR that has improvement in 52 Week DatScan compared to baseline.
- If applicable: post-hoc stratification of NR subgroup based upon a single or combination of metabolomic, epigenomic and transcriptomic factors.
- Compare subjects who have performed fMRI to see if there are changes in the default resting state network comparing the NR arm and Placebo arm.

### 6.3. Statistical Analysis

**Table 7. Statistical analysis**

| | | |
|---|---|---|
| Primary analysis: | Total MDS-UPDRS score. Assessed by the MDS-UPDRS questionnaire. | Mean change in total MDS-UPDRS score between the NR and Placebo arm. |
| Sensitivity analysis: | Total MDS-UPDRS score. Assessed by the MDS-UPDRS questionnaire. | Mean change in total MDS-UPDRS score between the NR and Placebo arm. |
| Secondary analysis: | Each subsection I-III of MDS-UPDRS. Assessed by the MDS-UPDRS questionnaire. | Mean change in each subsection I-III MDS-UPDRS score between the NR and Placebo arm. |
| | NMSQ total score. Assessed by the NMSQ questionnaire. | Mean change in NMSQ total score between the NR and Placebo arm. |
| | NMSS total score. Assessed by the NMSS questionnaire. | Mean change in NMSS total score between the NR and Placebo arm. |
| | MoCA total score. Assessed by the MoCA questionnaire. | Mean change in MoCA total score between the NR and Placebo arm. |
| | EQ-5L score. Assessed by the EQ-5L questionnaire. | Mean change in EQ-5L total score between the NR and Placebo arm. |
| | Hoehn & Yahr Stage. Assessed by the Hoehn & Yahr stage in MDS-UPDRS. | Mean change in Hoehn & Yahr stage between the NR and Placebo arm. |
| | | Lower number of stage increases (nominal) in Hoehn and Yahr in the NR arm compared to the placebo arm. |
| | Nigrostriatal metabolic DAT change between baseline and week 52. Measured by DatScan imaging. | Lower nigrostriatal deterioration measured by DatScan in the NR arm vs. the placebo arm. |
| | Volumetric cortical thickness in brain regions of interest. Measured by MRI. | Lower cortical atrophy measured by cortical thickness with MRI in the NR arm vs. the placebo arm. |
| Subgroup analysis: | Total MDS-UPDRS score. Assessed by the MDS-UPDRS questionnaire. | Mean change in total MDS-UPDRS score between the NR and Placebo arm. |
| | Difference in default resting state network | Difference in the default restating state network comparing Baseline and 52 Week fMRI measurement between the NR and placebo arm. |

### 6.4. Outcomes

Primary outcome: The between-group (NR vs. placebo) difference of change in total MDS-UPDRS (part I - IV score) after 52 weeks of follow-up.

Key secondary outcomes: Differences between the NR and placebo groups in:
- Long-term safety and tolerability of treatment.
- Individual subsections of the MDS-UPDRS (e.g. MDS-UPDRS part I, II, III and IV), as well score difference measured by NMSQ, NMSS, Hoehn&Yahr, MoCA and EQ-5D.
- Clinical laboratory values.
- Dopamine transporter density as measured by DaTscan
- Brain volume (total and area specific) measured using MRI.
- Default resting state measured using fMRI.
- Brain spatiotemporal functional and structural connectivity measures using MRI and fMRI.
- In vivo levels of cerebral NAD and other neurochemistry compounds using dual tuned MRS acquisitions ([1H]/(31P]).
- NAD metabolome measured by mass spectrometry (LC-MS/MS Q-Exactive HF)
- Epigenomic profiling: histone acetylome (ChlP-Seq)
- Transcriptome (RNA-seq)
- Proteome (LC-MS)
- Transcriptome (RNA sequencing)
- Neurofilament light-chain difference, measured in serum
- Stratified analysis comparing the NR and placebo arm with respect to MDS-UPDRS. Stratifying variables based on metabolic or radiological parameters.

The present study is a randomized, double-blind phase II trial, where a total of 400 individuals with PD receive 1000mg NR or placebo for a period of 52 weeks. Extensive medical testing and screening, including brain MRI, is performed both at baseline and end-of-study (i.e., after 52 weeks of exposure). Among the 80 individuals who have completed the study and performed brain MRI scans at baseline and week 52, there have been no cases of glioma or any other brain neoplasms. The therapy can therefore be considered not to be associated with an increase in glioma or brain neoplasm prevalence over the duration of treatment.

### EXAMPLE 4: FOLLOW-UP STUDY OF DIFFERENCE IN THE PROGRESSION OF THE DAT LOSS

Current evidence; e.g., from Example 2, does not support a hypothesis that NR may adversely influence DA metabolism in patients with PD.

In the study of Example 2, 30 individuals with PD were randomized on NR 1000 mg daily (n = 15) or placebo (n = 15) and followed for one month. All examinations and biological sampling and analyses were conducted at baseline and after a month of treatment.

Metabolomic analyses in peripheral blood mononuclear cells (PBMC) and skeletal muscle biopsies from the NAD-PARK cohort (n = 30), showed no significant changes in SAM or its related metabolites SAH and homocysteine, indicating that NR supplementation does not limit SAM availability for other vital reactions.

The PD patients who received NR showed no worsening of motor symptoms - as would have been expected upon DA depletion - but rather exhibited a mild clinical improvement, as measured by the UPDRS scale.

In the study of the present Example 4, 21 of the participants from the study of Example 2 (9 receiving placebo and 11 receiving NR) were examined by a second DAT-scan 1-1.5 year after the completion of the study, for entry into the study of Example 3. Comparing the first and second DAT-scans showed no significant difference in the progression of the DAT loss (i.e., nigrostriatal degeneration or denervation) between the NR and placebo group. In fact, the NR group exhibited a non-significant trend for decreased progression, as evident by a smaller difference between the two examinations **(****FIG. 15****)**.

To further investigate this, the DA metabolome in cerebrospinal fluid (CSF) of the Example 2 cohort can be measured, comparing between visits. In the phase II study of Example 3, controlling for this event by clinical measures, biochemical measures (metabolomics as described above) and DAT-scan at baseline and after one year of treatment is performed.

**FIG. 15** represents box plots showing the change (delta) of the DAT-scan tracer uptake in the posterior putamen between the second and first examination (delta = scan2 - scan1). The NR group shows a trend for decreased progression of the nigrostriatal denervation, but this difference is not significant (t-test, p = 0.35). The data is normally distributed (Shapiro-Wilk normality test).

### EXAMPLE 5: SAFETY STUDY INVESTIGATING TREATMENT WITH HIGH-DOSE NICOTINAMIDE RIBOSIDE (NR) IN PARKINSON'S DISEASE

Nicotinamide riboside (NR) is fully approved for human use and no evidence of toxicity has been found. NR has undergone extensive preclinical testing and is Generally Recognized as Safe (GRAS) for use in food products by the United States Food and Drug Administration and by the European Food Safety Authority. NR is well tolerated with no evidence of toxicity in adult humans with doses up to at least 2000 mg daily. We therefore propose that dosages up to 3000 mg is highly unlikely to cause evidence of toxicity. Active study drug capsules contain 250 mg NR (as chloride). Placebo contains microcrystalline cellulose, which are identical in appearance and taste.

We propose that oral administration of the NAD precursor NR (Niagen^{®}, Chromadex) in dosages of up to 3000 mg daily is unlikely to cause moderate or severe side effects as measured biochemically and physiologically, when administered short-term for 4 weeks. We propose that it is unlikely to have significant tolerability issues for treated individuals. To enable estimating the optimal biological dose for NR, we perform a single center, randomized double-blinded safety study.

### 1. Protocol Synopsis

| | | |
|---|---|---|
| **Investigational Product (IP) (including active comparator and placebo):** | | Nicotinamide Riboside |
| | | Placebo |
| **Treatment Duration:** | | 4 weeks (observation period 5 weeks) |
| **Objectives** | **Primary Objective:** | |
| | To determine the safety of oral NR 3000 mg daily for a period of 4 weeks in individuals with Parkinson's disease (PD). Safety is defined as the absence of NR-associated moderate or severe adverse events (AE). | |
| | **Secondary objectives:** | |
| | | 1) Determine whether oral NR 3000 mg daily is associated with mild AE. |
| | | 2) Assess the effects of oral NR 3000 mg daily on the NAD metabolome in blood and urine. |
| | | 3) Assess the effects of oral NR 3000 mg daily on clinical severity of PD, measured by UPDRS. |
| | **Exploratory objectives:** | |
| | | 1) Assess the effects of oral NR 3000 mg daily on serum homocysteine levels. |
| | | 2) Assess the effects of oral NR 3000 mg daily on fasting blood glucose and serum insulin levels. |
| **Outcomes:** | **Primary Outcomes** | |
| | | Between-group (NR vs placebo) difference in treatment-associated moderate and severe AEs. |
| | **Secondary outcomes:** | |
| | | 1) Between-group difference in treatment associated mild AEs. |
| | | 2) Between-group difference in changes of the NAD metabolome in blood and urine, measured by mass spectrometry (LC-MS/MS Q-Exactive HF). |
| | | 3) Between-group difference in change of clinical severity of PD, measured by UPDRS. |
| | **Exploratory outcomes:** | |
| | | 1) Between-group difference in the change of serum homocysteine levels. |
| | | 2) Between-group difference in the change of fasting blood glucose and serum insulin levels. |
| **Study Design:** | Single center, double-blinded, randomized, placebo controlled | |
| **Main Inclusion Criteria:** | | • Age equal to or greater than 35 years and lower than 100 years at time of enrolment. |
| | | • Clinical diagnosis of idiopathic PD according to the MDS criteri. |
| | | • Hoehn and Yahr score < 4 at enrolment. |
| **Main Exclusion Criteria:** | | • Dementia or other neurodegenerative disorder at baseline visit. |
| | | • Any psychiatric disorder that would interfere with compliance in the study. |
| | | • Any severe somatic illness that would make the individual unable to comply and participate in the study. |
| | | • Use of high dose vitamin B3 supplementation within 30 days of enrolment |
| | | • Metabolic, neoplastic, or other physically or mentally debilitating disorder at baseline visit. |
| **Sample Size:** | 20 patients (10 in placebo group, 10 in treatment group) | |
| **Efficacy Assessments:** | Clinical laboratory values, vital parameters and self reported adverse effects. | |
| **Safety Assessments:** | Biochemistry: Routine blood analysis. | |
| | Vital signs: Pulse, Blood-pressure. | |
| | Registration of adverse events. | |

### 2. Study Objectives and Related Endpoints

### 2.1. Primary Objective

To determine the safety of oral NR 3000 mg daily for a period of 4 weeks in individuals with Parkinson's disease (PD). Safety is defined as the absence of NR-associated moderate or severe adverse events (AE).

### 2.2. Secondary Objectives

1) Determine whether oral NR 3000 mg daily is associated with mild AE.
2) Assess the effects of oral NR 3000 mg daily on the NAD metabolome in blood and urine.
3) Assess the effects of oral NR 3000 mg daily on clinical severity of PD, measured by UPDRS.

### 2.3. Exploratory Objectives

1) Assess the effects of oral NR 3000 mg daily on serum homocysteine levels.
2) Assess the effects of oral NR 3000 mg daily on fasting blood glucose and serum insulin levels.

### 2.4. Primary Outcome

Between-group (NR vs placebo) difference in treatment-associated moderate and severe AEs baseline.

### 2.5. Secondary Outcomes

1) Between-group difference in treatment associated mild AEs.
2) Between-group difference in changes of the NAD metabolome in blood and urine, measured by mass spectrometry (LC-MS/MS Q-Exactive HF).
3) Between-group difference in change of clinical severity of PD, measured by UPDRS.

### 2.6. Exploratory Outcomes

1) Between-group difference in the change of serum homocysteine levels.
2) Between-group difference in the change of fasting blood glucose and serum insulin levels.

### 3. Overall Study Design

This is a single center, phase I double blinded, randomized, placebo controlled drug-safety study.

### 4. Study Population

### 4.1. Number of Patients

20 patients are included in this study: 10 receiving 3000 mg oral NR and 10 receiving placebo.

### 4.2. Inclusion Criteria

The following condition must apply to the prospective patient at screening prior to receiving study agent:
Age equal to or greater than 35 and less than 100 years at time of enrolment.

Have a clinical diagnosis of idiopathic PD according to the MDS clinical diagnostic criteria for Parkinson's disease (Postuma RB, Berg D, Stern M, et al. MDS clinical diagnostic criteria for Parkinson's disease. Mov Disord 2015;30(12):1591-601; Postuma RB, Berg D. The New Diagnostic Criteria for Parkinson's Disease. Int Rev Neurobiol 2017;132:55-78).

Hoehn and Yahr score < 4 at enrolment.

### 4.3. Exclusion Criteria

Patients are excluded from the study if they meet any of the following criteria:
- Dementia or other neurodegenerative disorder at baseline visit.
- Any psychiatric disorder that would interfere with compliance in the study.
- Any severe somatic illness that would make the individual unable to comply and participate in the study.
- Use of high dose vitamin B3 supplementation within 30 days of enrolment
- Metabolic, neoplastic, or other physically or mentally debilitating disorder at baseline visit.

### 5. Treatment

For this study NR (Niagen^{®}, Chromadex) is defined as the Investigational Product(s) (IP). IP includes also active comparator and placebo.

### 5.1. Drug Identity, Supply and Storage

NR and Placebo are prepared as identical capsules. The NR and placebo have a 1-year expiry date. Both the NR and placebo are stored in room temperature with temperature <25.

### 5.2. Dosage and Drug Administration

Each capsule of NR contains 250 mg of NR. Patients administer orally 6 capsules (1500 mg) two times daily (3000 mg daily total) for the duration of the study (4 weeks). Placebo capsules are administered similarly (6 capsules two times daily). The study medication is to be taken every day during the treatment period, including prior to study visits.

### 5.3. Duration of Therapy

Therapy duration for the study is 4 weeks.

### 5.4. Dopaminergic Therapy During Screening and IP treatment period

During the screening and IP treatment period no changes are made to dopaminergic therapy.

### 5.5. Concomitant Medication

There are no restrictions on any other use of medications. All patients should use medications prescribed prior to enrolment in the study. There are no restrictions with respect to starting new medications that are necessary for the patient. The Patient should not take any vitamin B3 supplements for the duration of the study.

### 5.6. Subject Compliance

Patient compliance is determined based on self-report at study visits. A pill count of remaining medication is performed when providing new study medication and at the end of the study.

### 6. Study Procedures

### 6.1. Flow Chart

**Table 8. Trial flow chart**

| | **Screening Period** | **Treatment Period Study Visit:** | | | | | | | **End of study visit** | |
|---|---|---|---|---|---|---|---|---|---|---|
| **Time** | **First Screening** | **Day 0/baseline** | **Day 1** | **Day 3** | **Day 5** | **Day 7** | **Day 14** | **Day 21** | **Day 28** | **Day 35** |
| Informed consent | X | | | | | | | | | |
| Inclusion/exclusion Evaluation | X | | | | | | | | | |
| Informed concent biobank, optional | X | | | | | | | | | |
| Physical Examination1 | X | X | | | | X | X | X | X | |
| UPDRS | | X | | | | | | | X | |
| Telephone control from study nurse | | | | X | | | | | | X⁶ |
| Record of concomitant medication | X | X | | | | X | X | X | X | |
| Medical history | X | X | | | | | | | | |
| Vital signs^{2,3} | | X² | | | | X² | X² | X² | X² | |
| Blood samples4 (safety) | | X | | X | X | X | X | X | X | |
| Blood samples for biobanking³ | | X | | | | | | | X | |
| Urin samples for biobanking | | X | | | | | | | X | |
| Treatment (IP) administration/dispensation | | X | | | | (X⁶) | (X⁶) | (X⁶) | | |
| Screening for adverse event | | X | | X | | X | X | X | X | X⁶ |

| | | | | | | | | | | |
|---|---|---|---|---|---|---|---|---|---|---|
| 1. General physical examination (chest/lung auscultation, palpation/percussion of abdomen, examination for peripheral edema) 2. Blood pressure, pulse, body weight, electrocardiogram (EKG) 3. Height (measured at Baseline visit) 4. CRP, ALAT, ASAT, GT, Bilirubin, ALP, Creatinin, Urea, RBC, Hb, WBC with differential, Platelets, CK, FT4, TSH, B12, Folic acid, homocysteine, Methylmalonic acid, Sodium, Potassium, fasting blood glucose, insulin, hCG (only at baseline) 5. EDTA blood, snap frozen EDTA, PAXgene, serum 6. If necessary to resupply. 7. Recording AE and SAEs begins after baseline (week 0) and continue to be monitored and registered throughout the duration of the study up until 7 days after last study visit (Day 35). | | | | | | | | | | |

### 6.2. By Visit

### 6.2.1. Screening Visits/ Before start of Investigational Product (IP)

The first screening visit aims to determine if the patient is eligible to be included in the study. A full physical examination and anamnestic medical history is performed. If the patient fulfils the inclusion/exclusion criteria and gives informed consent the patient is included in the study and scheduled for the baseline visit. The patient should at screening be advised to stop using any Vit B3 supplement to fulfil inclusion criteria. There should not be more than 3 months from last screening to baseline visit.

### Screening checklist:

- Informed consent
- General physical examination (chest/lung auscultation, palpation/percussion of abdomen, examination for peripheral edema)
- Record current use of medication. Advise to stop any use of vit B3 supplements
- Medical history.

### Baseline/Week 0

- At the first study visit the investigator needs to verify the informed consent for the study and offer the subject to sign the informed consent for storage and analysis of biological material.
- Verify anamnestic information gathered at screening, current use of medication and medical history.
- Verify fulfillment of inclusion and exclusion criteria.
- Clinical examination and biosampling as illustrated in Table 8.
- If the subject is enrolled (fulfills the inclusion/exclusion criteria), the study medication is dispensed at the visit to the subject by the study nurse.
- The patient is instructed to take the study medication every day for the remainder of the study. The study medication is to be taken as: 6 capsules two times a day (6 capsules x2). The capsules are taken in the morning and evening. There is no specified time of day the dosages should be taken, only that they should be taken with about 12 hours apart if possible. If a dose is missed, the patient can take the missed dose as soon as it is remembered, provided it is shorter time to the missed dose than the next scheduled dose. There are no restrictions with respect to combining the dose with other medication and/or food.
- Study medication should, if possible, be taken prior to study visits.

### 6.3. Criteria for Patient Discontinuation

Patients may be discontinued from study treatment and assessments at any time. Discontinuation and the reason for discontinuation (withdrawn from the study) are registered. Specific reasons for discontinuing a patient for this study are:
- Voluntary discontinuation by the patient who is at any time free to discontinue his/her participation in the study, without prejudice to further treatment.
- Safety reason as judged by the Principal Investigator.
- Incorrect enrolment ie, the patient does not meet the required inclusion/exclusion criteria for the study.
- Deterioration in the patient's condition which in the opinion of the Principal Investigator warrants study medication discontinuation (to be recorded as an AE or under Investigator Discretion).

### 6.4. Procedures for Discontinuation

### 6.4.1. Patient Discontinuation

Patients who are withdrawn from the study before start of treatment, are replaced. Withdrawn patients are not followed up.

### 6.4.2. Trial Discontinuation

The whole trial may be discontinued at the discretion of the PI or the sponsor in the event of any of the following: Occurrence of AEs unknown to date in respect of their nature, severity and duration. Medical or ethical reasons affecting the continued performance of the trial. Difficulties in the recruitment of patients.

### 6.5. Laboratory Tests

Relevant laboratory tests and biosampling are listed in below.

**Table 9. Safety laboratory parameters**

| **Flow chart** | | | | | | | | | **TUBES** |
|---|---|---|---|---|---|---|---|---|---|
| Visits | **Day 0 / Baseline** | **Day 1** | **Day 3** | **Day 5** | **Day 7** | **Day 14** | **Day 21** | **Day 28** | |
| Week | **0** | **1** | **1** | **1** | **1** | **2** | **3** | **4** | |
| **Routine:** | | | | | | | | | |
| hCG1 | X | | | | | | | | |
| CRP | X | | X | X | X | X | X | X | |
| ALAT | X | | X | X | X | X | X | X | |
| ASAT | X | | X | X | X | X | X | X | |
| GT | X | | X | X | X | X | X | X | |
| Bilirubin | X | | X | X | X | X | X | X | |
| ALP | X | | X | X | X | X | X | X | |
| Creatinin | X | | X | X | X | X | X | X | |
| Urea | X | | X | X | X | X | X | X | |
| RBC | X | | X | X | X | X | X | X | |
| Hb | X | | X | X | X | X | X | X | |
| WBC with differential | X | | X | X | X | X | X | X | |
| Platelets | X | | X | X | X | X | X | X | |
| CK | X | | X | X | X | X | X | X | |
| FT4 | X | | X | X | X | X | X | X | |
| TSH | X | | X | X | X | X | X | X | |
| B12 | X | | X | X | X | X | X | X | |
| Folic acid | X | | X | X | X | X | X | X | |
| homocystein | X | | X | X | X | X | X | X | |
| Methylmalonic acid | X | | X | X | X | X | X | X | |
| Sodium | X | | X | X | X | X | X | X | |
| Potassium | X | | X | X | X | X | X | X | |
| Ionised calcium | X | | X | X | X | X | X | X | |
| Fasting blood qlucose | X | | X | X | X | X | X | X | |
| Serum insulin | X | | X | X | X | X | X | X | |

| **Biobank:** | | | | | | | | | |
|---|---|---|---|---|---|---|---|---|---|
| Snap-frozen whole blood | X | | | | | | | X | FluidX, 0,7ml, 8 aliquotes |
| Morning urine | X | | | | | | | X | 3.6 ml cryotubes (2.4 ml urine in total 8 tubes) |

| | | | | | | | | | |
|---|---|---|---|---|---|---|---|---|---|
| All safety laboratory parameters are collected at the timepoints as indicated in the Flow Chart, and include hematology, liver enzymes/parameters, clinical chemistry, thyroid status, fasting glucose and insulin, as detailed in Table 9. The samples re-analysed at the local laboratory at each study site. | | | | | | | | | |

### 7. Assessments

### 7.1. Safety and Tolerability Assessments

Safety is monitored by the assessments described below as well as the collection of AEs at every visit. For the assessment schedule refer to study flow chart in Table 8.

### 8. Safety monitoring and reporting

Each patient is instructed to contact the investigator immediately should they manifest any signs or symptoms they perceive as serious. The methods for collection of safety data are described below.

### 8.1. Definitions

### 8.1.1. Adverse Event (AE)

An AE is any untoward medical occurrence in a patient administered a pharmaceutical product and which does not necessarily have a causal relationship with this treatment. An adverse event (AE) can therefore be any unfavourable and unintended sign (including an abnormal laboratory finding), symptom, or disease temporally associated with the use of a investigational product, whether or not related to the investigational product. The term AE is used to include both serious and non-serious AEs. If an abnormal laboratory value/vital sign is associated with clinical signs and symptoms, the sign/symptom is reported as an AE and the associated laboratory result/vital sign is considered as additional information that must be collected. Only intensity 2 and 3 is registered as AE. An AE has to interfere with everyday life to be of intensity 2 or 3.

### 8.1.2. Serious Adverse Event (SAE)

Any untoward medical occurrence that at any dose:
- Results in death
- Is immediately life-threatening
- Requires in-patient hospitalization or prolongation of existing hospitalization
- Results in persistent or significant disability or incapacity
- Is a congenital abnormality or birth defect

Is an important medical event that may jeopardize the subject or may require medical intervention to prevent one of the outcomes listed above. Medical and scientific judgment is to be exercised in deciding on the seriousness of a case. Important medical events may not be immediately life-threatening or result in death or hospitalization, but may jeopardize the subject or may require intervention to prevent one of the listed outcomes in the definitions above. In such situations, or in doubtful cases, the case should be considered as serious. Hospitalization for administrative reason (for observation or social reasons) is allowed at the investigator's discretion and does not qualify as serious unless there is an associated adverse event warranting hospitalization.

### 8.2. Time Period for Reporting AE and SAE

Recording AE and SAEs begins after baseline (week 0) and continues to be monitored and registered throughout the duration of the study up until 7 days after last study visit. During the course of the study all AEs and SAEs are proactively followed up for each patient; events should be followed up to resolution, unless the event is considered by the investigator to be unlikely to resolve due to the underlying disease. Every effort should be made to obtain a resolution for all events, even if the events continue after discontinuation/study completion.

### 8.3. Recording of Adverse Events

If the patient has experienced adverse event(s), the following information is recorded:
• The nature of the event(s) is described by the investigator in precise standard medical terminology (i.e. not necessarily the exact words used by the patient).
• The duration of the event is described in terms of event onset date and event ended data.
• The intensity of the adverse event: Only intensity 2 and 3 is registered as AE.

| **Assessment of Intensity** |
|---|
| The investigator makes an assessment of intensity for each AE and SAE reported during the study and assign it to 1 of the following categories: |
| 1 Mild: An event that is easily tolerated by the participant, causing minimal discomfort and not interfering with everyday activities. |
| 2 Moderate: An event that causes sufficient discomfort to interfere with normal everyday activities. |
| 3 Severe: An event that prevents normal everyday activities. An AE that is assessed as severe should not be confused with an SAE. Severe is a category utilized for rating the intensity of an event; and both AEs and SAEs can be assessed as severe. |

The Causal relationship of the event to the study medication is assessed as one of the following:
**Unrelated:** There is not a temporal relationship to investigational product administration (too early, or late, or investigational product not taken), or there is a reasonable causal relationship between non-investigational product, concurrent disease, or circumstance and the AE.
**Unlikely:** There is a temporal relationship to investigational product administration, but there is not a reasonable causal relationship between the investigational product and the AE.
**Possible:** There is reasonable causal relationship between the investigational product and the AE. Dechallenge information is lacking or unclear.
**Probable:** There is a reasonable causal relationship between the investigational product and the AE. The event responds to dechallenge. Rechallenge is not required.
**Definite:** There is a reasonable causal relationship between the investigational product and the AE.

The outcome of the adverse event, the action taken and whether the event is resolved or still ongoing are recorded. It is important to distinguish between serious and severe AEs. Severity is a measure of intensity whereas seriousness is defined by the criteria above. An AE of severe intensity need not necessarily be considered serious. For example, nausea that persists for several hours may be considered severe nausea, but is not an SAE. On the other hand, a stroke that results in only a limited degree of disability may be considered a mild stroke, but would be an SAE.

### 9. Statistical Methods and Data Analysis

### 9.1. Determination of Sample Size

NR is generally deemed non-toxic. It has undergone extensive preclinical testing, and is Generally Recognized as Safe (GRAS) for use in food products by the United States Food and Drug Administration and by the European Food Safety Authority. NR has been shown to be safe in humans in dosages up to 2000 mg daily and is non-lethal at oral doses as high as 5000 mg/kg in rats. We, therefore, assume that any severe adverse effects with a dosage of 3000 mg are highly unlikely. To confirm this, we opted for a sample size of 10 study participants in each of the treatment and placebo groups. In our previous study (Example 3), the metabolic response to NR was seen to be homogenous between patients and we therefore assume this sample size to be sufficient to assess the short-term safety of an oral dosage of 3000 mg NR.

### 9.2. Randomization

Randomization is done by e-CRF upon enrolment to the study.

### 9.3. Population for Analysis

Under the intention-to-treat principle, all randomized patients are included in the primary analyses.

### 9.4. Analyses & statistical approach

### Primary analysis

The null hypothesis (H₀) is that NR 3000 mg is safe (i.e., not associated with AE of moderate or severe intensity). In the main analysis, AEs are summarized by severity (mild, moderate, severe) and group (NR, placebo) using descriptive statistics. The presence of AEs of moderate or severe intensity with a possible, probable or definite causal relationship to NR will reject H₀. In a posthocy analysis, we compare the total frequency of AE of moderate and severe intensity and the frequency of SAE between the NR and placebo groups using Fisher's (or chi square) test.

### Secondary analyses

- Comparison of the frequency of mild AE between the NR and placebo arm using Fisher's test.
- Paired t-test to compare the mean between-visit change in NAD metabolites in blood and urine. t-test to compare the between-group (NR vs placebo) difference in the mean change (delta) in NAD-metabolites between baseline and last visit.
- Paired t-test for mean change in total UPDRS and subparts I-IV in the placebo and NR arms.
- t-test to compare the between-group (NR vs placebo) difference in the mean change (delta) in total UPDRS and subparts I-IV between baseline and last visit.

### Exploratory analyses

- Paired t-test to compare the mean between-visit change in serum homocysteine levels. t-test to compare the between-group (NR vs placebo) difference in the mean change (delta) serum homocysteine levels between baseline and last visit.
- Paired t-test to compare the mean between-visit change in fasting blood glucose and serum insulin levels. t-test to compare the between-group (NR vs placebo) difference in the mean change (delta) in fasting blood glucose and serum insulin levels between baseline and last visit.

Throughout the study duration, no serious adverse events or any adverse events of medical significance were reported. It can be therefore concluded that the NR dose of 3000 mg daily is safe.

### Discussion and conclusions

Thus, as demonstrated herein above, nicotinamide riboside is a compound capable of effectively increasing the NAD levels in the brain which can be used as a safe, well-tolerated therapy and as neuroprotective treatment for inhibiting PD progression. The present therefore invention can thus provide an improved, efficient and safe treatment of PD addressing the physiological causes of PD in humans, rather than just for alleviating the disease symptoms.

## Claims

1. Nicotinamide riboside for use in a method for treatment of Parkinson's disease (PD) in a human subject.

2. Nicotinamide riboside for use according to claim 1, wherein:
a) the method involves oral administration of nicotinamide riboside to the subject;
b) the treatment is for preventing, decreasing and/or delaying the progression of PD;
c) the treatment is a neuroprotective therapy;
d) the PD is early PD;
e) the PD is idiopathic (IPD); and/or wherein the subject does not have pathogenic mutations in genes linked to monogenic PD, such as *SNCA, LRRK2, VPS35, PRKN,* or *PINK1;*
f) the subject has nigrostriatal degeneration or denervation; and/or wherein the treatment delays nigrostriatal degeneration or denervation, and clinical disease progression;
g) the subject is diagnosed with PD within 2 years before start of treatment;
h) the subject has a Hoehn and Yahr score < 3 at start of treatment;
i) the subject is of age equal to or greater than 35 years at begin of treatment, preferably equal to or greater than 65 years, more preferably 35 to 85, more preferably 40 to 80, more preferably 55 to 75, more preferably 60 to 75, most preferably 65 to 75 years; and/or
j) the treatment duration is at least 1, 2, 6 or 12 months, preferably 52 weeks.

3. Nicotinamide riboside for use according to claim 1 or 2, wherein:
a) the subject's cerebral NAD levels increase upon nicotinamide riboside administration;
b) the subject's cerebral NAD/ATP-α molar ratio increases, within 30 days of nicotinamide riboside administration:
(i) by more than 10% relative to baseline before the treatment, or
(ii) by 0.01 or more, preferably 0.03 or more, more preferably 0.07 or more;
c) an increase in the subject's cerebral NAD levels occurs in the subject's occipital cortex upon nicotinamide riboside administration;
d) the subject's cerebral NAD levels or NAD/ATP-α molar ratios measured by ³¹P-MRS increase upon nicotinamide riboside administration;
e) the treatment increases average total NAD levels in PBMCs relative to baseline before the treatment;
f) the average total NAD levels in PBMCs within 27-33, preferably 30 days are increased relative to baseline before the treatment;
g) the treatment causes gene expression changes affecting mitochondrial pathways, the mitochondrial ribosome, RNA metabolism, and/or oxidative stress defence;
h) the score according to one or more parts (part I, II, III and/or IV) of MDS-UPDRS, or the total score according to parts I-IV or parts I-III thereof is improved as compared to the respective score at baseline before the treatment, preferably wherein a mean change in the score according to one or more parts (part I, II, III and/or IV), or the total MDS-UPDRS score according to parts I-IV or parts I-III, is at least 1 point, preferably at least 2 points as compared to the respective score at baseline before the treatment;
i) a difference in the default restating state network in fMRI measurement is obtained as compared to baseline before the treatment, preferably wherein a difference in the default restating state network in fMRI measurement is observed, as compared to baseline before the treatment;
j) the treatment acts as a neuroprotective, disease modifying therapy for PD dementia (PDD) and/or dementia with Lewy bodies (DLB).

4. Nicotinamide riboside for use according to any one of claims 1 to 3, for:
a) improving motor, non-motor and/or cognitive symptoms;
b) preventing motor, non-motor and/or cognitive symptoms;
c) improving a score as measured by Part I, II, III or IV of MDS-UPDRS;
d) improving the total score as measured by part I, II, III and IV of MDS-UPDRS;
e) improving score difference measured by the Non-Motor Symptoms Questionnaire (NMSQ), by the Non-Motor Symptoms Scale for Parkinson's Disease (NMSS), on the Hoehn & Yahr scale, by the MoCA and/or EQ-5L;
f) delaying nigrostriatal degeneration or denervation, and/or improving dopamine transporter density as measured by DaTscan;
g) delaying brain atrophy (global or focal) as measured by MRI volumetry;
h) lowering cortical atrophy as measured by cortical thickness with MRI;
i) rectifying NAD metabolism and/or mitochondrial function as measured by multi-omics in patient biosamples and brain (31)P-MRS;
j) correcting aberrant histone acetylation and gene expression profile as measured by multi-omics in patient biosamples;
k) improving brain spatiotemporal functional and/or structural connectivity as measured by fMRI;
l) delaying progression of neuronal loss, as measured by neurofilament light-chain in patient serum; and/or
m) improving the score according to the MDS Non-Motor Rating Scale (MDS-NMS).

5. Nicotinamide riboside for use according to any one of claims 1 to 4, wherein nicotinamide riboside is administered to the subject at a total dosage of 200 to 2000 mg, 500 to 2000 mg per day or 800 to 1200 mg per day; or
at a dosage of 400 to 800 mg twice per day, preferably 500 mg twice per day, more preferably 2 x 250 mg twice per day.

6. Nicotinamide riboside for use according to any one of claims 1 to 5, wherein nicotinamide riboside is administered as a monotherapy, or as a monotherapy in combination with a dopaminergic agent plus MAO-B inhibitor.

7. Nicotinamide riboside for use according to any one of claims 1 to 6, wherein nicotinamide riboside is administered in combination with a dopaminergic agent plus MAO-B inhibitor, wherein:
a) the MAO-B inhibitor is or comprises selegiline; and/or
b) the dopaminergic agent is or comprises levodopa, preferably in combination with a decarboxylase inhibitor such as carbidopa or benserazide, and with or without the addition of a COMT-inhibitor such as entacapone or tolcapone; and/or wherein the dopaminergic agent is or comprises a dopamine-agonist such as pramipexole, ropinirole, rotigotine, bromocriptine or pergolide.

8. Nicotinamide riboside for use according to claim 7, wherein:
a) 8-12 mg, preferably 10 mg selegiline is administered to the subject per day; and 150-800 mg, preferably 300-450 mg levodopa, and 37.5-200 mg, preferably 75-112.5 mg carbidopa are administered to the subject per day;
b) 10 mg selegiline is administered to the subject once per day; and 100 mg levodopa and 25 mg carbidopa are each administered to the subject three times per day;
c) 8-12 mg, preferably 10 mg selegiline is administered to the subject per day; and 150-800 mg, preferably 300-450 mg levodopa, and 37.5-200 mg, preferably 75-112.5 mg benserazide are administered to the subject per day; or
d) 10 mg selegiline is administered to the subject once per day; and 100 mg levodopa and 25 mg benserazide are each administered to the subject three times per day.

9. Nicotinamide riboside for use in the method as defined in any one of claims 1 to 8, wherein nicotinamide riboside is administered as a pharmaceutically acceptable salt, solvate and/or hydrate thereof,
preferably wherein the salt is selected from fluoride, chloride, bromide, iodide, formate, acetate, ascorbate, aspartate, benzoate, butyrate, carbonate, citrate, carbamate, formate, gluconate, glutamate, lactate, malate, methyl bromide, methyl sulfate, nitrate, phosphate, propionate, diphosphate, succinate, sulfate, sulfonate, hydrogen tartrate, hydrogen malate, trifluoroacetate, tribromomethanesulfonate, trichloromethanesulfonate, and trifluoromethanesulfonate.

10. Nicotinamide riboside for use according to any one of claims 1 to 9, wherein nicotinamide riboside is nicotinamide riboside chloride.

11. A pharmaceutical composition for use in the method as defined in any one of claims 1 to 8, the composition comprising nicotinamide riboside as defined in any one of claims 1 to 10, and optionally:
a) further comprising a pharmaceutically acceptable excipient; or
b) comprising nicotinamide riboside as the sole active ingredient, or as the sole active ingredient in combination with one or more of a dopaminergic agent and MAO-B inhibitor.

12. Dosage form for use in a method as defined in any one of claims 1 to 8, wherein the dosage form comprises nicotinamide riboside or a pharmaceutical composition as defined in any one of claims 1 to 11.

13. The dosage form for use according to claim 12, wherein the dosage form:
a) is an oral dosage form; preferably a capsule;
b) comprises 100, 250 or 300 mg of nicotinamide riboside active ingredient; and/or
c) is an oral capsule comprising, preferably consisting of, nicotinamide riboside chloride, microcrystalline cellulose, hydroxypropyl methylcellulose and magnesium stearate.

14. A pharmaceutical combination comprising nicotinamide riboside, a dopaminergic agent and a MAO-B inhibitor.

15. The pharmaceutical combination according to claim 14, wherein:
a) the MAO-B inhibitor comprises selegiline;
b) the dopaminergic agent comprises levodopa, carbidopa, and/or benserazide;
c) the pharmaceutical combination comprises nicotinamide riboside, levodopa and carbidopa; or nicotinamide riboside, levodopa and benserazide; and/or
d) one, two or three of the active ingredients are simultaneously present within a dosage form.

## Patentansprüche

1. Nicotinamid-Ribosid zur Verwendung in einem Verfahren zur Behandlung der Parkinson-Krankheit (PD) bei einem menschlichen Subjekt.

2. Nicotinamid-Ribosid zur Verwendung nach Anspruch 1, wobei:
a) das Verfahren eine orale Verabreichung von Nicotinamid-Ribosid an das Subjekt umfasst;
b) die Behandlung einem Vorbeugen, Verringern und/oder Verzögern des Fortschreitens von PD dient;
c) die Behandlung eine neuroprotektive Therapie ist;
d) die PD eine frühe PD ist;
e) die PD idiopathisch (IPD) ist; und/oder wobei das Subjekt keine pathogenen Mutationen in Genen aufweist, die mit monogener PD in Verbindung stehen, wie beispielsweise *SNCA, LRRK2, VPS35, PRKN,* oder *PINK1;*
f) das Subjekt eine nigrostriatale Degeneration oder Denervierung aufweist; und/oder wobei die Behandlung eine nigrostriatale Degeneration oder Denervierung sowie ein klinisches Fortschreiten der Krankheit verzögert;
g) bei dem Subjekt innerhalb von 2 Jahren vor Behandlungsbeginn PD diagnostiziert wird;
h) das Subjekt zu Behandlungsbeginn einen Hoehn- und Yahr-Wert von < 3 aufweist;
i) das Subjekt bei Behandlungsbeginn 35 Jahre oder älter ist, bevorzugt 65 Jahre oder älter, bevorzugter 35 bis 85, bevorzugter 40 bis 80, bevorzugter 55 bis 75, bevorzugter 60 bis 75, besonders bevorzugt 65 bis 75 Jahre alt; und/oder
j) die Behandlungsdauer mindestens 1, 2, 6 oder 12 Monate, bevorzugt 52 Wochen, beträgt.

3. Nicotinamid-Ribosid zur Verwendung nach Anspruch 1 oder 2, wobei:
a) die zerebralen NAD-Spiegel des Subjekts nach Verabreichung von Nicotinamid-Ribosid ansteigen;
b) das zerebrale NAD/ATP-α-Molverhältnis des Subjekts innerhalb von 30 Tagen nach Verabreichung von Nicotinamid-Ribosid:
(i) um mehr als 10 % relativ zum Ausgangswert vor der Behandlung, oder
(ii) um 0,01 oder mehr, bevorzugt 0,03 oder mehr, bevorzugter 0,07 oder mehr, ansteigt;
c) nach Verabreichung von Nicotinamid-Ribosid im Okzipitalkortex des Subjekts ein Anstieg der zerebralen NAD-Spiegel des Subjekts auftritt;
d) die zerebralen NAD-Spiegel oder die NAD/ATP-α-Molverhältnisse des Subjekts, gemessen mittels ³¹P-MRS nach Verabreichung von Nicotinamid-Ribosid, ansteigen;
e) die Behandlung durchschnittliche insgesamte NAD-Spiegel in PBMCs relativ zum Ausgangswert vor der Behandlung erhöht;
f) die durchschnittlichen insgesamten NAD-Spiegel in PBMCs innerhalb von 27-33, bevorzugt 30 Tagen relativ zum Ausgangswert vor der Behandlung erhöht sind;
g) die Behandlung Genexpressionsveränderungen verursacht, die sich auf mitochondriale Wege, das mitochondriale Ribosom, den RNA-Stoffwechsel und/oder eine Abwehr gegen oxidativen Stress auswirken;
h) der Wert gemäß einem oder mehreren Teilen (Teil I, II, III und/oder IV) von MDS-UPDRS oder der Gesamtwert gemäß den Teilen I-IV oder den Teilen I-III davon im Vergleich zum jeweiligen Ausgangswert vor der Behandlung verbessert ist, wobei bevorzugt eine durchschnittliche Änderung des Werts gemäß einem oder mehreren Teilen (Teil I, II, III und/oder IV) oder des Gesamt-MDS-UPDRS-Werts gemäß den Teilen I-IV oder den Teilen I-III mindestens 1 Punkt, bevorzugt mindestens 2 Punkte im Vergleich zum jeweiligen Ausgangswert vor der Behandlung beträgt;
i) ein Unterschied im Standard-Restating-State-Netzwerk bei einer fMRI-Messung im Vergleich zum Ausgangswert vor der Behandlung erhalten wird, wobei bevorzugt ein Unterschied im Standard-Restating-State-Netzwerk bei einer fMRI-Messung im Vergleich zum Ausgangswert vor der Behandlung beobachtet wird;
j) die Behandlung als eine neuroprotektive, krankheitsmodifizierende Therapie für PD-Demenz (PDD) und/oder Demenz mit Lewy-Körpern (DLB) wirkt.

4. Nicotinamid-Ribosid zur Verwendung nach einem der Ansprüche 1 bis 3, zum:
a) Verbessern motorischer, nicht-motorischer und/oder kognitiver Symptome;
b) Vorbeugen motorischer, nicht-motorischer und/oder kognitiver Symptome;
c) Verbessern eines Werts gemäß Messung durch Teil I, II, III oder IV von MDS-UPDRS;
d) Verbessern des Gesamtwerts, gemäß Messung durch Teil I, II, III und IV von MDS-UPDRS;
e) Verbessern einer Wertdifferenz, gemessen durch den Fragebogen zu nicht-motorischen Symptomen (Non-Motor Symptoms Questionnaire (NMSQ)), durch die Skala für nicht-motorische Symptome für die Parkinson-Krankheit (Non-Motor Symptoms Scale for Parkinson's Disease (NMSS)), durch die Hoehn- und Yahr-Skala, durch den MoCA und/oder den EQ-5L;
f) Verzögern einer nigrostriatalen Degeneration oder Denervierung, und/oder Verbessern einer Dopamintransporterdichte, gemessen mittels DaTscan;
g) Verzögern einer Hirnatrophie (global oder fokal), gemessen durch MRI-Volumetrie;
h) Verringern von kortikaler Atrophie, gemessen durch eine kortikale Dicke mittels MRI;
i) Berichtigen des NAD-Stoffwechsels und/oder der Mitochondrienfunktion, gemessen mittels Multi-Omics in Patientenbioproben und Gehirn-(31)P-MRS;
j) Korregieren abweichender Histonacetylierung und eines Genexpressionsprofils, gemessen mittels Multi-Omics in Patientenbioproben;
k) Verbessern einer räumlich-zeitlichen funktionellen und/oder strukturellen Konnektivität eines Gehirns, gemessen mittels fMRI;
l) Verzögern eines Fortschreitens von Nervenzellverlust, gemessen mittels Neurofilament-Leichtketten in Patientenserum; und/oder
m) Verbessern des Werts gemäß der nicht-mortorischen MDS-Bewertungsskala (MDS Non-Motor Rating Scale (MDS-NMS)).

5. Nicotinamid-Ribosid zur Verwendung nach einem der Ansprüche 1 bis 4, wobei Nicotinamid-Ribosid dem Subjekt in einer Gesamtdosierung von 200 bis 2000 mg, 500 bis 2000 mg pro Tag oder 800 bis 1200 mg pro Tag; oder
in einer Dosierung von 400 bis 800 mg zweimal täglich, bevorzugt 500 mg zweimal täglich, bevorzugter 2 × 250 mg zweimal täglich, verabreicht wird.

6. Nicotinamid-Ribosid zur Verwendung nach einem der Ansprüche 1 bis 5, wobei Nicotinamid-Ribosid als eine Monotherapie oder als eine Monotherapie in Kombination mit einem dopaminergen Wirkstoff und einem MAO-B-Hemmer verabreicht wird.

7. Nicotinamid-Ribosid zur Verwendung nach einem der Ansprüche 1 bis 6, wobei Nicotinamid-Ribosid in Kombination mit einem dopaminergen Wirkstoff und einem MAO-B-Hemmer verabreicht wird, wobei:
a) der MAO-B-Hemmer Selegilin ist oder umfasst; und/oder
b) der dopaminerge Wirkstoff Levodopa, bevorzugt in Kombination mit einem Decarboxylasehemmer, wie Carbidopa oder Benserazid, und mit oder ohne dem Zusatz eines COMT-Hemmers, wie Entacapon oder Tolcapon, ist oder umfasst; und/oder wobei der dopaminerge Wirkstoff ein Dopaminagonist, wie Pramipexol, Ropinirol, Rotigotin, Bromocriptin oder Pergolid, ist oder einen solchen umfasst.

8. Nicotinamid-Ribosid zur Verwendung nach Anspruch 7, wobei:
a) dem Subjekt täglich 8-12 mg, bevorzugt 10 mg Selegilin verabreicht werden; und dem Subjekt täglich 150-800 mg, bevorzugt 300-450 mg Levodopa und 37,5-200 mg, bevorzugt 75-112,5 mg Carbidopa verabreicht werden;
b) dem Subjekt einmal täglich 10 mg Selegilin verabreicht werden; und dem Subjekt dreimal täglich jeweils 100 mg Levodopa und 25 mg Carbidopa verabreicht werden;
c) dem Subjekt 8-12 mg, bevorzugt 10 mg Selegilin pro Tag verabreicht werden; und dem Subjekt 150-800 mg, bevorzugt 300-450 mg Levodopa und 37,5-200 mg, bevorzugt 75-112,5 mg Benserazid pro Tag verabreicht werden; oder
d) dem Subjekt einmal täglich 10 mg Selegilin verabreicht werden; und dem Subjekt dreimal täglich jeweils 100 mg Levodopa und 25 mg Benserazid verabreicht werden.

9. Nicotinamid-Ribosid zur Verwendung in dem Verfahren wie in einem der Ansprüche 1 bis 8 definiert, wobei Nicotinamid-Ribosid als ein pharmazeutisch akzeptables Salz, Solvat und/oder Hydrat davon verabreicht wird,
wobei das Salz bevorzugt ausgewählt ist aus Fluorid, Chlorid, Bromid, lodid, Formiat, Acetat, Ascorbat, Aspartat, Benzoat, Butyrat, Carbonat, Citrat, Carbamat, Formiat, Gluconat, Glutamat, Laktat, Malat, Methylbromid, Methylsulfat, Nitrat, Phosphat, Propionat, Diphosphat, Succinat, Sulfat, Sulfonat, Hydrogentartrat, Hydrogenmalat, Trifluoracetat, Tribrommethansulfonat, Trichlormethansulfonat und Trifluormethansulfonat.

10. Nicotinamid-Ribosid zur Verwendung nach einem der Ansprüche 1 bis 9, wobei Nicotinamid-Ribosid Nicotinamid-Ribosidchlorid ist.

11. Pharmazeutische Zusammensetzung zur Verwendung in dem Verfahren wie in einem der Ansprüche 1 bis 8 definiert, wobei die Zusammensetzung Nicotinamid-Ribosid wie in einem der Ansprüche 1 bis 10 definiert umfasst, und optional:
a) weiter umfassend einen pharmazeutisch akzeptablen Hilfsstoff; oder
b) umfassend Nicotinamid-Ribosid als den einzigen Wirkstoff, oder als den einzigen Wirkstoff in Kombination mit einem oder mehreren von einem dopaminergen Wirkstoff und einem MAO-B-Hemmer.

12. Dosierungsform zur Verwendung in einem Verfahren, wie in einem der Ansprüche 1 bis 8 definiert, wobei die Dosierungsform Nicotinamid-Ribosid oder eine pharmazeutische Zusammensetzung wie in einem der Ansprüche 1 bis 11 definiert umfasst.

13. Dosierungsform zur Verwendung nach Anspruch 12, wobei die Dosierungsform:
a) eine orale Dosierungsform; bevorzugt eine Kapsel ist;
b) 100, 250 oder 300 mg des Wirkstoffs Nicotinamid-Ribosid umfasst; und/oder
c) eine orale Kapsel ist, die Nicotinamid-Ribosidchlorid, mikrokristalline Cellulose, Hydroxypropylmethylcellulose und Magnesiumstearat umfasst oder bevorzugt daraus besteht.

14. Pharmazeutische Kombination, umfassend Nicotinamid-Ribosid, einen dopaminergen Wirkstoff und einen MAO-B-Hemmer.

15. Pharmazeutische Kombination nach Anspruch 14, wobei:
a) der MAO-B-Hemmer Selegilin umfasst;
b) der dopaminerge Wirkstoff Levodopa, Carbidopa und/oder Benserazid umfasst;
c) die pharmazeutische Kombination Nicotinamid-Ribosid, Levodopa und Carbidopa; oder Nicotinamid-Ribosid, Levodopa und Benserazid umfasst; und/oder
d) innerhalb einer Dosierungsform ein, zwei oder drei der Wirkstoffe gleichzeitig vorhanden sind.

## Revendications

1. Nicotinamide riboside pour utilisation dans un procédé pour le traitement de la maladie de Parkinson (PD) chez un sujet humain.

2. Nicotinamide riboside pour utilisation selon la revendication 1, dans lequel :
a) le procédé implique une administration orale de nicotinamide riboside au sujet ;
b) le traitement sert à empêcher, à diminuer et/ou à retarder la progression de la PD ;
c) le traitement est une thérapie neuroprotectrice ;
d) la PD est une PD précoce ;
e) la PD est idiopathique (IPD) ; et/ou dans lequel le sujet ne présente pas de mutations pathogènes de gènes liés une PD monogénique, tels que *SNCA, LRRK2, VPS35, PRKN,* ou *PINK1 ;*
f) le sujet présente une dégénérescence nigrostriée ou une dénervation ; et/ou dans lequel le traitement retarde la dégénérescence nigrostriée ou la dénervation et la progression de maladie clinique ;
g) le sujet est diagnostiqué atteint de PD dans les 2 ans avant le début du traitement ;
h) le sujet présente un score de Hoehn et Yahr < 3 au début du traitement ;
i) le sujet est âgé de 35 ans ou plus au début du traitement, de préférence de 65 ans ou plus, plus préférentiellement de 35 à 85, plus préférentiellement de 40 à 80, plus préférentiellement de 55 à 75, plus préférentiellement de 60 à 75, le plus préférentiellement de 65 à 75 ans ; et/ou
j) la durée du traitement est d'au moins 1, 2, 6 ou 12 mois, de préférence de 52 semaines.

3. Nicotinamide riboside pour utilisation selon la revendication 1 ou la revendication 2, dans lequel :
a) les niveaux de NAD cérébral du sujet augmentent lors de l'administration de nicotinamide riboside ;
b) le rapport molaire de NAD cérébral/ATP-α du sujet augmente, dans les 30 jours suivant l'administration de nicotinamide riboside :
(i) de plus de 10 % par rapport à une ligne de base avant le traitement, ou
(ii) de 0,01 ou plus, de préférence de 0,03 ou plus, plus préférentiellement de 0,07 ou plus ;
c) une augmentation des niveaux de NAD cérébral du sujet survient dans le cortex occipital du sujet lors de l'administration de nicotinamide riboside ;
d) les niveaux de NAD cérébral ou les rapports molaires de NAD/ATP-α du sujet, mesurés par ³¹P-MRS augmentent lors de l'administration de nicotinamide riboside ;
e) le traitement augmente des niveaux de NAD total moyens dans les PBMC par rapport à une ligne de base avant le traitement ;
f) les niveaux de NAD total moyens dans les PBMC dans les 27-33, de préférence 30 jours sont augmentés par rapport à une ligne de base avant le traitement ;
g) le traitement entraîne des changements d'expression génique affectant les voies mitochondriales, le ribosome mitochondrial, le métabolisme d'ARN et/ou la défense contre le stress oxydatif ;
h) le score selon une ou plusieurs parties (partie I, II, III et/ou IV) de MDS-UPDRS, ou le score total selon les parties I-IV ou parties I-III de celui-ci est amélioré par rapport au score respectif au niveau d'une ligne de base avant le traitement, de préférence dans lequel un changement moyen du score selon une ou plusieurs parties (partie I, II, III et/ou IV), ou le score total de MDS-UPDRS selon les parties I-IV ou parties I-III, est au moins de 1 point, de préférence d'au moins 2 points par rapport au score respectif au niveau d'une ligne de base avant le traitement ;
i) une différence dans le réseau d'état de repos par défaut lors d'une mesure de IRMf est obtenue par rapport à une ligne de base avant le traitement, de préférence dans lequel une différence dans le réseau d'état de repos par défaut lors d'une mesure de IRMf est observée, par rapport à une ligne de base avant le traitement ;
j) le traitement agit comme un neuroprotecteur, une thérapie de modification de maladie pour la démence à PD (PDD) et/ou la démence à corps de Lewy (DLB).

4. Nicotinamide riboside pour utilisation selon l'une quelconque des revendications 1 à 3, pour :
a) améliorer les symptômes moteurs, non moteurs et/ou cognitifs ;
b) prévenir les symptômes moteurs, non moteurs et/ou cognitifs ;
c) améliorer un score tel que mesuré par la Partie I, II, III ou IV de MDS-UPDRS ;
d) améliorer le score total tel que mesuré par la partie I, II, III et IV de MDS-UPDRS ;
e) améliorer une différence de score mesurée par le questionnaire de symptômes non moteurs (NMSQ), par l'échelle de symptômes non moteurs pour la maladie de Parkinson (NMSS), sur l'échelle de Hoehn & Yahr, par le MoCA et/ou l'EQ-5L ;
f) retarder la dégénérescence nigrostriée ou la dénervation, et/ou améliorer la densité de transporteurs de dopamine telle que mesurée par DaTscan ;
g) retarder l'atrophie cérébrale (globale ou focale) telle que mesurée par volumétrie IRM ;
h) abaisser l'atrophie corticale telle que mesurée par l'épaisseur corticale avec IRM ;
i) rectifier le métabolisme de NAD et/ou la fonction mitochondriale tel que mesuré par multi-omique dans des bio-échantillons de patient et (31)P-MRS cérébral ;
j) corriger l'acétylation d'histone aberrante et le profil d'expression génique tels que mesurés par multi-omique dans des bio-échantillons de patient ;
k) améliorer la connectivité spatiotemporelle fonctionnelle et/ou structurale cérébrale telle que mesurée par IRMf ;
l) retarder la progression de perte neuronale, telle que mesurée par chaîne légère de neurofilament dans du sérum de patient ; et/ou
m) améliorer le score en fonction de l'échelle d'évaluation MSD non moteur (MDS-NMS).

5. Nicotinamide riboside pour utilisation selon l'une quelconque des revendications 1 à 4, dans lequel le nicotinamide riboside est administré au sujet à une posologie totale de 200 à 2000 mg, de 500 à 2000 mg par jour ou 800 à 1200 mg par jour ; ou à une posologie de 400 à 800 mg deux fois par jour, de préférence de 500 mg deux fois par jour, plus préférentiellement 2 × 250 mg deux fois par jour.

6. Nicotinamide riboside pour utilisation selon l'une quelconque des revendications 1 à 5, dans lequel le nicotinamide riboside est administré comme monothérapie, ou comme monothérapie en combinaison avec un agent dopaminergique plus un inhibiteur de MAO-B.

7. Nicotinamide riboside pour utilisation selon l'une quelconque des revendications 1 à 6, dans lequel le nicotinamide riboside est administré en combinaison avec un agent dopaminergique plus un inhibiteur de MAO-B, dans lequel :
a) l'inhibiteur de MAO-B est ou comprend de la sélégiline ; et/ou
b) l'agent dopaminergique est ou comprend de la lévodopa, de préférence en combinaison avec un inhibiteur de décarboxylase tel que la carbidopa ou la bensérazide, et avec ou sans l'ajout d'un inhibiteur de COMT tel que l'entacapone ou le tolcapone ; et/ou dans lequel l'agent dopaminergique est ou comprend un agoniste de dopamine tel que le pramipexole, ropinirole, rotigotine, bromocriptine ou pergolide.

8. Nicotinamide riboside pour utilisation selon la revendication 7, dans lequel :
a) 8-12 mg, de préférence 10 mg de sélégiline est administré au sujet par jour ; et 150-800 mg, de préférence 300-450 mg de lévodopa, et 37,5-200 mg, de préférence 75-112,5 mg de carbidopa sont administrés au sujet par jour ;
b) 10 mg de sélégiline est administré au sujet une fois par jour ; et 100 mg de lévodopa et 25 mg de carbidopa sont chacun administrés au sujet trois fois par jour ;
c) 8-12 mg, de préférence 10 mg de sélégiline est administré au sujet par jour ; et 150-800 mg, de préférence 300-450 mg de lévodopa, et 37,5-200 mg, de préférence 75-112,5 mg de bensérazide sont administrés au sujet par jour ; ou
b) 10 mg de sélégiline est administré au sujet une fois par jour ; et 100 mg de lévodopa et 25 mg de bensérazide sont chacun administrés au sujet trois fois par jour.

9. Nicotinamide riboside pour utilisation dans le procédé tel que défini dans l'une quelconque des revendications 1 à 8, dans lequel le nicotinamide riboside est administré comme sel, solvate et/ou hydrate pharmaceutiquement acceptable de celui-ci, de préférence dans lequel le sel est sélectionné parmi fluorure, chlorure, bromure, iodure, formate, acétate, ascorbate, aspartate, benzoate, butyrate, carbonate, citrate, carbamate, formate, gluconate, glutamate, lactate, malate, bromure de méthyle, sulfate de méthyle, nitrate, phosphate, propionate, diphosphate, succinate, sulfate, sulfonate, tartrate d'hydrogène, malate d'hydrogène, trifluoroacétate, tribromométhanesulfonate, trichlorométhanesulfonate et trifluorométhanesulfonate.

10. Nicotinamide riboside pour utilisation selon l'une quelconque des revendications 1 à 9, dans lequel le nicotinamide riboside est du chlorure de nicotinamide riboside.

11. Composition pharmaceutique pour utilisation dans le procédé tel que défini dans l'une quelconque des revendications 1 à 8, la composition comprenant du nicotinamide riboside tel que défini dans l'une quelconque des revendications 1 à 10, et facultativement :
a) comprenant en outre un excipient pharmaceutiquement acceptable ; ou
b) comprenant du nicotinamide riboside comme principe actif unique, ou principe actif unique en combinaison avec un ou plusieurs d'un agent dopaminergique et d'un inhibiteur de MAO-B.

12. Forme galénique pour utilisation dans un procédé tel que défini dans l'une quelconque des revendications 1 à 8, dans laquelle la forme galénique comprend du nicotinamide riboside ou une composition pharmaceutique tel que défini dans l'une quelconque des revendications 1 à 11.

13. Forme galénique pour utilisation selon la revendication 12, dans laquelle la forme galénique :
a) est une forme galénique orale ; de préférence une capsule ;
b) comprend 100, 250 ou 300 mg de principe actif de nicotinamide riboside ; et/ou
c) est une capsule orale comprenant, de préférence consistant en, chlorure de nicotinamide riboside, cellulose microcristalline, méthylcellulose d'hydroxypropyle et stéarate de magnésium.

14. Combinaison pharmaceutique comprenant du nicotinamide riboside, un agent dopaminergique et un inhibiteur de MAO-B.

15. Composition pharmaceutique selon la revendication 14, dans laquelle :
a) l'inhibiteur de MAO-B comprend de la sélégiline ;
b) l'agent dopaminergique comprend de la lévodopa, de la carbidopa et/ou de la bensérazide ;
c) la combinaison pharmaceutique comprend du nicotinamide riboside, de la lévodopa et de la carbidopa ; ou du nicotinamide riboside, de la lévodopa et de la bensérazide ; et/ou
d) un, deux ou trois des principes actifs sont simultanément présents dans une forme galénique.
